# EUROPEAN PATENT SPECIFICATION

(11) **EP 0 888 340 B1**
(45) Date of publication and mention of the grant of the patent: **17.07.2002**
(21) Application number: 97905897.1
(22) Date of filing: 10.02.1997
(51) Int. Cl.: C07D 405/04, A61K 31/40

(54) **4-(BENZO-1,3-DIOXOLYL)-PYRROLIDINE-3-CARBOXYLIC ACID DERIVATIVES AS ENDOTHELIN ANTAGONISTS**
4-(BENZO-1,3-DIOXOLYL)-PYRROLIDIN-3-CARBONSAURE-DERIVATE ALS ENDOTHELIN-ANTAGONISTEN
DERIVES D'ACIDE 4-(BENZO-1,3-DIOXOLYL)-PYRROLIDINE-3-CARBOXYLIQUE UTILISES COMME ANTAGONISTES DE L'ENDOTHELINE

(30) Priority: 13.02.1996 US 600724; 04.02.1997 US 794505
(43) Date of publication of application: 07.01.1999
(73) Proprietor: Abbott Laboratories, Abbott Park, Illinois 60064-3500 (US)
(72) Inventor: TASKER, Andrew, S., Gurnee, IL 60031 (US); BOYD, Steven, A., Mundelein, IL 60060 (US); SORENSEN, Bryan, K., Waukegan, IL 60087 (US); WINN, Martin, Deerfield, IL 60015 (US); JAE, Hwan-Soo, Glencoe, IL 60022 (US); VON GELDERN, Thomas, W., Richmond, IL 60071 (US); HENRY, Kenneth, J., Waukegan, IL 60085 (US)
(74) Representative: Modiano, Guido, Dr.-Ing.
(86) International application number: US9702128
(87) International publication number: WO9730046

(56) References cited:
- WO-A-93/08799
- WO-A-94/02474
- WO-A-96/06095
- TETRAHEDRON LETT., vol. 37, no. 27, 1 July 1996, pages 4627-4630, XP002031190
- J. MED. CHEMISTRY, vol. 39, no. 5, 1 March 1996, pages 1039-1048, XP002031191

## Description

This is a continuation-in-part application of US Serial No. 08/600,724 filed February 13, 1996.

### Technical Field

The present invention relates to compounds which are endothelin antagonists, processes for making such compounds, synthetic intermediates employed in these processes and methods and compositions for antagonizing endothelin.

### Background of the Invention

Endothelin (ET) is a 21 amino acid peptide that is produced by endothelial cells. ET is produced by enzymatic cleavage of a Trp-Val bond in the precursor peptide big endothelin (Big ET). This cleavage is caused by an endothelin converting enzyme (ECE). Endothelin has been shown to constrict arteries and veins, increase mean arterial blood pressure, decrease cardiac output, increase cardiac contractility in vitro, stimulate mitogenesis in vascular smooth muscle cells in vitro, contract non-vascular smooth muscle including guinea pig trachea, human urinary bladder strips and rat uterus in vitro, increase airway resistance in vivo, induce formation of gastric ulcers, stimulate release of atrial natriuretic factor in vitro and in vivo, increase plasma levels of vasopressin, aldosterone and catecholamines, inhibit release of renin in vitro and stimulate release of gonadotropins in vitro.

It has been shown that vasoconstriction is caused by binding of endothelin to its receptors on vascular smooth muscle (Nature 332 411 (1988), FEBS Letters 231 440 (1988) and Biochem. Biophys. Res. Commun. 154 868 (1988)). An agent which suppresses endothelin production or an agent which binds to endothelin or which inhibits the binding of endothelin to an endothelin receptor will produce beneficial effects in a variety of therapeutic areas. In fact, an anti-endothelin antibody has been shown, upon intrarenal infusion, to ameliorate the adverse effects of renal ischemia on renal vascular resistance and glomerular filtration rate (Kon, et al., J. Clin. Invest. 83 1762 (1989)). In addition, an anti-endothelin antibody attenuated the nephrotoxic effects of intravenously administered cyclosporin (Kon, et al., Kidney Int. 37 1487 (1990)) and attenuated infarct size in a coronary artery ligation-induced myocardial infarction model (Watanabe, et al., Nature 344 114 (1990)).

Clozel et al. (Nature 365: 759-761 (1993)) report that Ro 46-2005, a nonpeptide ET-A/B antagonist, prevents post-ischaemic renal vasoconstriction in rats, prevents the decrease in cerebral blood flow due to subarachnoid hemorrhage (SAH) in rats, and decreases MAP in sodium-depleted squirrel monkeys when dosed orally. A similar effect of a linear tripeptide-like ET-A antagonist, BQ-485, on arterial caliber after SAH has also been recently reported (S.ltoh, T. Sasaki, K. Ide, K. Ishikawa, M. Nishikibe, and M. Yano, Biochem. Biophys. Res. Comm., 195: 969-75 (1993). These results indicate that agents which antagonize ET/ET receptor binding will provide therapeutic benefit in the indicated disease states.

### Disclosure of the Invention

In accordance with the present invention there are compounds of the formula (I): 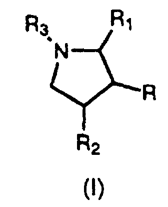 wherein
R is -(CH₂)ₘ-W wherein m is an integer from 0 to 6 and W is
(a) -C(O)₂-G wherein G is hydrogen or a carboxy protecting group,
(b) -PO₃H₂,
(c) -P(O)(OH)E wherein E is hydrogen, loweralkyl or arylalkyl,
(d) -CN,
(e) -C(O)NHR₁₇ wherein R₁₇ is loweralkyl,
(f) alkylaminocarbonyl,
(g) dialkylaminocarbonyl,
(h) tetrazolyl,
(i) hydroxy,
(j) alkoxy,
(k) sulfonamido,
(I) -C(O)NHS(O)₂R₁₆ wherein R₁₆ is loweralkyl, haloalkyl, aryl or dialkylamino,
(m) -S(O)₂NHC(O)R₁₆ wherein R₁₆ is defined as above,
(n) 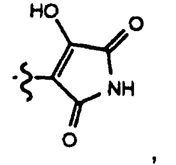
(o) 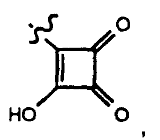
(p) 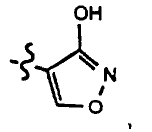
(q) 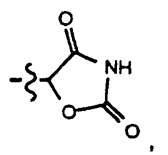
(r) 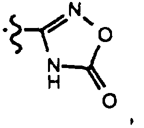
(s) 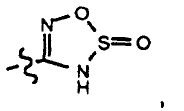
(t) 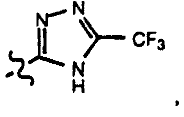 or
(u) 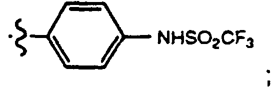
R₁ and R₂ are independently selected from hydrogen, loweralkyl, alkenyl, alkynyl, alkoxyalkyl, alkoxycarbonylalkyl, hydroxyalkyl, haloalkyl, haloalkoxyalkyl, alkoxyalkoxyalkyl, thioalkoxyalkoxyalkyl, cycloalkyl, cycloalkylalkyl, aminocarbonylalkyl, alkylaminocarbonylalkyl, dialkylaminocarbonylalkyl, aminocarbonylalkenyl, alkylaminocarbonylalkenyl, dialkylaminocarbonylalkenyl, hydroxyalkenyl, aryl, arylalkyl, aryloxyalkyl, arylalkoxyalkyl, heterocyclic, (heterocyclic)alkyl and (Rₐₐ)(R_{bb})N-R_{cc}- wherein Rₐₐ is aryl or arylalkyl, R_{bb} is hydrogen or alkanoyl and R_{cc} is alkylene, with the proviso that one or both of R₁ and R₂ is other than hydrogen;
R₃ is R₄-C(O)-R₅- or R₆-S(O)₂-R₇- wherein R₅ is (i) a covalent bond, (ii) alkylene, (iii) alkenylene, (iv) -N(R₂₀)-R₈- or -R₈ₐ-N(R₂₀)-R₈- wherein R₈ and R₈ₐ are independently selected from the group consisting of alkylene and alkenylene; and
R₂₀ is hydrogen, loweralkyl, alkenyl, haloalkyl, alkoxyalkyl, haloalkoxyalkyl, cylcoalkyl or cycloalkylalkyl or (v) -O-R₉- or -R₉ₐ-O-R₉- wherein R₉ and R₉ₐ are independently selected from alkylene;
R₇ is (i) a covalent bond, (ii) alkylene, (iii) alkenylene or (iv) -N(R₂₁)-R₁₀- or -R₁₀ₐ-N(R₂₁)-R₁₀- wherein R₁₀ and R₁₀ₐ are independently selected from the group consisting of alkylene and alkenylene and R₂₁ is hydrogen, loweralkyl, alkenyl, haloalkyl, alkoxyalkyl, haloalkoxyalkyl, aryl or arylalkyl;
wherein R₄ and R₆ are 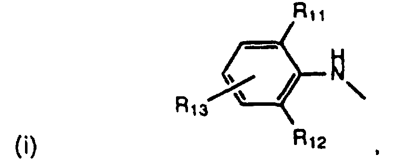 wherein R₁₁ and R₁₂ are independently selected from the group consisting of loweralkyl, cyano, alkoxy, halo, haloalkyl and phenyl and R₁₃ is hydrogen, loweralkyl, halo, carboxy, nitro, or cyano; or
(ii) heterocyclic(amino),
or a pharmaceutically acceptable salt thereof.

A preferred embodiment of the invention is a compound of formula (II) 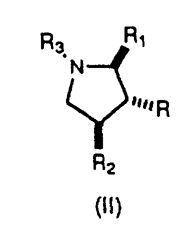 wherein the substituents -R₂, -R and -R₁ exist in a *trans,trans* relationship and R, R₁, R₂, and R₃ are as defined above.

A more preferred embodiment of the invention is a compound of formula (I) or (II) wherein R₃ is R₄-C(O)-R₅- wherein R₄ is as defined above and R₅ is alkylene or R₃ is R₆-S(O)₂-R₇- wherein R₇ is alkylene and R₆ is defined as above.

An even more preferred embodiment of the invention is a compound of formula (I) or (II) wherein R is -C(O)₂-G wherein G is hydrogen or a carboxy protecting group or R is tetrazolyl or R is -C(O)-NHS(O)₂R₁₆ wherein R₁₆ is loweralkyl, haloalkyl or aryl, R₁ and R₂ are independently selected from (i) loweralkyl, (ii) cycloalkyl, (iii) substituted aryl wherein aryl is phenyl substituted with one, two or three substituents independently selected from loweralkyl, alkoxy, halo, alkoxyalkoxy and carboxyalkoxy and (iv) substituted or unsubstituted heterocyclic, and R₃ is R₄-C(O)-R₅- wherein R₄ is as defined above and R₅ is alkylene or R₃ is R₆-S(O)₂-R₇- wherein R₇ is alkylene and R₆ is defined as above.

A yet more preferred embodiment of the invention is a compound of formula (I) or (II) wherein R is -C(O)₂-G wherein G is hydrogen or a carboxy protecting group, tetrazolyl or -C(O)-NHS(O)₂R₁₆ wherein R₁₆ is loweralkyl, haloalkyl or aryl, R₁ is (i) alkoxyalkyl, (ii) cycloalkyl, (iii) phenyl, (iv) pyridyl, (v) furanyl or (vi) substituted or unsubstituted 4-methoxyphenyl, 4-fluorophenyl, 3-fluorophenyl, 4-ethoxyphenyl, 4-propoxyphenyl 4-trifluoromethylphenyl, 4-pentafluoroethylphenyl, 3-fluoro-4-methoxyphenyl, 3-fluoro-4-ethoxyphenyl, 3-fluoro-4-propoxyphenyl, 3-methoxy-4-propoxyphenyl, 2-fluorophenyl, 4-methoxymethoxyphenyl, 4-hydroxyphenyl, 1,3-benzodioxolyl, 1,4-benzodioxanyl or dihydrobenzofuranyl wherein the substituent is selected from alkoxy, alkoxyalkoxy and carboxyalkoxy, R₂ is substituted or unsubstituted 1,3-benzodioxolyl, 7-methoxy-1,3-benzodioxolyl, 1,4-benzodioxanyl, 8-methoxy-1,4-benzodioxanyl, dihydrobenzofuranyl, benzofuranyl, 4-methoxyphenyl, dimethoxyphenyl, fluorophenyl or difluorophenyl and R₃ is R₄-C(O)-R₅- wherein R₄ is as defined above and R₅ is alkylene or R₃ is R₆-S(O)₂-R₇- wherein R₇ is alkylene and R₆ is defined as above.

Another yet more preferred embodiment of the invention is a compound of formula (I) or (II) wherein R is -C(O)₂-G wherein G is hydrogen or a carboxy protecting group, tetrazolyl or -C(O)-NHS(O)₂R₁₆ wherein R₁₆ is loweralkyl, haloalkyl or aryl, R₁ is (i) alkoxyalkyl, (ii) cycloalkyl, (iii) phenyl, (iv) pyridyl, (v) furanyl or (vi) substituted or unsubstituted 4-methoxyphenyl, 4-fluorophenyl, 3-fluorophenyl, 4-ethoxyphenyl, 4-propoxyphenyl 4-trifluoromethylphenyl, 4-pentafluoroethylphenyl, 3-fluoro-4-methoxyphenyl, 3-fluoro-4-ethoxyphenyl, 3-fluoro-4-propoxyphenyl, 3-methoxy-4-propoxyphenyl, 2-fluorophenyl, 4-methoxymethoxyphenyl, 4-hydroxyphenyl, 1,3-benzodioxolyl, 1,4-benzodioxanyl or dihydrobenzofuranyl wherein the substituent is selected from alkoxy, alkoxyalkoxy and carboxyalkoxy, R₂ is substituted or unsubstituted 1,3-benzodioxolyl, 7-methoxy-1,3-benzodioxolyl, 1,4-benzodioxanyl, 8-methoxy-1,4-benzodioxanyl, dihydrobenzofuranyl, benzofurnayl, 4-methoxyphenyl, dimethoxyphenyl, fluorophenyl or difluorophenyl and R₃ is R₄-C(O)-R₅- wherein R₄ is as defined above and R₅ is alkylene.

A still more preferred embodiment of the invention is a compound of formula (I) or (II) wherein R is -C(O)₂-G wherein G is hydrogen or a carboxy protecting group, tetrazolyl or -C(O)-NHS(O)₂R₁₆ wherein R₁₆ is loweralkyl or haloalkyl, R₁ is substituted or unsubstituted 4-methoxyphenyl, 4-fluorophenyl, 2-fluorophenyl, 4-ethoxyphenyl, 4-propoxyphenyl, 3-fluoro-4-methoxyphenyl, 3-fluoro-4-ethoxyphenyl, 3-fluoro-4-propoxyphenyl, 3-methoxy-4-propoxyphenyl, 4-trifluoromethylphenyl, 4-pentafluoroethylphenyl, 4-methoxymethoxyphenyl, 4-hydroxyphenyl, 1,3-benzodioxolyl, 1,4-benzodioxanyl or dihydrobenzofuranyl wherein the substituent is selected from alkoxy, alkoxyalkoxy and carboxyalkoxy, R₂ is 1,3-benzodioxolyl, 7-methoxy-1,3-benzodioxolyl, 1,4-benzodioxanyl, dihydrobenzofuranyl, benzofuranyl, 4-methoxyphenyl, dimethoxyphenyl, fluorophenyl or difluorophenyl and R₃ is R₄-C(O)-R₅- wherein R₄ is as defined above and R₅ is alkylene.

A most highly preferred embodiment of the invention is a compound of formula (I) or (II) wherein R is -C(O)₂-G wherein G is hydrogen or a carboxy protecting group, R₁ is substituted or unsubstituted 4-methoxyphenyl, 4-fluorophenyl, 2-fluorophenyl, 3-fluoro-4-ethoxyphenyl, 3-fluoro-4-methoxyphenyl, 4-ethoxyphenyl, 4-propoxyphenyl, 3-fluoro-4-propoxypheyl, 3-methoxy-4-propoxyphenyl, 4-methoxymethoxyphenyl, 4-hydroxyphenyl, 4-ethylphenyl, 1,3-benzodioxolyl, 1,4-benzodioxanyl or dihydrobenzofuranyl wherein the substituent is selected from alkoxy, alkoxyalkoxy and carboxyalkoxy, R₂ is 1,3-benzodioxolyl, 7-methoxy-1,3-benzodioxolyl, 1,4-benzodioxanyl, dihydrobenzofuranyl, benzofuranyl, 4-methoxyphenyl, dimethoxyphenyl, fluorophenyl or difluorophenyl and R₃ is R₄-C(O)-R₅- wherein R₄ is 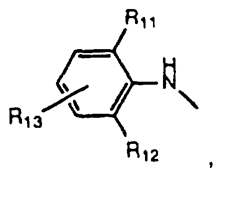 wherein R₁₁ and R₁₂ are independently selected from the group consisting of loweralkyl and R₁₃ is hydrogen, loweralkyl, halo, carboxy, nitro, or cyano and R₅ is methylene.

A most highly preferred embodiment of the invention is a compound of formula (I) or (II) wherein R is -C(O)₂-G wherein G is hydrogen or a carboxy protecting group, R₁ is substituted or unsubstituted 4-methoxyphenyl, 4-fluorophenyl, 2-fluorophenyl, 3-fluoro-4-ethoxyphenyl, 3-fluoro-4-methoxyphenyl, 4-ethoxyphenyl, 4-propoxyphenyl, 3-fluoro-4-propoxyphenyl, 3-methoxy-4-propoxyphenyl, 4-methoxymethoxyphenyl, 4-hydroxyphenyl, 4-ethylphenyl, 1,3-benzodioxolyl, 1,4-benzodioxanyl or dihydrobenzofuranyl wherein the substituent is selected from alkoxy, alkoxyalkoxy and carboxyalkoxy, R₂ is 1,3-benzodioxolyl, 7-methoxy-1,3-benzodioxolyl, 1,4-benzodioxanyl, dihydrobenzofuranyl, benzofuranyl, 4-methoxyphenyl, dimethoxyphenyl, fluorophenyl or difluorophenyl and R₃ is R₄-C(O)-R₅- wherein R₄ is 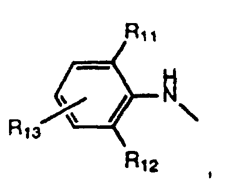 wherein R₁₁ and R₁₂ are independently selected from the group consisting of alkoxy and halo, and R₁₃ is hydrogen, loweralkyl, halo, carboxy, nitro or cyano and R₅ is methylene.

Another most highly preferred embodiment of the invention is a compound of formula (I) or (It) wherein R is -C(O)₂-G wherein G is hydrogen or a carboxy protecting group, R₁ is substituted or unsubstituted 4-methoxyphenyl, 4-fluorophenyl, 2-fluorophenyl, 3-fluoro-4-ethoxyphenyl, 3-fluoro-4-methoxyphenyl, 4-ethoxyphenyl, 4-propoxyphenyl, 3-fluoro-4-propoxypheyl, 3-methoxy-4-propoxyphenyl, 4-methoxymethoxyphenyl, 4-hydroxyphenyl, 4-ethylphenyl, 1,3-benzodioxolyl, 1,4-benzodioxanyl or dihydrobenzofuranyl wherein the substituent is selected from alkoxy, alkoxyalkoxy and carboxyalkoxy, R₂ is 1,3-benzodioxolyl, 7-methoxy-1,3-benzodioxolyl, 1,4-benzodioxanyl, dihydrobenzofuranyl, benzofuranyl, 4-methoxyphenyl, dimethoxyphenyl, fluorophenyl or difluorophenyl and R₃ is R₄-C(O)-R₅- wherein R₄ is 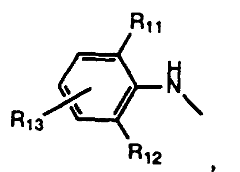 wherein R₁₁ and R₁₂ are independently selected from the group consisting of methyl, ethyl, and isopropyl, and R₁₃ is hydrogen, loweralkyl, halo, carboxy, nitro, or cyano and R₅ is methylene.

Another most highly preferred embodiment of the present invention is a compound of formula (I) or (II) wherein
- R: is -C(O)₂-G wherein G is hydrogen or a carboxy protecting group,
- R₁: is substituted or unsubstituted 4-methoxyphenyl, 4-fluorophenyl, 2-fluorophenyl, 3-fluoro-4-ethoxyphenyl, 3-fluoro-4-methoxyphenyl 4-ethoxyphenyl, 4-propoxyphenyl, 3-fluoro-4-propoxypheyl, 3-methoxy-4-propoxyphenyl, 4-methoxymethoxyphenyl, 4-hydroxyphenyl, 4-ethylphenyl, 1,3-benzodioxolyl, 1,4-benzodioxanyl or dihydrobenzofuranyl wherein the substituent is selected from alkoxy, alkoxyalkoxy and carboxyalkoxy,
- R₂: is 1,3-benzodioxolyl, 7-methoxy-1,3-benzodioxolyl, 1,4-benzodioxanyl, dihydrobenzofuranyl, benzofuranyl, 4-methoxyphenyl, dimethoxyphenyl, fluorophenyl or difluorophenyl and
- R₃: is R₄-C(O)-R₅- wherein R₄ is 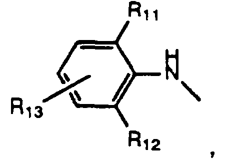 wherein R₁₁ and R₁₂ are independently selected from the group consisting of halo and alkoxy, and R₁₃ is hydrogen, loweralkyl, halo, carboxy, nitro, or cyano and R₅ is methylene.

The present invention also relates to processes for preparing the compounds of formula (I) and (II) and to the synthetic intermediates employed in these processes.

The present invention also relates to a method of antagonizing endothelin in a mammal (preferably, a human) in need of such treatment, comprising administering to the mammal a therapeutically effective amount of a compound of formula (I) or (II).

The invention further relates to endothelin antagonizing compositions comprising a pharmaceutical carrier and a therapeutically effective amount of a compound of formula (I) or (II).

The compounds of the invention comprise two or more asymmetrically substituted carbon atoms. As a result, racemic mixtures, mixtures of diastereomers, as well as single diastereomers of the compounds of the invention are included in the present invention. The terms "S" and "R" configuration are as defined by the IUPAC 1974 Recommendations for Section E, Fundamental Stereochemistry, Pure Appl. Chem. (1976) 45, 13 - 30.

The term "carboxy protecting group" as used herein refers to a carboxylic acid protecting ester group employed to block or protect the carboxylic acid functionality while the reactions involving other functional sites of the compound are carried out. Carboxy protecting groups are disclosed in Greene, "Protective Groups in Organic Synthesis" pp. 152-186 (1981), which is hereby incorporated herein by reference. In addition, a carboxy protecting group can be used as a prodrug whereby the carboxy protecting group can be readily cleaved *in vivo*, for example by enzymatic hydrolysis, to release the biologically active parent. T. Higuchi and V. Stella provide a thorough discussion of the prodrug concept in "Pro-drugs as Novel Delivery Systems", Vol 14 of the A.C.S. Symposium Series, American Chemical Society (1975), which is hereby incorporated herein by reference. Such carboxy protecting groups are well known to those skilled in the art, having been extensively used in the protection of carboxyl groups in the penicillin and cephalosporin fields, as described in U.S. Pat. No. 3,840,556 and 3,719,667, the disclosures of which are hereby incorporated herein by reference. Examples of esters useful as prodrugs for compounds containing carboxyl groups can be found on pages 14-21 of "Bioreversible Carriers in Drug Design: Theory and Application", edited by E.B. Roche, Pergamon Press, New York (1987), which is hereby incorporated herein by reference. Representative carboxy protecting groups are C₁ to C₈ alkyl (e.g., methyl, ethyl or tertiary butyl and the like); haloalkyl; alkenyl; cycloalkyl and substituted derivatives thereof such as cyclohexyl, cylcopentyl and the like; cycloalkylalkyl and substituted derivatives thereof such as cyclohexylmethyl, cylcopentylmethyl and the like; arylalkyl, for example, phenethyl or benzyl and substituted derivatives thereof such as alkoxybenzyl or nitrobenzyl groups and the like; arylalkenyl, for example, phenylethenyl and the like; aryl and substituted derivatives thereof, for example, 5-indanyl and the like; dialkylaminoalkyl (e.g., dimethylaminoethyl and the like); alkanoyloxyalkyl groups such as acetoxymethyl, butyryloxymethyl, valeryloxymethyl, isobutyryloxymethyl, isovaleryloxymethyl, 1-(propionyloxy)-1-ethyl, 1-(pivaloyloxyl)-1-ethyl, 1-methyl-1-(propionyloxy)-1-ethyl, pivaloyloxymethyl, propionyloxymethyl and the like; cycloalkanoyloxyalkyl groups such as cyclopropylcarbonyloxymethyl, cyclobutylcarbonyloxymethyl, cyclopentylcarbonyloxymethyl, cyclohexylcarbonyloxymethyl and the like; aroyloxyalkyl, such as benzoyloxymethyl, benzoyloxyethyl and the like; arylalkylcarbonyloxyalkyl, such as benzylcarbonyloxymethyl, 2-benzylcarbonyloxyethyl and the like; alkoxycarbonylalkyl, such as methoxycarbonylmethyl, cyclohexyloxycarbonylmethyl, 1-methoxycarbonyl-1-ethyl, and the like; alkoxycarbonyloxyalkyl, such as methoxycarbonyloxymethyl, t-butyloxycarbonyloxymethyl, 1-ethoxycarbonyloxy-1-ethyl, 1-cyclohexyloxycarbonyloxy-1-ethyl and the like; alkoxycarbonylaminoalkyl, such as t-butyloxycarbonylaminomethyl and the like; alkylaminocarbonylaminoalkyl, such as methylaminocarbonylaminomethyl and the like; alkanoylaminoalkyl, such as acetylaminomethyl and the like; heterocycliccarbonyloxyalkyl, such as 4-methylpiperazinylcarbonyloxymethyl and the like; dialkylaminocarbonylalkyl, such as dimethylaminocarbonylmethyl, diethylaminocarbonylmethyl and the like; (5-(loweralkyl)-2-oxo-1,3-dioxolen-4-yl)alkyl, such as (5-t-butyl-2-oxo-1,3-dioxolen-4-yl)methyl and the like; and (5-phenyl-2-oxo-1,3-dioxolen-4-yl)alkyl, such as (5-phenyl-2-oxo-1,3-dioxolen-4-yl)methyl and the like.

The term "N-protecting group" or "N-protected" as used herein refers to those groups intended to protect the N-terminus of an amino acid or peptide or to protect an amino group against undersirable reactions during synthetic procedures. Commonly used N-protecting groups are disclosed in Greene, "Protective Groups In Organic Synthesis," (John Wiley & Sons, New York (1981)), which is hereby incorporated by reference. N-protecting groups comprise acyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl and the like: carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl and the like; alkyl groups such as benzyl, triphenylmethyl, benzyloxymethyl and the like; and silyl groups such as trimethylsilyl and the like. Preferred N-protecting groups are formyl, acetyl, benzoyl, pivaloyl, t-butylacetyl, phenylsulfonyl, benzyl, t-butyloxycarbonyl (Boc) and benzyloxycarbonyl (Cbz).

The term "alkanoyl" as used herein refers to an alkyl group as previously defined appended to the parent molecular moiety through a carbonyl (-C(O)-) group. Examples of alkanoyl include acetyl, propionyl and the like.

The term "alkanoylamino" as used herein refers to an alkanoyl group as previously defined appended to an amino group. Examples alkanoylamino include acetamido, propionylamido and the like.

The term "alkanoylaminoalkyl" as used herein refers to R₄₃-NH-R₄₄- wherein R₄₃ is an alkanoyl group and R₄₄ is an alkylene group.

The term "alkanoyloxyalkyl" as used herein refers to R₃₀-O-R₃₁- wherein R₃₀ is an alkanoyl group and R₃₁ is an alkylene group. Examples of alkanoyloxyalkyl include acetoxymethyl, acetoxyethyl and the like.

The term "alkenyl" as used herein refers to a straight or branched chain hydrocarbon radical containing from 2 to 15 carbon atoms and also containing at least one carbon-carbon double bond. Alkenyl groups include, for example, vinyl (ethenyl), allyl (propenyl), butenyl, 1-methyl-2-buten-1-yl and the like.

The term "alkenylene" denotes a divalent group derived from a straight or branched chain hydrocarbon containing from 2 to 15 carbon atoms and also containing at least one carbon-carbon double bond. Examples of alkenylene include -CH=CH-, -CH₂CH=CH-, -C(CH₃)=CH-, -CH₂CH=CHCH₂-, and the like.

The term "alkenyloxy" as used herein refers to an alkenyl group, as previously defined, connected to the parent molecular moiety through an oxygen (-O-) linkage. Examples of alkenyloxy include allyloxy, butenyloxy and the like.

The term "alkoxy" as used herein refers to R₄₁O- wherein R₄₁ is a loweralkyl group, as defined herein. Examples of alkoxy include, but are not limited to, methoxy, ethoxy, propoxy, tert-butoxy, and the like.

The term "alkoxyalkoxy" as used herein refers to R₈₀O-R₈₁O- wherein R₈₀ is loweralkyl as defined above and R₈₁ is alkylene. Representative examples of alkoxyalkoxy groups include methoxymethoxy, ethoxymethoxy, t-butoxymethoxy and the like.

The term "alkoxyalkoxyalkyl" as used herein refers to an alkoxyalkoxy group as previously defined appended to an alkyl radical. Representative examples of alkoxyalkoxyalkyl groups include methoxyethoxyethyl, methoxymethoxymethyl, and the like.

The term "alkoxyalkyl" as used herein refers to an alkoxy group as previously defined appended to an alkyl radical as previously defined. Examples of alkoxyalkyl include, but are not limited to, methoxymethyl, methoxyethyl, isopropoxymethyl and the like.

The term "alkoxycarbonyl" as used herein refers to an alkoxyl group as previously defined appended to the parent molecular moiety through a carbonyl group. Examples of alkoxycarbonyl include methoxycarbonyl, ethoxycarbonyl, isopropoxycarbonyl and the like.

The term "alkoxycarbonylalkenyl" as used herein refers to an alkoxycarbonyl group as previously defined appended to an alkenyl radical. Examples of alkoxycarbonylalkenyl include methoxycarbonylethenyl, ethoxycarbonylethenyl and the like.

The term "alkoxycarbonylalkyl" as used herein refers to R₃₄-C(O)-R₃₅- wherein R₃₄ is an alkoxy group and R₃₅ is an alkylene group. Examples of alkoxycarbonylalkyl include methoxycarbonylmethyl, methoxcarbonylethyl, ethoxycarbonylmethyl and the like.

The term "alkoxycarbonylaminoalkyl" as used herein refers to R₃₈-C(O)-NH-R₃₉- wherein R₃₈ is an alkoxy group and R₃₉ is an alkylene group.

The term "alkoxycarbonyloxyalkyl" as used herein refers to R₃₆-C(O)-O-R₃₇- wherein R₃₆ is an alkoxy group and R₃₇ is an alkylene group.

The term "(alkoxycarbonyl)thioalkoxy" as used herein refers to an alkoxycarbonyl group as previously defined appended to a thioalkoxy radical. Examples of (alkoxycarbonyl)thioalkoxy include methoxycarbonylthiomethoxy, ethoxycarbonylthiomethoxy and the like.

The terms "alkyl" and "loweralkyl" as used herein refer to straight or branched chain alkyl radicals containing from 1 to 15 carbon atoms including, but not limited to, methyl, ethyl, n-propyl, iso-propyl, n-butyl, iso-butyl, sec-butyl, t-butyl, n-pentyl, 1-methylbutyl, 2,2-dimethylbutyl, 2-methylpentyl, 2,2-dimethylpropyl, n-hexyl and the like.

The term "alkylamino" as used herein refers to R₅₁NH- wherein R₅₁ is a loweralkyl group, for example, ethylamino, butylamino, and the like.

The term "alkylaminocarbonyl" as used herein refers to an alkylamino group, as previously defined, appended to the parent molecular moiety through a carbonyl (-C(O)-) linkage. Examples of alkylaminocarbonyl include methylaminocarbonyl, ethylaminocarbonyl, isopropylaminocarbonyl and the like.

The term "alkylaminocarbonylalkenyl" as used herein refers to an alkenyl radical to which is appended an alkylaminocarbonyl group.

The term "alkylaminocarbonylalkyl" as used herein refers to a loweralkyl radical to which is appended an alkylaminocarbonyl group.

The term "alkylaminocarbonylaminoalkyl" as used herein refers to R₄₀-C(O)-NH-R₄₁- wherein R₄₀ is an alkylamino group and R₄₁ is an alkylene group.

The term "alkylene" denotes a divalent group derived from a straight or branched chain saturated hydrocarbon having from 1 to 15 carbon atoms by the removal of two hydrogen atoms, for example -CH₂-, -CH₂CH₂-, -CH(CH₃)-, -CH₂CH₂CH₂-, -CH₂C(CH₃)₂CH₂- and the like.

The term "alkylsulfonylamino" as used herein refers to an alkyl group as previously defined appended to the parent molecular moiety through a sulfonylamino (-S(O)₂-NH-) group. Examples of alkylsulfonylamino include methylsulfonylamino, ethylsulfonylamino, isopropylsulfonylamino and the like.

The term "alkynyl" as used herein refers to a straight or branched chain hydrocarbon radical containing from 2 to 15 carbon atoms and also containing at least one carbon-carbon triple bond. Examples of alkynyl include -C≡C-H, H-C≡C-CH₂-, H-C≡C-CH(CH₃)- and the like.

The term "aminocarbonyl" as used herein refers to H₂N-C(O)-.

The term "aminocarbonylalkenyl" as used herein refers to an alkenyl radical to which is appended an aminocarbonyl (NH₂C(O)-) group.

The term "aminocarbonylalkoxy" as used herein refers to H₂N-C(O)- appended to an alkoxy group as previously defined. Examples of aminocarbonylalkoxy include aminocarbonylmethoxy, aminocarbonylethoxy and the like.

The term "aminocarbonylalkyl" as used herein refers to a loweralkyl radical to which is appended an aminocarbonyl (NH₂C(O)-) group.

The term "aroyloxyalkyl" as used herein refers to R₃₂-C(O)-O-R₃₃- wherein R₃₂ is an aryl group and R₃₃ is an alkylene group. Examples of aroyloxyalkyl include benzoyloxymethyl, benzoyloxyethyl and the like.

The term "aryl" as used herein refers to a mono- or bicyclic carbocyclic ring system having one or two aromatic rings including, but not limited to, phenyl, naphthyl, tetrahydronaphthyl, indanyl, indenyl and the like. Aryl groups can be unsubstituted or substituted with one, two or three substituents independently selected from loweralkyl, halo, haloalkyl, haloalkoxy, hydroxyalkyl, alkenyloxy, alkoxy, alkoxyalkoxy, alkoxycarbonyl, alkoxycarbonylalkenyl, (alkoxycarbonyl)thioalkoxy, thioalkoxy, amino, alkylamino, dialkylamino, aminocarbonyl, aminocarbonylalkoxy, alkanoylamino, arylalkoxy, aryloxy, mercapto, cyano, nitro, carboxaldehyde, carboxy, carboxyalkenyl, carboxyalkoxy, alkylsulfonylamino, cyanoalkoxy, (heterocyclic)alkoxy, hydroxy, hydroxalkoxy, phenyl and tetrazolylalkoxy. In addition, substituted aryl groups include tetrafluorophenyl and pentafluorophenyl.

The term "arylalkenyl" as used herein refers to an alkenyl radical to which is appended an aryl group, for example, phenylethenyl and the like.

The term "arylalkoxy" as used herein refers to R₄₂O- wherein R₄₂ is an arylalkyl group, for example, benzyloxy, and the like.

The term "arylalkoxyalkyl" as used herein refers to a loweralkyl radical to which is appended an arylalkoxy group, for example, benzyloxymethyl and the like.

The term "arylalkyl" as used herein refers to an aryl group as previously defined, appended to a loweralkyl radical, for example, benzyl and the like.

The term "aryloxy" as used herein refers to R₄₅O- wherein R₄₅ is an aryl group, for example, phenoxy, and the like.

The term "arylalkylcarbonyloxyalkyl" as used herein refers to a loweralkyl radical to which is appended an arylalkylcarbonyloxy group (i.e., R₆₂C(O)O- wherein R₆₂ is an arylalkyl group).

The term "aryloxyalkyl" refers to an aryloxy group as previously defined appended to an alkyl radical. Examples of aryloxyalkyl include phenoxymethyl, 2-phenoxyethyl and the like.

The term "carboxaldehyde" as used herein refers to a formaldehyde radical, -C(O)H.

The term "carboxy" as used herein refers to a carboxylic acid radical, -C(O)OH.

The term "carboxyalkenyl" as used herein refers to a carboxy group as previously defined appended to an alkenyl radical as previously defined. Examples of carboxyalkenyl include 2-carboxyethenyl, 3-carboxy-1-ethenyl and the like.

The term "carboxyalkoxy" as used herein refers to a carboxy group as previously defined appended to an alkoxy radical as previously defined. Examples of carboxyalkoxy include carboxymethoxy, carboxyethoxy and the like.

The term "cyanoalkoxy" as used herein refers to an alkoxy radical as previously defined to which is appended a cyano (-CN) group. Examples of cyanoalkoxy include 3-cyanopropoxy, 4-cyanobutoxy and the like.

The term "cycloalkanoyloxyalkyl" as used herein refers to a loweralkyl radical to which is appended a cycloalkanoyloxy group (i.e., R₆₀-C(O)-O- wherein R₆₀ is a cycloalkyl group).

The term "cycloalkyl" as used herein refers to an aliphatic ring system having 3 to 10 carbon atoms and 1 to 3 rings including, but not limited to, cyclopropyl, cyclopentyl, cyclohexyl, norbornyl, adamantyl, and the like. Cycloalkyl groups can be unsubstituted or substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide.

The term "cycloalkylalkyl" as used herein refers to a cycloalkyl group appended to a loweralkyl radical, including but not limited to cyclohexylmethyl.

The term "dialkylamino" as used herein refers to R₅₆R₅₇N- wherein R₅₆ and R₅₇ are independently selected from loweralkyl, for example diethylamino, methyl propylamino, and the like.

The term "dialkylaminoalkyl" as used herein refers to a loweralkyl radical to which is appended a dialkylamino group.

The term "dialkylaminocarbonyl" as used herein refers to a dialkylamino group, as previously defined, appended to the parent molecular moiety through a carbonyl (-C(O)-) linkage. Examples of dialkylaminocarbonyl include dimethylaminocarbonyl, diethylaminocarbonyl and the like.

The term "dialkylaminocarbonylalkenyl" as used herein refers to an alkenyl radical to which is appended a dialkylaminocarbonyl group.

The term "dialkylaminocarbonylalkyl" as used herein refers to R₅₀-C(O)-R₅₁- wherein R₅₀ is a dialkylamino group and R₅₁ is an alkylene group.

The term "halo" or "halogen" as used herein refers to I, Br, Cl or F.

The term "haloalkenyl" as used herein refers to an alkenyl radical to which is appended at least one halogen substituent.

The term "haloalkoxy" as used herein refers to an alkoxy radical as defined above, bearing at least one halogen substituent, for example,
2-fluoroethoxy, 2,2,2-trifluoroethoxy, trifluoromethoxy, 2,2,3,3,3-pentafluoropropoxy and the like.

The term "haloalkoxyalkyl" as used herein refers to a loweralkyl radical to which is appended a haloalkoxy group.

The term "haloalkyl" as used herein refers to a lower alkyl radical, as defined above, to which is appended at least one halogen substituent, for example, chloromethyl, fluoroethyl, trifluoromethyl or pentafluoroethyl and the like.

The term "heterocyclic ring" or "heterocyclic" or "heterocycle" as used herein refers to any 3- or 4-membered ring containing a heteroatom selected from oxygen, nitrogen and sulfur; or a 5-, 6- or 7-membered ring containing one, two or three nitrogen atoms; one oxygen atom; one sulfur atom; one nitrogen and one sulfur atom; one nitrogen and one oxygen atom; two oxygen atoms in non-adjacent positions; one oxygen and one sulfur atom in non-adjacent positions; or two sulfur atoms in non-adjacent positions. The 5-membered ring has 0-2 double bonds and the 6- and 7-membered rings have 0-3 double bonds. The nitrogen heteroatoms can be optionally quaternized. The term "heterocyclic" also includes bicyclic groups in which any of the above heterocyclic rings is fused to a benzene ring or a cyclohexane ring or another heterocyclic ring (for example, indolyl, dihydroindolyl, quinolyl, isoquinolyl, tetrahydroquinolyl, tetrahydroisoquinolyl, decahydroquinolyl, decahydroisoquinolyl, benzofuryl, dihydrobenzofuryl or benzothienyl and the like). Heterocyclics include: aziridinyl, azetidinyl, pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, homopiperidinyl, pyrazinyl, piperazinyl, pyrimidinyl, pyridazinyl, oxazolyl, oxazolidinyl, isoxazolyl, isoxazolidinyl, morpholinyl, thiomorpholinyl, thiazolyl, thiazolidinyl, isothiazolyl, isothiazolidinyl, indolyl, quinolinyl, isoquinolinyl, benzimidazolyl, benzothiazolyl, benzoxazolyl, oxetanyl, furyl, tetrahydrofuranyl, thienyl, thiazolidinyl, isothiazolyl, triazolyl, tetrazolyl, isoxazolyl, oxadiazolyl, thiadiazolyl, pyrrolyl, pyrimidyl and benzothienyl. Heterocyclics also include compounds of the formula 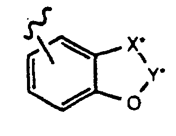 where X* is -CH2- or -O- and Y* is -C(O)- or [-C(R")₂-]ᵥ where R" is hydrogen or C₁-C₄-alkyl and v is 1, 2 or 3 such as 1,3-benzodioxolyl, 1,4-benzodioxanyl and the like. Heterocyclics also include bicyclic rings such as quinuclidinyl and the like.

Heterocyclics can be unsubstituted, monosubstituted, disubstituted, or trisubstituted with substituents independently selected from hydroxy, halo, oxo (=O), alkylimino (R*N= wherein R* is a loweralkyl group), amino, alkylamino, dialkylamino, alkoxy, alkoxyalkoxy, haloalkyl, cycloalkyl, aryl, phenyl, arylalkyl, -COOH, -SO₃H, alkoxycarbonyl, nitro, cyano and loweralkyl. In addition, nitrogen containing heterocycles can be N-protected.

The term "(heterocyclic)alkoxy" as used herein refers to a heterocyclic group as defined above appended to an alkoxy radical as defined above. Examples of (heterocyclic)alkoxy include 4-pyridylmethoxy, 2-pyridylmethoxy and the like.

The term "(heterocyclic)alkyl" as used herein refers to a heterocyclic group as defined above appended to a loweralkyl radical as defined above.

The term "heterocyclic(amino)" refers to R₇₇-NH- wherein R₇₇ is an aromatic heterocyclic group as defined above which is appended to an amino group. The aromatic heterocycle is substituted with substituents R₇₅ and R₇₆ which are both bonded to the atoms of the aromatic heterocycle which are directly appended to the nitrogen. R₇₅ and R₇₆ are substituents independently selected from hydroxy, halo, oxo (=O), alkylimino (R*N= wherein R* is a loweralkyl group), amino, alkylamino, dialkylamino, alkoxy, alkoxyalkoxy, haloalkyl, cycloalkyl, aryl, phenyl, arylalkyl, -COOH, -SO₃H, alkoxycarbonyl, nitro, cyano and loweralkyl. The aromatic heterocycle may also be optionally substituted with a third substituent which is selected from the group hydroxy, halo, oxo (=O), alkylimino (R*N= wherein R* is a loweralkyl group), amino, alkylamino, dialkylamino, alkoxy, alkoxyalkoxy, haloalkyl, cycloalkyl, aryl, phenyl, arylalkyl, -COOH, -SO₃H, alkoxycarbonyl, nitro, cyano and loweralkyl.

The term "heterocycliccarbonyloxyalkyl" as used herein refers to R₄₆-C(O)-O-R₄₇- wherein R₄₆ is a heterocyclic group and R₄₇ is an alkylene group.

The term "hydroxy" as used herein refers to -OH.

The term "hydroxyalkenyl" as used herein refers to an alkenyl radical to which is appended a hydroxy group.

The term "hydroxyalkoxy" as used herein refers to an alkoxy radical as previously defined to which is appended a hydroxy (-OH) group. Examples of hydroxyalkoxy include 3-hydroxypropoxy, 4-hydroxybutoxy and the like.

The term "hydroxyalkyl" as used herein refers to a loweralkyl radical to which is appended a hydroxy group.

The term "mercapto" as used herein refers to -SH.

The terms "methylenedioxy" and "ethylenedioxy" refer to one or two carbon chains attached to the parent molecular moiety through two oxygen atoms. In the case of methylenedioxy, a fused 5 membered ring is formed. In the case of ethylenedioxy, a fused 6 membered ring is formed. Methylenedixoy substituted on a phenyl ring results in the formation of a benzodioxolyl radical. 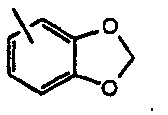 Ethylenedioxy substituted on a phenyl ring results in the formation of a benzodioxanyl radical

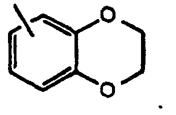

The term "substantially pure" as used herein means 95% or more of the specified compound.

The term "tetrazolyl" as used herein refers to a radical of the formula 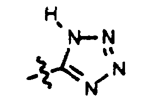 or a tautomer thereof.

The term "tetrazolylalkoxy" as used herein refers to a tetrazolyl radical as defined above appended to an alkoxy group as defined above. Examples of tetrazolylalkoxy include tetrazolylmethoxy, tetrazolylethoxy and the like.

The term "thioalkoxy" as used herein refers to R₇₀S- wherein R₇₀ is loweralkyl. Examples of thioalkoxy include, but are not limited to, methylthio, ethylthio and the like.

The term "thioalkoxyalkoxy" as used herein refers to R₈₀S-R₈₁O- wherein R₈₀ is loweralkyl as defined above and R₈₁ is alkylene. Representative examples of alkoxyalkoxy groups include CH₃SCH₂O-, EtSCH₂O-, t-BuSCH₂O- and the like.

The term "thioalkoxyalkoxyalkyl" as used herein refers to a thioalkoxyalkoxy group appended to an alkyl radical. Representative examples of alkoxyalkoxyalkyl groups include CH₃SCH₂CH₂OCH₂CH₂-, CH₃SCH₂OCH₂-, and the like.

The term *"trans,trans"* as used herein refers to the orientation of substituents (R₁ and R₂) relative to the central substituent R as shown

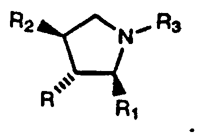

The term *"trans,cis"* as used herein refers to the orientation of substituents (R₁ and R₂) relative to the central substituent R as shown 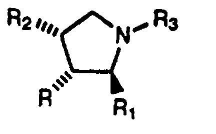 or 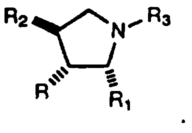 This definition encompasses both the case where R and R₂ are *cis* and R and R₁ are *trans* and the case where R₂ and R are *trans* and R and R₁ are *cis.*

The term *"cis,cis"* as used herein refers to the orientation of substituents (R₁ and R₂) relative to the central substituent R as shown

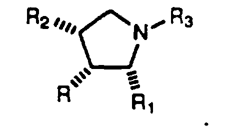

Representative compounds of the invention include:
*trans,trans-*2-(4-Methoxyphenyl)-4-(1,3-benzodioxol-5-yl)-1-((2,4,6-trimethyl)phenylaminocarbonylmethyl)-pyrrolidine-3-carboxylic acid,
*trans,trans*-2-(3-Fluoro-4-methoxyphenyl)-4-(1,3-benzodioxol-5-yl)-1-((2,4,6-trimethyl)phenylaminocarbonylmethyl)-pyrrolidine-3-carboxylic acid,
*trans,trans*-2-(4-Propoxyphenyl)-4-(1,3-benzodioxol-5-yl)-1-((2,4,6-trimethyl)phenylaminocarbonylmethyl)-pyrrolidine-3-carboxylic acid,
*trans,trans*-2-(4-Methoxyphenyl)-4-(1,3-benzodioxol-5-yl)-1-((2,6-diethyl)phenylaminocarbonylmethyl)-pyrrolidine-3-carboxylic acid,
*trans,trans*-2-(4-Propoxyphenyl)-4-(1,3-benzodioxol-5-yl)-1-((2,6-diethyl)phenylaminocarbonylmethyl)-pyrrolidine-3-carboxylic acid,
*trans,trans*-2-(3-Fluoro-4-methoxyphenyl)-4-(1,3-benzodioxol-5-yl)-1-((2,6-diethyl)phenylaminocarbonylmethyl)-pyrrolidine-3-carboxylic acid,
*trans,trans*-2-(3-Fluoro-4-ethoxyphenyl)-4-(1,3-benzodioxol-5-yl)-1-((2,6-diethyl)phenylaminocarbonylmethyl)-pyrrolidine-3-carboxylic acid,
*trans,trans*-2-(3-Fluoro-4-methoxyphenyl)-4-(7-methoxy-1,3-benzodioxol-5-yl)-1-((2,6-diethyl)phenylaminocarbonylmethyl)-pyrrolidine-3-carboxylic acid,
*trans,trans*-2-(3-methoxy-4-propoxyphenyl)-4-(1,3-benzodioxol-5-yl)-1-((2,6-diethylphenyl)aminocarbonylmethyl)-pyrrolidine-3-carboxylic acid,
*trans,trans*-2-(4-ethoxyphenyl)-4-(1,3-benzodioxol-5-yl)-1-((2,6-diethylphenyl)aminocarbonylmethyl)-pyrrolidine-3-carboxylic acid,
*trans,trans*-2-(4-methoxyphenyl)-4-(1,3-benzodioxol-5-yl)-1-((2,6-dimethylphenyl)aminocarbonylmethyl)-pyrrolidine-3-carboxylic acid,
*trans,trans*-2-(4-propoxyphenyl)-4-(7-methoxy-1,3-benzodioxol-5-yl)-1-((2,6-diethylphenyl)aminocarbonylmethyl)-pyrrolidine-3-carboxylic acid,
*trans,trans*-2-(3-methoxy-4-propoxyphenyl)-4-(7-methoxy-1,3-benzodioxol-5-yl)-1-((2,6-diethylphenyl)aminocarbonylmethyl)-pyrrolidine-3-carboxylic acid,
*trans,trans*-2-(4-methoxyphenyl)-4-(1,3-benzodioxol-5-yl)-1-((2,6-dibromo)phenylaminocarbonylmethyl)-pyrrolidine-3-carboxylic acid,
[*2R,3R,4S*]2-(4-Methoxyphenyl)-4-(1,3-benzodioxol-5-yl)-1-(N-(2,6-diethyl)phenylaminocarbonylmethyl)-pyrrolidine-3-carboxylic acid,
*trans,trans*-2-(4-methoxyphenyl)-4-(1,3-benzodioxol-5-yl)-1-((2,6-dimethoxy)phenylaminocarbonylmethyl)-pyrrolidine-3-carboxylic acid,
*trans,trans*-2-(4-methoxyphenyl)-4-(1,3-benzodioxol-5-yl)-1-((4-bromo-2,6-diethyl)phenylaminocarbonylmethyl)-pyrrolidine-3-carboxylic acid,
*trans,trans*-2-(4-methoxyphenyl)-4-(1,3-benzodioxol-5-yl)-1-((2-ethyl-6-methyl)phenylaminocarbonylmethyl)-pyrrolidine-3-carboxylic acid,
*trans,trans*-2-(4-methoxyphenyl)-4-(1,3-benzodioxol-5-yl)-1-((2,4,6-triethyl)phenylaminocarbonylmethyl)-pyrrolidine-3-carboxylic acid,
[*2R,3R,4S*]2-(4-Propoxyphenyl)-4-(1,3-benzodioxol-5-yl)-1-(N-(2,6-diethyl)phenylaminocarbonylmethyl)-pyrrolidine-3-carboxylic acid,
*trans,trans*-2-(4-methoxyphenyl)-4-(1,3-benzodioxol-5-yl)-1-((2,6-diisopropyl)phenylaminocarbonylmethyl)-pyrrolidine-3-carboxylic acid,
*trans,trans*-2-(4-methoxyphenyl)-4-(1,3-benzodioxol-5-yl)-1-((2,6-diethyl-4-methyl)phenylaminocarbonylmethyl)-pyrrolidine-3-carboxylic acid,
(2*R*,3*R*,4*S*)-2-(4-ethoxyphenyl)-4-(1,3-benzodioxol-5-yl)-1-((2,6-diethyl)phenylaminocarbonylmethyl)-pyrrolidine-3-carboxylic acid,
*trans,trans*-2-(4-methoxyphenyl)-4-(1,3-benzodioxol-5-yl)-1-((4-carboxy-2,6-diethyl)phenylaminocarbonylmethyl)-pyrrolidine-3-carboxylic acid,
*trans,trans*-2-(4-methoxyphenyl)-4-(1,3-benzodioxol-5-yl)-1-((4-nitro-2,6-diethyl)phenylaminocarbonylmethyl)-pyrrolidine-3-carboxylic acid,
*trans,trans*-2-(4-methoxyphenyl)-4-(1,3-benzodioxol-5-yl)-1-((2-isopropyl-6-methyl)phenylaminocarbonylmethyl)-pyrrolidine-3-carboxylic acid,
*trans,trans*-2-(4-Methoxyphenyl)-4-(1,3-benzodioxol-5-yl)-1-(N-(2-ethyl-6-methoxy)phenylaminocarbonylmethyl)-pyrrolidine-3-carboxylic acid,
or a pharmaceutically acceptable salt thereof.

Preferred compounds of the invention are selected from the group consisting of:
*trans,trans*-2-(4-Methoxyphenyl)-4-(1,3-benzodioxol-5-yl)-1-((2,6-diethyl)phenylaminocarbonylmethyl)-pyrrolidine-3-carboxylic acid;
*trans,trans*-2-(4-Propoxyphenyl)-4-(1,3-benzodioxol-5-yl)-1-((2,6-diethyl)phenylaminocarbonylmethyl)-pyrrolidine-3-carboxylic acid;
*trans,trans*-2-(3-Fluoro-4-methoxyphenyl)-4-(1,3-benzodioxol-5-yl)-1-((2,6-diethyl)phenylaminocarbonylmethyl)-pyrrolidine-3-carboxylic acid;
*trans,trans*-2-(3-Fluoro-4-ethoxyphenyl)-4-(1,3-benzodioxol-5-yl)-1-((2,6-diethyl)phenylaminocarbonylmethyl)-pyrrolidine-3-carboxylic acid;
*trans,trans*-2-(3-Fluoro-4-methoxyphenyl)-4-(7-methoxy-1,3-benzodioxol-5-yl)-1-((2,6-diethyl)phenylaminocarbonylmethyl)-pyrrolidine-3-carboxylic acid;
*trans,trans*-2-(3-methoxy-4-propoxyphenyl)-4-(1,3-benzodioxol-5-yl)-1-((2,6-diethyl)phenylaminocarbonylmethyl)-pyrrolidine-3-carboxylic acid;
*trans,trans*-2-(4-ethoxyphenyl)-4-(1,3-benzodioxol-5-yl)-1-((2,6-diethyl)phenylaminocarbonylmethyl)-pyrrolidine-3-carboxylic acid;
*trans,trans*-2-(4-propoxyphenyl)-4-(7-methoxy-1,3-benzodioxol-5-yl)-1-((2,6-diethyl)phenylaminocarbonylmethyl)-pyrrolidine-3-carboxylic acid
*trans,trans*-2-(3-methoxy-4-propoxyphenyl)-4-(7-methoxy-1,3-benzodioxol-5-yl)-1-((2,6-diethyl)phenylaminocarbonylmethyl)-pyrrolidine-3-carboxylic acid;
[*2R,3R,4S*]2-(4-Methoxyphenyl)-4-(1,3-benzodioxol-5-yl)-1-(N-(2,6-diethyl)phenylaminocarbonylmethyl)-pyrrolidine-3-carboxylic acid,
[*2R,3R,4S*]2-(4-Propoxyphenyl)-4-(1,3-benzodioxol-5-yl)-1-(N-(2,6-diethyl)phenylaminocarbonylmethyl)-pyrrolidine-3-carboxylic acid; and
(2*R*,3*R*,4*S*)-2-(4-ethoxyphenyl)-4-(1,3-benzodioxol-5-yl)-1-((2,6-diethyl)phenylaminocarbonylmethyl)-pyrrolidine-3-carboxylic acid,
or a pharmaceutically acceptable salt thereof.

Most preferred is the compound *trans,trans*-2-(4-Propoxyphenyl)-4-(1,3-benzodioxol-5-yl)-1-((2,6-diethyl)phenylaminocarbonylmethyl)-pyrrolidine-3-carboxylic acid;
[*2R,3R,4S*]2-(4-Methoxyphenyl)-4-(1,3-benzodioxol-5-yl)-1-(N-(2,6-diethyl)phenylaminocarbonylmethyl)-pyrrolidine-3-carboxylic acid;
[*2R,3R,4S*]2-(4-Propoxyphenyl)-4-(1,3-benzodioxol-5-yl)-1-(N-(2,6-diethyl)phenylaminocarbonylmethyl)-pyrrolidine-3-carboxylic acid; and
(2*R*,3*R*,4*S*)-2-(4-ethoxyphenyl)-4-(1,3-benzodioxol-5-yl)-1-((2,6-diethyl)phenylaminocarbonylmethyl)-pyrrolidine-3-carboxylic acid;
or a pharmaceutically acceptable salt thereof.

Methods for preparing the compounds of the invention are shown in Schemes I-VII.

Scheme I illustrates the general procedure for preparing the compounds of the invention when m is 0 and W is -CO₂H. A β-ketoester 1, where E is loweralkyl or a carboxy protecting group, is reacted with a nitro vinyl compound 2, in the presence of a base (for example, 1,8-diazabicyclo[5.4.0]undec-7-ene (DBU) or sodium ethoxide or sodium hydride and the like) in an inert solvent such as toluene, benzene, tetrahydrofuran or ethanol and the like. The condensation product 3 is reduced (for example, hydrogenation using a Raney nickel or platinum catalyst). The resulting amine cyclizes to give the dihydro pyrrole 4. Reduction of 4 (for example, sodium cyanoborohydride or catalytic hydrogenation and the like) in a THF solvent and the like gives the pyrrolidine compound 5 as a mixture of *cis-cis, trans,trans* and *cis,trans* products. Chromatographic separation removes the *cis-cis* isomer leaving a mixture of the *trans,trans* and *cis,trans* isomers which is further elaborated. The *cis-cis* isomer can be epimerized (for example, using sodium ethoxide in ethanol) to give the *trans,trans* isomer and then carried on as described below. The pyrrolidine nitrogen is (1) acylated or sulfonylated with R₃-X (R₃ is R₄-C(O)- or R₆-S(O)₂- and X is a leaving group such as a halide (Cl is preferred) or X taken together with R₄-C(O)- or R₆-S(O)₂- forms an activated ester including esters or anhydrides derived from formic acid, acetic acid and the like, alkoxycarbonyl halides, N-hydroxysuccinimide, N-hydroxyphthalimide, N-hydroxybenzotriazole, N-hydroxy-5-norbornene-2,3-dicarboxamide, 2,4,5-trichlorophenol and the like) or (2) alkylated with R₃-X where X is a leaving group (for example, X is a halide (for example, Cl, Br or I) or X is a leaving group such as a sulfonate (for example, mesylate, tosylate, triflate and the like)) in the presence of a base such as diisopropyl ethylamine or triethylamine and the like to give the N-derivatized pyrrolidine 6 which is still a mixture of *trans,trans* and *cis,trans* isomers. Hydrolysis of the ester 6 (for example, using a base such a sodium hydroxide in EtOH/H₂O) selectively hydrolyzes the *trans,trans* ester to give a mixture of 7 and 8, which are readily separated.

Many of the β-ketoester starting materials employed in the preparation of the compounds of the present inbvention are commercially available. They may also be prepared using the methods indicated in Scheme VIII. In the method of Scheme VIII(a), an aromatic, heteroaromatic, or α- quarternary methyl ketone is deprotonated (e.g., with sodium hydride or lithium diisopropylamide) and treated with a reagent capable of transferring a carboalkoxy group (e.g., diethyl carbonate, methyl chloroformate, or di-tert-butyldicarbonate). Alternatively, as shown in Scheme VIII(b), a carboxylic acid may be activated (e.g., with carbonyldiimidazole or oxalyl chloride) and treated with an acetate equivalent (e.g., ethyl lithioacetate, magnesium methylmalonate, or Meldrum's acid followed by thermal alcoholysis).

A preferred embodiment is shown in Schemes II and III. A benzoyl acetate 26 is reacted with a nitro vinyl benzodioxolyl compound 27 using 1,8-diazabicyclo[5.4.0]undec-7-ene (DBU) as the base in toluene to give compound 28. Catalytic hydrogenation using Raney nickel leads to reduction of the nitro group to an amine and subsequent cyclization to give the dihydropyrrole 29. The double bond is reduced with sodium cyanoborohydride to give the pyrrolidine compound 30 as a mixture of *cis-cis, trans,trans* and *cis,trans* isomers. Chromatography separates out the *cis-cis* isomer, leaving a mixture of the *trans,trans* and *cis,trans* isomers (31).

Scheme III illustrates the further elaboration of the *trans,trans* isomer. The mixture (31) of *trans,trans* and *cis,trans* pyrrolidines described in Scheme IV is reacted with Br-CH₂C(O)NHR₄ in acetonitrile in the presence of ethyldiisopropylamine to give the alkylated pyrrolidine compound 32, still as a mixture of *trans,trans* and *cis,trans* isomers. Sodium hydroxide in ethanol-water hydrolyzes the ethyl ester of the *trans,trans* compound but leaves the ethyl ester of the cis,trans compound untouched, thus allowing separation of the *trans,trans* carboxylic acid 33 from the *cis,trans* ester 34.

Scheme IV illustrates the preparation of compounds where W is other than carboxylic acid. Compound 55, which can be prepared by the procedures described in Scheme IV, is converted (for example, using peptide coupling condition, e.g. N-methylmorpholine, EDCI and HOBt, in the presence of ammonia or other amide forming reactions) to give carboxamide 56. The carboxamide is dehydrated (for example, using phosphorus oxychloride in pyridine) to give nitrile 57. Nitrile 57 under standard tetrazole forming conditions (sodium azide and triethylamine hydrochloride or trimethylsilylazide and tin oxide) is reacted to give tetrazole 58. Alternatively nitrite 57 is reacted with hydroxylamine hydrochloride in the presence of a base (for example, potassium carbonate, sodium carbonate, sodium hydroxide, triethylamine, sodium methoxide or NaH) in a solvent such as DMF, DMSO, or dimethylacetamide to give amidoxime 59. The amidoxime 59 is allowed to react with a methyl or ethyl chloroformate in a conventional organic solvent (such as, chloroform, methylene chloride, dioxane, THF, acetonitrile or pyridine) in the presence of a base (for example, triethylamine, pyridine, potassium carbonate and sodium carbonate) to give an O-acyl compound. Heating of the O-acyl amidoxime in an inert solvent (such as benzene, toluene, xylene, dioxane, THF, dichloroethane, or chloroform and the like) results in cyclization to compound 60. Alternatively reacting the amidoxime 59 with thionyl chloride in an inert solvent (for example, chloroform, dichloromethane, dixoane and THF and the like) affords the oxathiadiazole 61.

Scheme V illustrates a method for synthesizing pyrrolidines by an azomethine ylide type [3+2]-cycloaddition to an acrylate. General structures such as compound 70 are known to add to unsaturated esters such as 71 to provide pyrrolidines such as compound 72 (O. Tsuge, S. Kanemasa, K. Matsuda, Chem. Lett. 1131-4 (1983), O. Tsuge, S. Kanemasa, T. Yamada, K. Matsuda, J. Org. Chem. 52 2523-30 (1987), and S. Kanemasa, K. Skamoto, O. Tsuge, Bull. Chem. Soc. Jpn. 62 1960-68 (1989)). Silylimine 73 is reacted with acrylate 74 in the presence of trimethylsilyl triflate and tetrabutylammonium fluoride to give the desired pyrrolidine 75 as a mixture of isomers. This method can be modified to provide the N-acetamido derivatives directly by reacting 73 and 74 with the appropriate bromoacetamide (for example, dibutyl bromoacetamide) in the presence of tetrabutylammonium iodide and cesium fluoride to give compound 76.

Scheme VI illustrates a method for producing an enantiomerically pure pyrrolidine 80, which can be further elaborated on the pyrrolidine nitrogen. Intermediate racemic pyrrolidine ester 77 (for example, prepared by the procedure described in Scheme V) is Boc-nitrogen protected (for example, by treatment with Boc₂O) and then the ester is hydrolyzed (for example, using sodium or lithium hydroxide in ethanol and water) to give t-butyl carbamoyl pyrrolidine carboxylic acid 78. The carboxylic acid is converted to its (+)-α-methylbenzylamine salt, which can be recrystallized (for example from ethyl acetate and hexane or chloroform and hexane) to afford the diastereomerically pure salt. This diastereomerically pure salt can be neutralized (for example, with sodium carbonate or citric acid) to afford enantiomerically pure carboxylic acid 79. The pyrrolidine nitrogen can be deprotected (for example, using trifluoroacetic acid) and the ester reformed by the use of ethanolic hydrochloric acid to give salt 80. Alternatively one can use ethanolic HCI to cleave the protecting group and form the ester in one step. The pyrrolidine nitrogen can be further elaborated (for example, by treatment with the dibutyl amide of bromoacetamide in acetonitrile in the presence of diisopropylethylamine) to give optically active compound 81. The use of (-)-α-methylbenzylamine will give the opposite enantiomer.

A preferred process is shown in Scheme VII. Nitro vinyl compound (88) is reacted with beta-keto ester 89 in the presence of a base such as sodium ethoxide and the like or a trialkylamine such as triethylamine or diisopropylethylamine and the like or an amidine such as DBU and the like in an inert solvent such as THF, toluene, DMF, acetonitrile, ethyl acetate, isopropyl acetate or methylene chloride and the like at a temperature of from about 0° C to about 100° C for a period of time from about 15 minutes to overnight to give compound 90. Reduction of the nitro group followed by cyclization was effected for example by catalytic hydrogenation with a hydrogen pressure of from about atmospheric pressure to 300 p.s.i. over from about 1 hour to about 1 day of compound 90 in an inert solvent such as THF, ethyl acetate, toluene, ethanol, isopropanol, DMF or acetonitrile and the like, using a hydrogenation catalyst such as Raney nickel, palladium on carbon, a platinum catalyst, such as platinum oxide, platinum on carbon or platinum on alumina and the like, or a rhodium catalyst, such as rhodium on carbon or rhodium on alumina and the like, and the like affords intermediate nitrone 91a or a mixture of nitrone 91a and imine 91b. The reaction mixture comprising the nitrone or nitrone/imine mixture is treated with an acid such as trifluoroacetic acid or acetic acid or sulfuric acid or phosphoric acid or methanesulfonic acid and the like, and the hydrogenation is continued to give pyrrolidine compound 92 as the cis,cis-isomer. Epimerization at C-3 is effected by treatment of compound 92 with a base such as sodium ethoxide, potassium t-butoxide, lithium t-butoxide or potassium t-amyloxide and the like or a trialkylamine such as triethylamine or diisopropylethylamine and the like or an amidine such as DBU and the like in an inert solvent such as ethanol, ethyl acetate, isopropyl acetate, THF, toluene or DMF and the like at a temperature of from about -20° C to about 120° C to give the trans,trans compound 93. Compound 93 itself can optionally be resolved into enantiomers prior to reacting with X-R₃. The substantially pure (i.e., at least 95% of the desired isomer) optically active (+)-isomer of compound 93 is obtained by treatment of a mixture of the (+)-isomer and the (-)-isomer of 93 with S-(+)-mandelic acid, D-tartaric acid or D-dibenzoyl tartaric acid and the like in a solvent such as acetonitrile, ethyl acetate, isopropyl acetate, ethanol or isopropanol and the like. The (+)-isomer of 93 selectively crystallizes as the salt, leaving the (-)-isomer of 93 in solution. Alternatively, the substantially pure (i.e., at least 95% of the desired isomer) optically active (-)-isomer of compound 93 can be selectively crystallized by reaction of a mixture of the (+)-isomer and the (-)-isomer of 93 with L-tartaric acid, L-dibenzoyl tartaric acid or L-pyroglutamic acid and the like, leaving the desired (+)-isomer of compound 93 in solution.

Compound 93 (racemic or optically active) is reacted with X-R₃ (where X is a leaving group (for example, a halide or a sulfonate) and R₃ is as previously defined) using a base such as diisopropylethylamine, triethylamine, sodium bicarbonate or potassium carbonate and the like in an inert solvent such as acetonitrile, THF, toluene, DMF or ethanol and the like at a temperature of from about 0° C to about 100° C to give the intermediate ester 94. The ester can be isolated or converted *in situ* to the carboxylic acid (95) using hydrolysis conditions such as a base such as sodium hydroxide or lithium hydroxide or potassium hydroxide and the like in a solvent such as ethanol-water or THF-ethanol and the like.

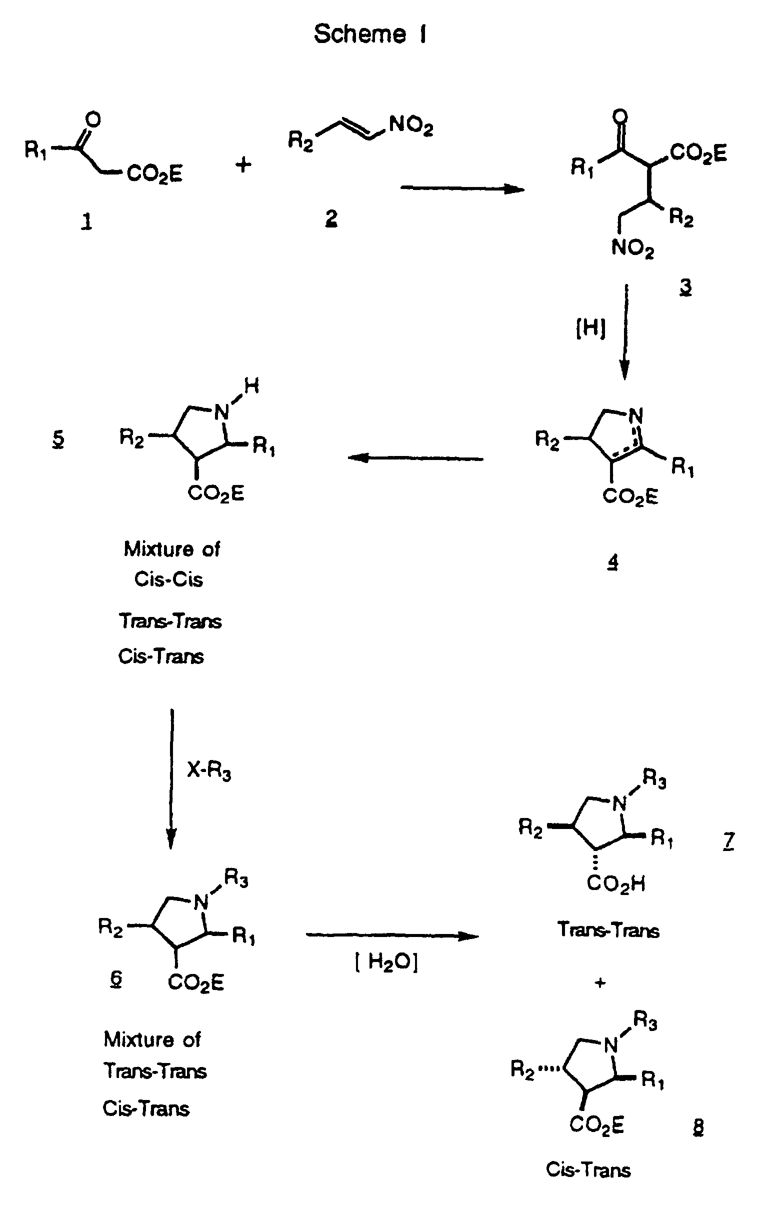

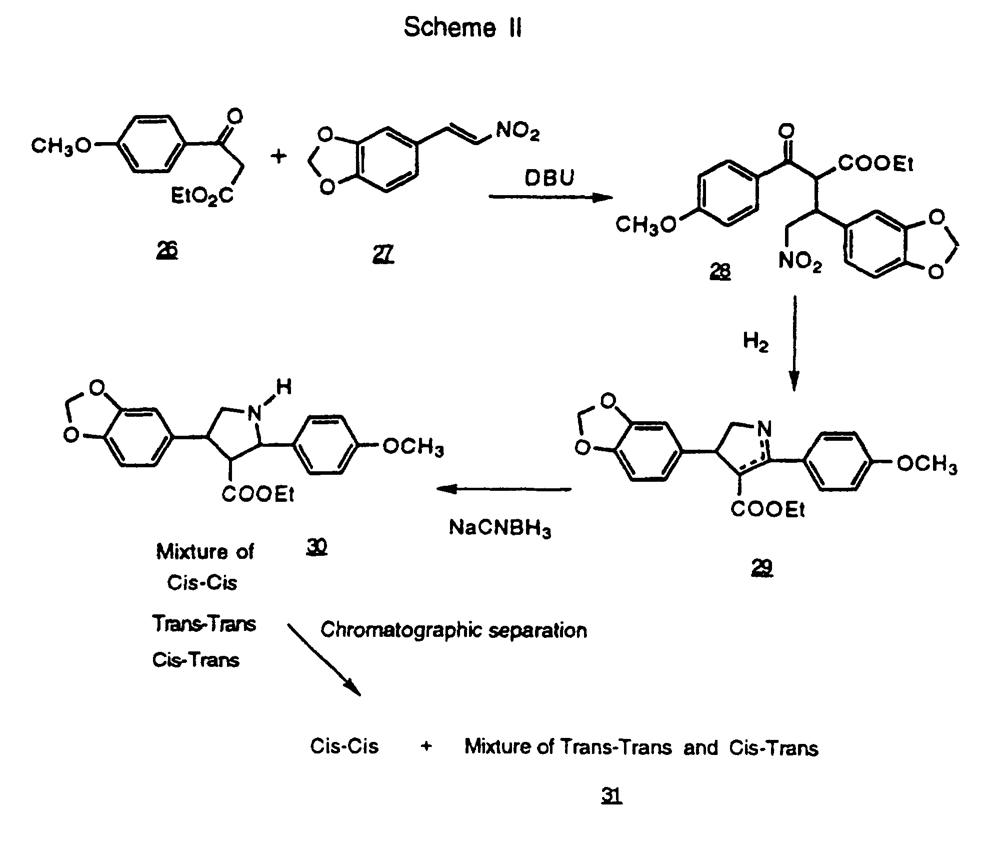

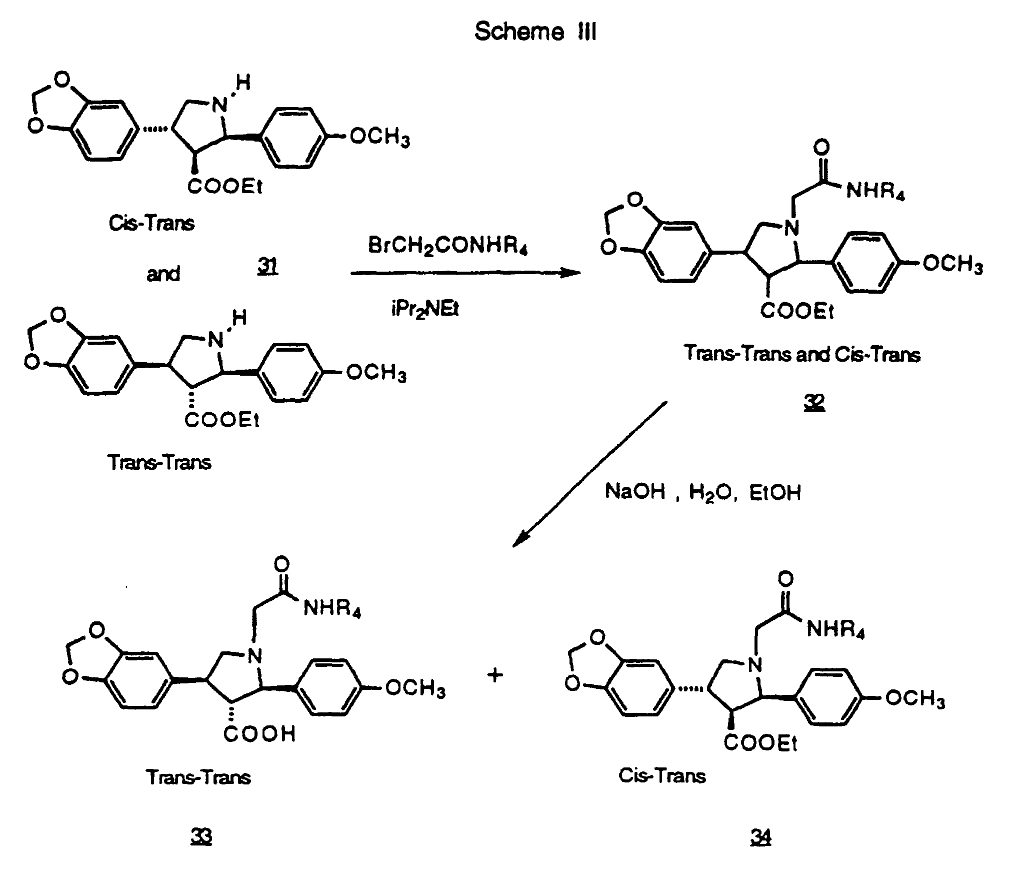

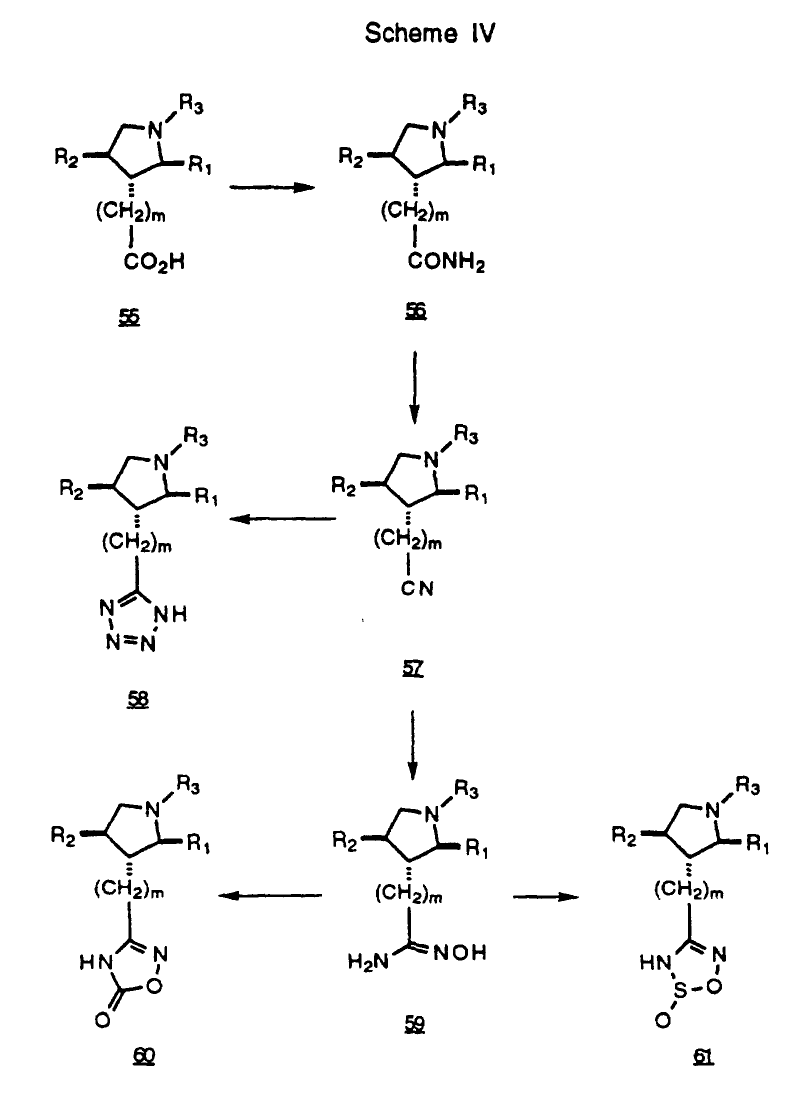

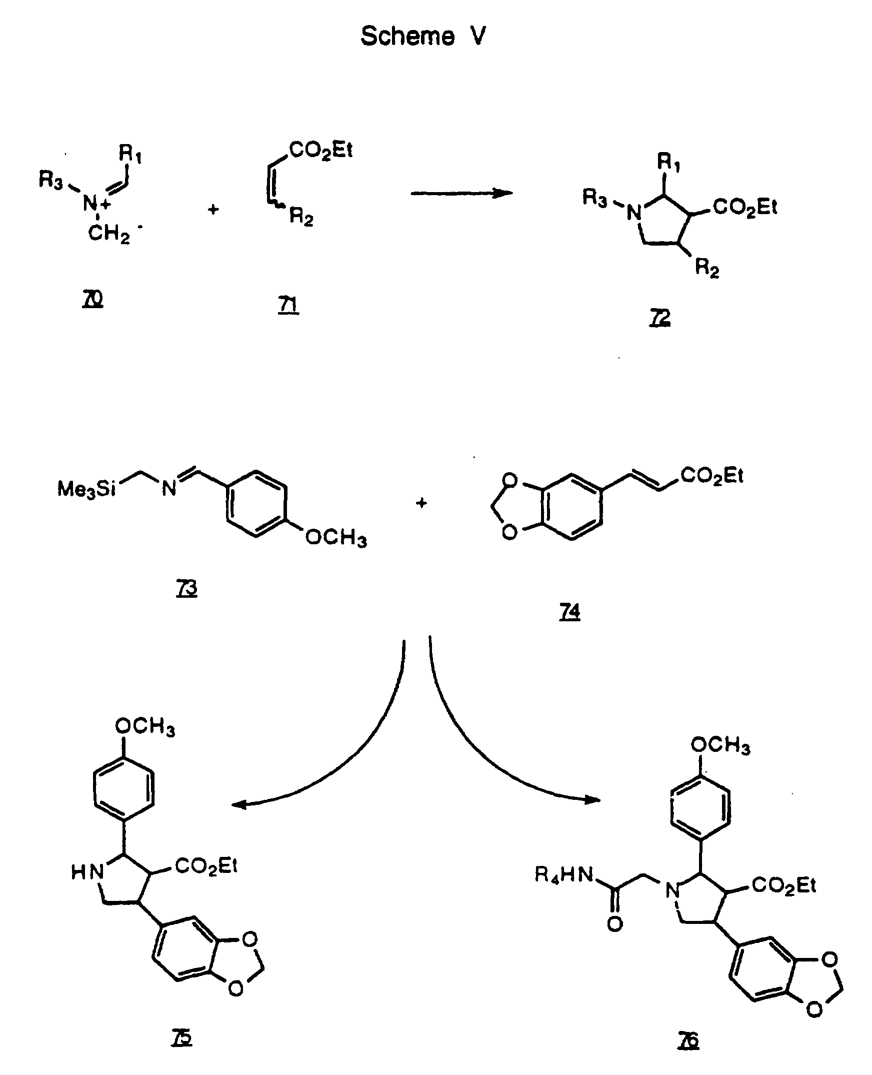

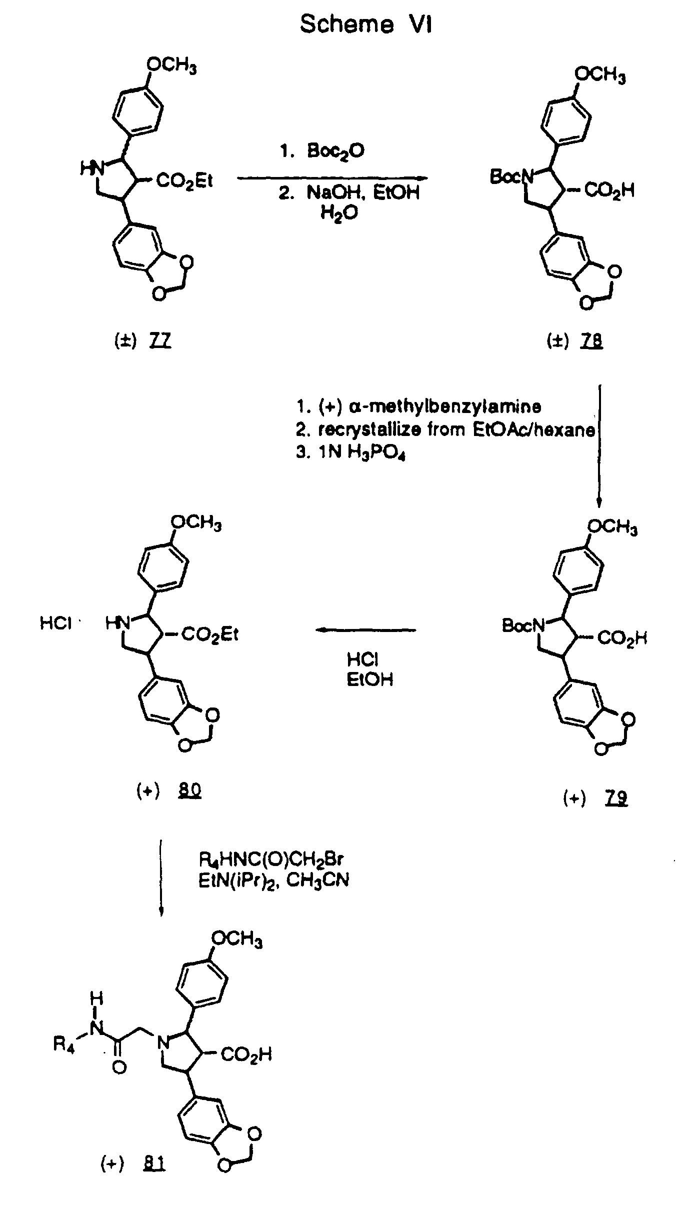

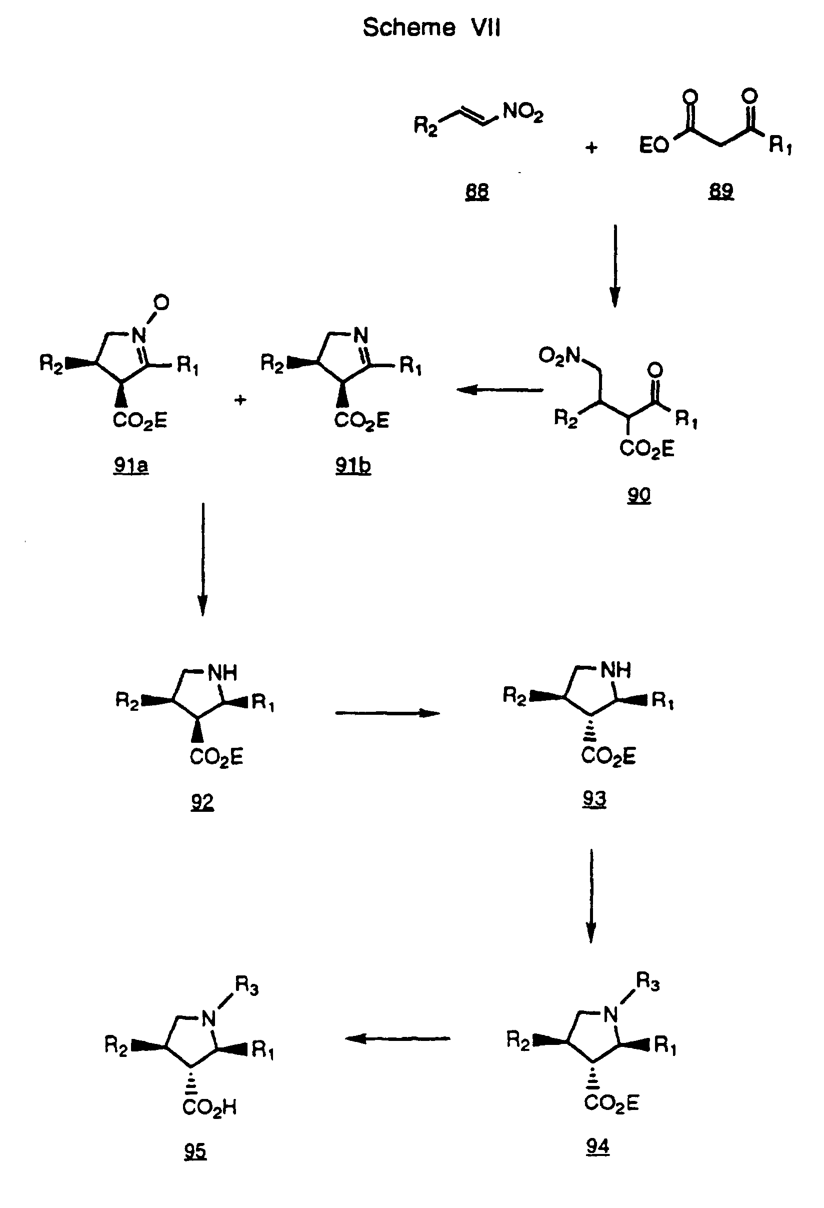

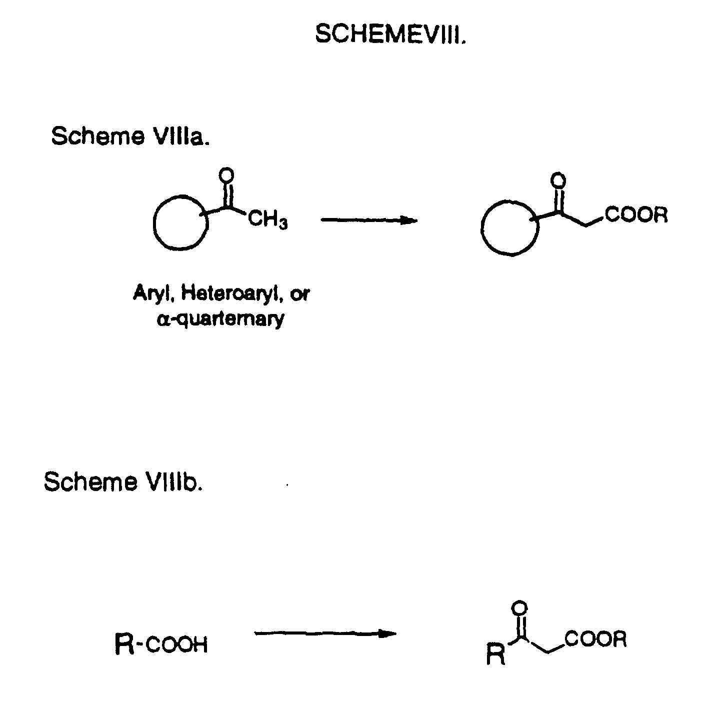

Compounds which are useful as intermediates for the preparation of compounds of the invention are: 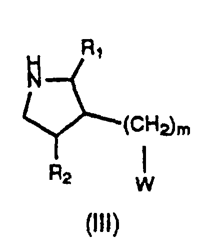 wherein
m is 0 to 6;
W is
(a) -C(O)₂-G where G is hydrogen or a carboxy protecting group,
(b) -PO₃H₂,
(c) -P(O)(OH)E where E is hydrogen, loweralkyl or arylalkyl,
(d) -CN,
(e) -C(O)NHR₁₇ where R₁₇ is loweralkyl,
(f) alkylaminocarbonyl,
(g) dialkylaminocarbonyl,
(h) tetrazolyl,
(i) hydroxy,
(j) alkoxy,
(k) sulfonamido,
(l) -C(O)NHS(O)₂R₁₆ where R₁₆ is loweralkyl, haloalkyl, phenyl or dialkylamino,
(m) -S(O)₂NHC(O)R₁₆,
(n) 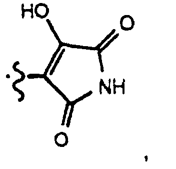
(o) 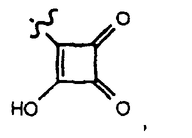
(p) 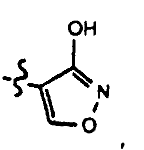
(q) 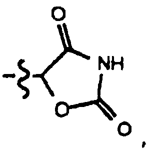
(r) 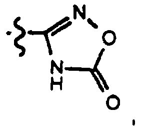
(s) 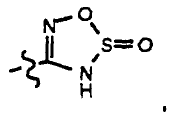
(t) 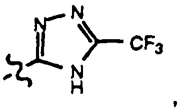 or
(u) 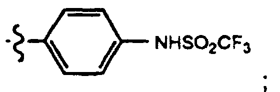 and
R₁ and R₂ are independently selected from hydrogen, loweralkyl, alkenyl, alkynyl, alkoxyalkyl, alkoxycarbonylalkyl, hydroxyalkyl, haloalkyl, haloalkoxyalkyl, alkoxyalkoxyalkyl, thioalkoxyalkoxyalkyl, cycloalkyl, cycloalkylalkyl, aminocarbonylalkyl, alkylaminocarbonylalkyl, dialkylaminocarbonylalkyl, aminocarbonylalkenyl, alkylaminocarbonylalkenyl, dialkylaminocarbonylalkenyl, hydroxyalkenyl, aryl, arylalkyl, aryloxyalkyl, arylalkoxyalkyl, heterocyclic, (heterocyclic)alkyl and
(Rₐₐ)(R_{bb})N-R_{cc}- wherein Rₐₐ is aryl or arylalkyl, R_{bb} is hydrogen or alkanoyl and R_{cc} is alkylene, with the proviso that one or both of R₁ and R₂ is other than hydrogen;
or a salt thereof;
and a compound of the formula: 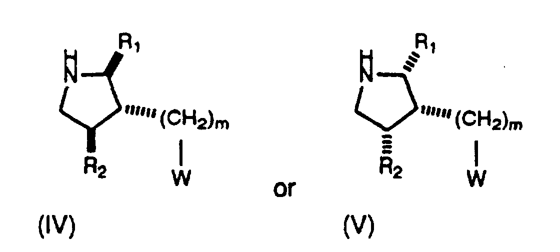 wherein n is 0 or 1;
m is 0 to 6;
W is
(a) -C(O)₂-G where G is hydrogen or a carboxy protecting group,
(b) -PO₃H₂,
(c) -P(O)(OH)E where E is hydrogen, loweralkyl or arylalkyl,
(d) -CN,
(e) -C(O)NHR₁₇ where R₁₇ is loweralkyl,
(f) alkylaminocarbonyl,
(g) dialkylaminocarbonyl,
(h) tetrazolyl,
(i) hydroxy,
(j) alkoxy,
(k) sulfonamido,
(I) -C(O)NHS(O)₂R₁₆ where R₁₆ is loweralkyl, haloalkyl, phenyl or dialkylamino,
(m) -S(O)₂NHC(O)R₁₆,
(n) 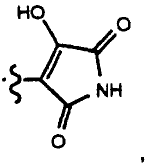
(o) 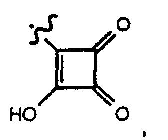
(p) 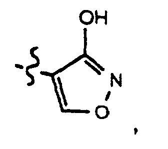
(q) 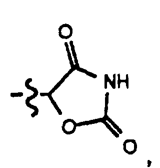
(r) 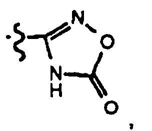
(s) 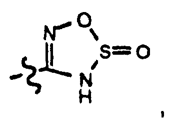
(t) 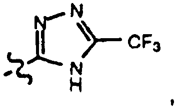 or
(u) 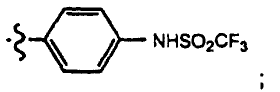 and
R₁ and R₂ are independently selected from hydrogen, loweralkyl, alkenyl, alkynyl, alkoxyalkyl, alkoxycarbonylalkyl, hydroxyalkyl, haloalkyl, haloalkoxyalkyl, alkoxyalkoxyalkyl, thioalkoxyalkoxyalkyl, cycloalkyl, cycloalkylalkyl, aminocarbonylalkyl, alkylaminocarbonylalkyl, dialkylaminocarbonylalkyl, aminocarbonylalkenyl, alkylaminocarbonylalkenyl, dialkylaminocarbonylalkenyl, hydroxyalkenyl, aryl, arylalkyl, aryloxyalkyl, arylalkoxyalkyl, heterocyclic, (heterocyclic)alkyl and
(Rₐₐ)(R_{bb})N-R_{cc}- wherein Rₐₐ is aryl or arylalkyl, R_{bb} is hydrogen or alkanoyl and R_{cc} is alkylene, with the proviso that one or both of R₁ and R₂ is other than hydrogen;
or a salt thereof.

Preferred intermediates include compounds of formula (III), (IV) and (V)
wherein
m is zero or 1;
W is -CO₂-G wherein G is hydrogen or a carboxy protecting group,
and R₁ and R₂ are as defined above; or
the substantially pure (+)- or (-)-isomer thereof.

Particularly preferred intermediates are compounds of formula (III), (IV) and (V) wherein
m is 0;
W is -CO₂-G wherein G is hydrogen or a carboxy protecting group;
and R₁ is (i) alkoxyalkylalkyl, (ii) cycloalkyl, (iii) phenyl, (iv) pyridyl, (v) furanyl or (vi) substituted or unsubstituted 4-methoxyphenyl, 4-fluorophenyl, 2-fluorophenyl, 4-trifluoromethylphenyl, 4-ethoxyphenyl, 3-fluoro-4-methoxyphenyl, 3-fluoro-4-ethoxyphenyl, 4-propoxyphenyl, 3-fluoro-4-propoxyphenyl, 3-methoxy-4-propoxyphenyl, 4-pentafluoroethylphenyl, 4-methoxymethoxyphenyl, 4-hydroxyphenyl, 1,3-benzodioxolyl, 7-methoxy-1,3-benzodioxolyl, 1,4-benzodioxanyl or dihydrobenzofuranyl wherein the substituent is selected from alkoxy, alkoxyalkoxy and carboxyalkoxy and R₂ is 1,3-benzodioxolyl, 1,4-benzodioxanyl, dihydrobenzofuranyl, benzofuranyl, 4-methoxyphenyl, dimethoxyphenyl, fluorophenyl or difluorophenyl; or
the substantially pure (+)- or (-)-isomer thereof.

The foregoing may be better understood by reference to the following examples which are provided for illustration and not intended to limit the scope of the inventive concept. The following abbreviations are used: Boc for tert-butyloxycarbonyl, Cbz for benzyloxycarbonyl, DBU for 1,8-diazabicyclo[5.4.0]undec-7-ene, EDCl for 1-(3-dimethylaminopropyl-3-ethylcarbodiimide hydrochloride, EtOAc for ethyl acetate, EtOH for ethanol, HOBt for 1-hydroxybenzotriazole, Et₃N for triethylamine, TFA for trifluoroacetic acid and THF for tetrahydrofuran.

### Example 1

### trans,trans-2-(4-Methoxyphenyl)-4-(1,3-benzodioxol-5-yl)-1-((2,4,6-trimethyl)phenylaminocarbonylmethyl)-pyrrolidine-3-carboxylic acid

### Example 1A

### Ethyl 2-(4-methoxybenzoyl)-4-nitromethyl-3-(1,3-benzodioxole-5-yl)butyrate

To ethyl (4-methoxybenzoyl)acetate (23.0 g, 0.104 mol), prepared by the method of Krapcho *et al*., Org. Syn. 47, 20 (1967), and 5-(2-nitrovinyl)-1,3-benzodioxole (17.0 g, 0.088 mol) dissolved in 180 mL of toluene and heated to 80 °C was added 1,8-diazabicyclo[5,4,0] undec-7-ene (DBU, 0.65 g) with stirring. The mixture was heated until all the nitro starting material dissolved. The solution was stirred without heating for 30 minutes and then an additional 0.65 g of DBU was added. After stirring an additional 45 minutes, thin layer chromatography (5% ethyl acetate in methylene chloride) indicated the absence of nitro starting material. Toluene (200 mL) was added, and the organic phase was washed with dilute hydrochloric acid and NaCI solution. The organic phase was dried over sodium sulfate and then concentrated under reduced pressure. The residue obtained was chromatographed on silica gel eluting with 3:1 hexane-ethyl acetate to give 21.22 g of the desired product as a mixture of isomers and 9.98 g. of recovered ethyl (4-methoxybenzoyl)acetate.

### Example 1B

### Ethyl 2-(4-methoxyphenyl)-4-(1,3-benzodioxol-5-yl)-4,5-dihydro-3H-pyrrole-3-carboxylate

The compound resulting from Example 1A (21 g) in 500 mL of ethanol was hydrogenated under 4 atmospheres of hydrogen pressure using a Raney nickel 2800 catalyst (51 g). (The Raney nickel was washed with ethanol three times before use.) The catalyst was removed by filtration, and the solution was concentrated under reduced pressure. The residue obtained was chromatographed on silica gel eluting with 8.5% ethyl acetate in methylene chloride to give 12.34 g of the desired product.

### Example 1C

### Ethyl 2-(4-methoxyphenyl-4-(1,3-benzodioxol-5-yl)-pyrrolidine-3-carboxylate) as a mixture of cis-cis: trans,trans; and cis,trans-isomers

The compound resulting from Example 1B (11.89 g, 0.324 mol) was dissolved in 27 mL of tetrahydrofuran and 54 mL of ethanol. Sodium cyanoborohydride (2.35 g, 0.374 mol) and 5 mg bromocresol green were added. To this blue solution was added dropwise a solution of 1:2 concentrated HCl in ethanol at such a rate that the color was kept at light yellow-green. After the yellow color persisted without additional HCl, the solution was stirred an additional 20 minutes. The solution was concentrated *in vacuo* and then partitioned between chloroform and an aqueous potassium bicarbonate solution. The organic phase was separated, dried over sodium sulfate, and concentrated under reduced pressure. The residue was chromatographed on silica gel eluting with 85:15 ethyl acetate-hexane to give 5.96 g. of a mixture of 64% *trans,trans*-compound and 34% *cis*,*trans*-compound. Further elution with pure ethyl acetate gave 0.505 g of an unknown solid followed by 3.044 g of pure *cis,cis*-compound.

### Example 1D

### N-(2,4,6-Trimethylphenyl) bromoacetamide

To a stirred solution of 2,4,6-trimethylaniline (1 g, 7.40 mmol) in methylene chloride (25 mL) at -50 °C was added successively *N,N*-diisopropylethylamine (1.58 mL, 8.14 mmol, 1.1 eq) and bromoacetyl bromide (0.72 mL, 7.40 mmol, 1 eq) such that the temperature did not exceed -40 °C. On completion of the addition, the cooling bath was removed, and the reaction mixture was allowed to warm to room temperature. After stirring for a further 30 minutes, the mixture was diluted with ether (70 mL) and poured into 1 N sodium bisulfate solution. The phases were separated, and the upper layer was washed successively with water and brine. The organic phase was dried (Na₂SO₄) and the solvent evaporated to half volume, at which point the product crystallized. The crystals were removed by vacuum filtration to afford the title compound (1.51 g, 80%).

### Example 1E

### trans,trans-2-(4-Methoxyphenyl)-4-(1,3-benzodioxol-5-yl)-1-((2,4,6-trimethyl)phenylaminocarbonylmethyl)-pyrrolidine-3-carboxylic acid

The mixture of 64% *trans,trans-* and 34% *cis,trans*-pyrrolidines (the mixture resulting from Example 1C) (5.72 g, 15.50 mmol), ethyldiisopropylamine (4.20 g, 32.56 mmol), and the compound resulting from Example 1D (19.0 mmol) in 30 mL of acetonitrile was heated at 50 °C for 1 hour. The solution was concentrated *in vacuo*. The residue was dissolved in toluene, shaken with potassium bicarbonate solution, dried over sodium sulfate and concentrated *in vacuo* to give the product as a mixture of *trans,trans-* and *cis,trans-* ethyl esters.

This mixture was dissolved in a solution of 50 mL of ethanol and 15 mL of water containing 5.00 g of sodium hydroxide and stirred for 3 hours at room temperature. The solution was concentrated *in vacuo* and 60 mL of water added. The mixture was extracted with ether to remove the unreacted *cis,trans-* ethyl ester. The aqueous phase was treated with hydrochloric acid until slightly cloudy. It was then further neutralized with acetic acid to give the crude acid product. The crude product was filtered and purified by dissolving it in tetrahydrofuran, drying over sodium sulfate, concentrating *in vacuo*, and crystallizing from ether to give the title compound. ¹H NMR (300MHz, CDCl₃) δ 8.22 (1H, bs), 7.78 (2H, d, *J*=8Hz), 6.95 (5H, m), 6.82 (1H, bd, *J*=8Hz), 6.77 (1H, d, *J*=8Hz), 5.96 (2H, s), 3.97 (1H, bd, *J*=10Hz), 3.81 (3H, s), 3.70 (1H, ddd, 6, 5&3Hz), 3.57 (bdd, 10&3Hz), 3.45 (1H, d, *J*=16Hz), 3.13 (2H, m), 2.24 (3H, s), 2.06 (6H, s). MS (DCl, NH₃) m/e 517 (M+H⁺). Anal. Calc for C₃₀H₃₂N₂O₆ · 0.5H₂O: C, 68.56, H, 6.33, N 5.33. Found: C, 68.84, H, 6.20, N, 5.31

### Example 2

### trans,trans-2-(3-Fluoro-4-methoxyphenyl)-4-(1,3-benzodioxol-5-yl)-1-((2,4,6-trimethyl)phenylaminocarbonylmethyl)-pyrrolidine-3-carboxylic acid

The title compound was prepared by the procedures described in Example 1. ¹H NMR (300MHz, CDCl₃) δ 8.22 (1H, bs), 7.21 (1H, dd, *J*=12&2Hz), 7.12 (1H, bd, *J*=8Hz), 6.95 (1H, t, 8Hz), 6.90 (2H, bs), 6.84 (1H, d, *J*=2Hz), 6.80 (1H, dd, *J*=8&3Hz), 6.76 (1H, d, *J*=8Hz), 5.93 (2H, s), 3.96 (1H, d, *J*=10Hz), 3.89 (3H, s), 3.70 (1H, ddd, 6, 5&3Hz), 3.56 (1H, dd, 11&5Hz), 3.45 (1H, d, *J*=16Hz), 3.10 (1H, t, *J*=10Hz), 3.07 (1H, dd, 8&6Hz), 3.02 (1H, d, *J*=16Hz), 2.17 (3H, s), 2.07 (6H, s). MS (DCI, NH₃) m/e 535 (M+H⁺). Anal. Calc for C₃₀H₃₁FN₂O₆ · 0.75H₂O : C, 65.74, H, 5.98, N 5.11. Found: C, 65.96, H, 5.88, N, 5.16

### Example 3

### trans,trans-2-(4-Propoxyphenyl)-4-(1,3-benzodioxol-5-yl)-1-((2,4,6-trimethyl)phenylaminocarbonylmethyl)-pyrrolidine-3-carboxylic acid

The title compound was prepared by the procedures described in Example 1. ¹H NMR (300MHz, CDCl₃) δ 8.21 (1H, bs), 7.38 (2H, d, *J*=8Hz), 6.90 (2H, d, *J*=8Hz), 6.89 (2H, d, 3Hz), 7.83 (1H, dd, *J*=8&2Hz), 6.75 (1H, d, *J*=8Hz), 5.94 (1H, d, *J*=3Hz), 5.93 (1H, d, *J*=3Hz), 3.96 (1H, d, *J*=10Hz), 3.85 (2H, q, *J*=7Hz), 3.70 (1H, ddd, 6, 5&3Hz), 3.58 (1H, dd, 11&5Hz), 3.48 (1H, d, *J*=16Hz), 3.15 (1H, dd, 8&6Hz), 3.13 (1H, t, *J*=10Hz), 2.99 (1H, d, *J*=16Hz), 2.25 (3H, s), 2.05 (6H, s), 1.81 (2H, sext, *J*=7Hz), 1.04 (3H, t, *J*=7Hz). MS (DCl, NH₃) m/e 545 (M+H⁺). Anal. Calc for C₃₂H₃₆N₂O₆ · 0.33H20: C, 69.79, H, 6.71, N 5.09, Found: C, 69.78, H, 6.73, N, 4.81

### Example 4

### trans,trans-2-(4-Methoxyphenyl)-4-(1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.24 (1H, bs), 7.39 (2H, d. *J*=8Hz), 7.21 (1H, dd, 8&6Hz), 7.11 (2H, d, *J*=8Hz), 6.92 (2H, d, 8Hz), 7.89 (1H, d, *J*=3Hz), 7.82 (1H, dd, *J*=8&2Hz), 6.75 (1H, d, *J*=8Hz), 5.94 (1H, d, *J*=3Hz), 5.93 (1H, d, *J*=3Hz), 3.96 (1H, d, *J*=10Hz), 3.82 (3H, s), 3.70 (1H, ddd, 6, 5&3Hz), 3.56 (1H, dd, 11&5Hz), 3.45 (1H, d, *J*=16Hz), 3.15 (1H, dd, 8&6Hz), 3.13 (1H, t, *J*=10Hz), 3.01 (1H, d, *J*=16Hz), 2.42 (4H, q, *J*=7Hz), 1.08 (6H, t, *J*=7Hz). MS (DCl, NH₃) m/e 559 (M+H₄⁺), 531 (M+H⁺). Anal. Calc for C₃₁H₃₄N₂O₆: C, 70.17, H, 6.46, N 5.28. Found: C, 69.88, H, 6.42, N, 5.09.

### Example 5

### trans,trans-2-(4-Propoxyphenyl)-4-(1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.27 (1H, bs), 7.37 (2H, d, *J*=8Hz), 7.21 (1H, dd, 8&6Hz), 7.11 (2H, d, *J*=8Hz), 6.90 (2H, d, 8Hz), 7.86 (1H, d, *J*=3Hz), 7.83 (1H, dd, *J*=8&2Hz), 6.75 (1H, d, *J*=8Hz), 5.93 (1H, d, *J*=3Hz), 5.92 (1H, d, *J*=3Hz), 3.96 (1H, d, *J*=10Hz), 3.85 (2H, q, *J*=7Hz), 3.70 (1H, ddd, 6, 5&3Hz), 3.55 (1H, dd, 11&5Hz), 3.48 (1H, d, *J*=16Hz), 3.15 (1H, dd, 8&6Hz), 3.13 (1H, t, *J*=10Hz), 3.01 (1H, d, *J*=16Hz), 2.43 (4H, q, *J*=7Hz), 1.82 (2H, sext, *J*=7Hz), 1.08 (6H, t, *J*=7Hz) 1.04 (3H, t, *J*=7Hz). MS (DCl, NH₃) m/e 559 (M+H⁺). Anal. Calc for C₃₃H₃₈N₂O₆ · 0.25H₂O: C, 70.38, H, 6.89, N 4.97. Found: C, 70.49, H, 6.85, N, 4.68.

### Example 6

### trans,trans-2-(3-Fluoro-4-methoxyphenyl)-4-(1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.21 (1H, bs), 7.22 (1H, dt, *J*=8&2Hz), 7.20 (1H, d, *J*=8Hz), 7.17 (1H, dt, *J*=8&2Hz), 7.10 (2H, d, *J*=8Hz), 6.96 (1H, t, *J*=8Hz), 6.83 (1H, dd, *J*=8&2Hz), 6.80 (1H, d, *J*=3Hz), 6.76 (1H, d, *J*=8Hz), 5.94 (1H, d, *J*=3Hz), 5.93 (1H, d, *J*=3Hz), 3.97 (1H, d, *J*=10Hz), 3.90 (3H, s), 3.72 (1H, ddd, 6, 5&3Hz), 3.58 (1H, dd, 11&5Hz), 3.46 (1H, d, *J*=16Hz), 3.14 (1H, t, *J*=10Hz), 3.12 (1H, dd, 8&6Hz), 3.05 (1H, d, *J*=16Hz), 2.45 (4H, q, *J*=7Hz), 1.09 (6H, t, *J*=7Hz). MS (DCI, NH₃) m/e 549 (M+H⁺). Anal. Calc for C₃₁H₃₃FN₂O₆ · 0.5H₂O: C, 66.78, H, 6.15, N 5.02. Found: C, 66.81, H, 5.89, N, 4.87.

### Example 7

### trans,trans-2-(3-Fluoro-4-ethoxyphenyl)-4-(1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.23 (1H, bs), 7.23 (1H, d, *J*=2Hz), 7.20 (1H, dd, *J*=8&3Hz), 7.11 (3H, m), 6.96 (1H, t, *J*=8Hz), 6.83 (1H, dd, *J*=8&2Hz), 6.80 (1H, d, *J*=3Hz), 6.76 (1H, d, *J*=8Hz), 5.93 (1H, d, *J*=3Hz), 5.92 (1H, d, J=3Hz), 4.11 (2H, t. *J*=7Hz), 3.97 (1H, d, *J*=10Hz), 3.72 (1H, ddd, 6, 5&3Hz), 3.55 (1H, dd, 11&5Hz), 3.47 (1H, d, *J*=16Hz), 3.14 (1H, t, *J*=10Hz), 3.12 (1H, dd, 8&6Hz), 3.04 (1H, d, *J*=16Hz), 2.45 (4H, q, *J*=7Hz), 1.47 (3H, t, *J*=7Hz), 1.09 (6H, t, *J*=7Hz). MS (DCl, NH₃) m/e 563 (M+H⁺). Anal. Calc for C₃₂H₃₅FN₂O₆ · 0.15TFA: C, 66.92, H, 6.11, N 4.83. Found: C, 67.19, H, 5.75, N, 4.69.

### Example 8

### trans,trans-2-(3-Fluoro-4-methoxyphenyl)-4-(7-methoxy-1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.24 (1H, s), 7.25 (1H, t, *J*=3Hz), 7.21 (1H, bd), 7.14 (1H, m), 7.08 (2H, d, *J*=8Hz), 6.96 (1H, t, *J*=8Hz), 6.56 (1H, d, *J*=3Hz), 6.50 (1H, d, *J*=3Hz), 5.93 (1H, d, *J*=2Hz), 5.91 (1H, d, *J*=2Hz), 3.97 (1H, d, *J*=10Hz), 3.90 (3H, s), 3.72 (1H, ddd, 6, 5&3Hz), 3.58 (1H, dd, 11&5Hz), 3.46 (1H, d, *J*=16Hz), 3.14 (1H, t, *J*=10Hz), 3.12 (1H, dd, 8&6Hz), 3.05 (1H, d, *J*=16Hz), 2.45 (4H, q, *J*=7Hz), 1.09 (6H, t, *J*=7Hz). MS (DCl, NH₃) m/e 579 (M+H⁺). Anal. calcd for C₃₂H₃₅FN₂O₇ · 1.5H₂O: C, 63.65, H, 6.31, N 4.64. Found: C, 64.00, H, 6.29, N, 4.26.

### Example 9

### trans,trans-2-(3-methoxy-4-propoxyphenyl)-4-(1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.22 (1H, s), 7.21 (1H, m), 7.12 (2H, d, *J*=10Hz), 7.02 (1H, dd, *J*=9&3Hz), 6.93 (1H, d, *J*=2Hz), 6.88 (1H, d, *J*=2Hz), 6.85 (1H, m), 6.82 (1H, d, *J*=2Hz), 6.75 (1H, d, *J*=9Hz), 5.95 (1H, d, *J*=2Hz), 5.93 (1H, d, *J*=2Hz), 3.97 (2H, q, *J*=9Hz), 3.84 (3H, s), 3.72 (2H, m), 3.60-3.45 (2H, m), 3.15 (2H, m), 3.03 (1H, d *J*=18Hz), 2.43 (4H, q, *J*=9Hz), 1.87 (2H, m), 1.08 (6H, t, *J*=9Hz), 1.04 (3H, t, *J*=9Hz). MS (DCl, NH₃) m/e 589 (MH⁺). Anal.calcd. for C₃₄H₄₀N₂O₇· 0.45 H₂O: C, 68.43, H, 6.91, N, 4.69. Found: C, 68.45, H, 6.91, N, 4.62.

### Example 10

### trans,trans-2-(4-ethoxyphenyl)-4-(1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.26 (1H, bs), 7.36 (2H, d, *J*=9Hz), 7.21 (1H, m), 7.11 (2H, d, *J*=10Hz), 6.90 (2H, d, *J*=9Hz), 6.86 (1H, d, *J*=2Hz), 6.83 (1H, dd, *J*=8&2Hz), 6.73 (1H, d, *J*=9Hz), 5.94 (1H, d, *J*=2Hz), 5.92 (1H, d, *J*=2Hz), 4.10-3.90 (3H, m), 3.71 (1H, m), 3.60-3.40 (2H, m), 3.15 (2H, m), 3.02 (1H, d *J*=18Hz), 2.43 (4H, q, *J*=9Hz), 1.42 (3H, t, *J*=9Hz), 1.08 (6H, t, *J*=9Hz). MS (DCI, NH₃) m/e 545 (MH⁺). Anal. calcd. for C₃₂H₃₆N₂O₆· 0.5 H₂O: C, 69.42, H, 6.74, N, 5.06. Found: C, 69.52, H, 6.52, N, 4.89.

### Example 11

### trans,trans-2-(4-methoxyphenyl)-4-(1,3-benzodioxol-5-yl)-1-((2,6-dimethyl)phenylaminocarbonylmethyl)-pyrrolidine-3-carboxylic acid

The title compound was prepared by the procedures described in Example 1. ¹H NMR (300MHz, CDCl₃) δ 8.32 (1H, bs), 7.37 (2H, d, *J*=9Hz), 7.08 (3H, m), 6.91 (2H, d, *J*=9Hz), 6.88 (1H, d, *J*=2Hz), 6.82 (1H, dd, *J*=8&2Hz), 6.75 (1H, d, *J*=9Hz), 5.95 (1H, d, *J*=2Hz), 5.93 (1H, d, *J*=2Hz), 3.95 (1H, d, *J*=10Hz), 3.81 (3H, s), 3.72 (1H, m), 3.55 (1H, dd, *J*=10&5Hz), 3.46 (1H, d *J*=18Hz), 3.13 (2H, m), 3.00 (1H, d, *J*=18Hz), 2.10 (6H, s). MS (DCI, NH₃) m/e 502 (MH⁺). Anal. calcd. for C₂₉H₃₀N₂O₆· 0.5 H₂O: C ,68.09, H, 6.11, N, 5.48. Found: C, 67.98, H, 6.02, N, 5.33.

### Example 12

### trans,trans-2-(4-propoxyphenyl)-4-(7-methoxy-1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.30 (1H, bs), 7.36 (2H, d, *J*=9Hz), 7.21 (1H, m), 7.09 (2H, d, *J*=10Hz), 6.91 (2H, d, *J*=9Hz), 6.59 (1H, d, *J*=2Hz), 6.51 (1H, d, *J*=2Hz), 5.93 (1H, d, *J*=2Hz), 5.91 (1H, d, *J*=2Hz), 3.93 (3H, m), 3.80 (3H, s), 3.72 (1H, m), 3.60-3.50 (2H, m), 3.15 (2H, m), 3.02 (1H, d *J*=18Hz), 2.43 (4H, q, *J*=9Hz), 1.82 (2H, m), 1.08 (6H, t, *J*=9Hz), 1.05 (3H, t, *J*=9Hz). MS (DCl, NH₃) m/e 589 (MH⁺). Anal. calcd. for C₃₄H₄₀N₂O₇· 0.25 H₂O: C, 68.84, H, 6.88, N, 4.72. Found: C, 68.80, H, 6.59, N, 4.52.

### Example 13

### trans,trans-2-(3-methoxy-4-propoxyphenyl)-4-(7-methoxy-1,3-benzodioxol-5-yl)-1-((2,6-diethyl)phenylaminocarbonylmethyl)-pyrrolidine-3-carboxylic acid

The title compound was prepared by the procedures described in Example 1. ¹H NMR (300MHz, CDCl₃) δ 8.22 (1H, s), 7.21 (1H, m), 7.09 (2H, d, *J*=10Hz), 7.02 (1H, dd, *J*=9&3Hz), 6.93 (1H, d, *J*=2Hz), 6.87 (1H, d, *J*=9Hz), 6.61 (1H, d, *J*=2Hz), 6.53 (1H, d, *J*=2Hz), 5.93 (1H, d, *J*=2Hz), 5.91 (1H, d, *J*=2Hz), 3.97 (3H, q, *J*=9Hz), 3.84 (3H, s), 3.82 (3H, s), 3.70 (1H, m), 3.60-3.45 (2H, m), 3.15 (2H, m), 3.02 (1H, d *J*=18Hz), 2.42 (4H, q, *J*=9Hz), 1.85 (2H, m), 1.08 (6H, t, *J*=9Hz), 1.05 (3H, t, *J*=9Hz). MS (DCI, NH₃) m/e 619 (MH⁺). Anal. calcd. for C₃₅H₄₂N₂O₈ : C, 67.94, H, 6.84, N, 4.53. Found: C, 67.65, H, 6.98, N, 4.44.

### Example 14

### trans,trans-2-(4-methoxyphenyl)-4-(1,3-benzodioxol-5-yl)-1-((2,6-dibromo)phenylaminocarbonylmethyl)-pyrrolidine-3-carboxylic acid

The title compound was prepared by the procedures described in Example 1. ¹H NMR (300MHz, CDCl₃) δ 8.58 (1H, bs), 7.58 (2H, d, *J*=9Hz), 7.40 (2H, bd, *J*=10Hz), 7.02 (1H, t, *J*=9Hz), 6.91 (2H, d, *J*=9Hz), 6.86 (1H, m), 6.76 (1H, d, *J*=9Hz), 5.93 (2H, s), 3.98 (1H, bd, *J*=10Hz), 3.81 (3H, s), 3.73 (2H, m), 3.55 (1H, bd, *J*=15Hz), 3.13 (2H, m), 3.01 (1H, bd, *J*=18Hz). MS (DCl, NH3) m/e 633 (MH⁺). Anal. calcd. for C₂₇H₂₄Br₂N₂O₆· 0.3 H₂O: C, 50.85, H, 3.89, N, 4.39. Found: C, 50.45, H, 3.48, N, 4.22.

### Example 15

### [2R,3R,4S]2-(4-Methoxyphenyl)-4-(1,3-benzodioxol-5-yl)-1-(N-(2,6-diethyl)phenylaminocarbonylmethyl)-pyrrolidine-3-carboxylic acid, hydrochloride salt

### Example 15A-E

### Alternative preparation of Ethyl trans-trans-2-(4-methoxyphenyl)-4-(1,3-benzodioxol-5-yl)-pyrrolidine-3-carboxylate

### Example 15A

### 5-(2-Nitrovinyl)-1,3-benzodioxole

To piperonal (15.55 kg, 103.5 mol) under mechanical stirring and under nitrogen was added ammonium acetate (13.4 kg, 173.8 mol), acetic acid (45.2 kg), and nitromethane (18.4 kg, 301.4 mol) sequentially. The mixture was warmed to 70 °C. After about 30 minutes, the yellow product began to crystallize. The reaction temperature was raised to 80 °C and stirred for about 10 hours until minimal piperonal remains. The somewhat thick reaction mixture was cooled to 10 °C and filtered. The precipitate was washed with acetic acid (2 x 8 kg) and then water (2 x 90 kg). The product was dried under a nitrogen purge and then in a vacuum oven at 50 °C for 2 days to afford 15.94 kg (80%) of the title compound as a bright yellow solid.

### Example 15B

### Ethyl (4-methoxybenzoyl)acetate

To potassium t-amylate (25 wt %, 50.8 kg, 99.26 mol) in toluene (15.2 kg) cooled to 5 °C under mechanical stirring and under nitrogen was added a mixture of 4-methoxyacetophenone (6.755 kg, 44.98 mol) and diethyl carbonate (6.40 kg, 54.18 mol) in toluene over 1 hour maintaining the temperature below 10 °C. The reaction mixture was heated to 60 °C for 8 hours until no 4-methoxyacetophenone was detected by HPLC. The mixture was cooled to 20 °C and quenched by adding to a mixture of acetic acid (8 kg) and water (90 kg) over 30 minutes while maintaining the temperature at <20 °C. The layers were separated, and the organic layer was washed with 5% sodium bicarbonate solution (41 kg) and concentrated to 14.65 kg. The temperature is maintained below 50 °C during the distillation. The yellow product concentrate was assayed by HPLC against an external standard and the yield was found to be 9.40 kg (94%).

### Example 15C

### Ethyl 2-(4-methoxybenzoyl)-4-nitromethyl-3-(1,3-benzodioxole-5-yl)butyrate

To the compound resulting from Example 15A (7.5 kg, 37.9 mol) suspended in THF (56 kg) with mechanical stirring under nitrogen was added the compound resulting from Example 15B (8.4 kg, 37.9 mol). The mixture was cooled to 17 °C, sodium ethoxide (6.4 g, 0.095 mol) was added, and the reaction was stirred for 30 minutes. After about 15 minutes, the nitrostyrene was completely dissolved. Sodium ethoxide (6.4 g, 0.095 mol) was added, and the mixture was stirred at 25 °C until HPLC shows less than 1 area % ketoester remaining. The reaction was concentrated to 32.2 kg which was determined by HPLC assay to be ~14.9 kg (95%).

### Example 15D

### Ethyl cis,cis-2-(4-methoxyphenyl)-4-(1,3-benzodioxol-5-yl)-pyrrolidine-3-carboxylate

Raney nickel (20.0 g), from which the water had been decanted, was charged to a stirred hydrogenator equipped with a thermocouple. THF (20 mL), the crude compound resulting from Example 15C (40.82 g, 0.0482 mol), and acetic acid (2.75 mL, 0.0482 mol) were added sequentially. The mixture was put under a hydrogen atmosphere at 60 psi until the hydrogen uptake slowed dramatically. TFA was added, and the mixture was hydrogenated at 200 psi until HPLC shows no residual imine and <2 area % nitrone. The catalyst was filtered away and washed with 100 mL of methanol. The filtrate was assayed by HPLC and found to contain 13.3 g (75% yield) of the cis, cis-pyrrolidine compound. The filtrate was concentrated and chased with additional THF (200 mL) to give a final volume of 100 mL. The mixture was neutralized with 2 N NaOH solution (50 mL), diluted with water (200 mL), and extracted with ethyl acetate (2 x 100 mL). The combined nearly colorless ethyl acetate layers were assayed against an external standard by HPLC to be13.0 g (73%) of the title compound.

### Example 15E

### Ethyl trans,trans-2-(4-methoxyphenyl)-4-(1,3-benzodioxol-5-yl)-pyrrolidine-3-carboxylate

The solution of the compound resulting from Example 15D (38.1 g, 0.103 mol) was chased with ethanol (200 mL) to a final volume of 100 mL and sodium ethoxide (3.40 g, 0.050 mol) was added. The mixture was heated to 75 °C. When HPLC shows <3% of the cis,cis isomer remaining, the mixture was cooled to room temperature. The product was assayed by HPLC against an external standard and found to contain 34.4 g (90% yield) of the title compound. The crude compound solution was concentrated and the residue taken up in isopropyl acetate (400 mL). The organic layer was washed with water (2 x 150 mL) and then extracted with 0.25 M phosphoric acid solution (2 x 400 mL). The combined phosphate layers were stirred with ethyl acetate (200 mL) and neutralized to pH 7 with solid sodium bicarbonate (21 g). The organic layer was separated and found to contain 32.9 g (87%) of the title compound.

### Example 15F

### [2R,3R,4S]2-(4-Methoxyphenyl)-4-(1,3-benzodioxol-5-yl)-pyrrolidine-3-carboxylic acid

The racemic amino ester from Example 1 (32.9 g) was dissolved in 50 mL of acetonitrile. (*S*)-(+)-Mandelic acid (2.06 g, 0.0136 mmol) was added and allowed to dissolve. The mixture was seeded with the product and allowed to stir at room temperature for 16 hours. The reaction mixture was cooled to 0 °C and stirred for 5 hours. The product was filtered and dried in a vacuum oven with a nitrogen purge for 1 day at 50 °C. The resultant salt (20.0 g, 0.0383 mol) was suspended in ethyl acetate (150 mL) and 5% sodium bicarbonate solution (150 mL). The mixture was stirred at room temperature until the salt dissolved and carbon dioxide evolution had ceased. The organic layer was separated and concentrated.

### Example 15G

### [2R,3R,4S]2-(4-Methoxyphenyl)-4-(1,3-benzodioxol-5-yl)-1-(N-(2,6-diethyl)phenylaminocarbonylmethyl)-pyrrolidine-3-carboxylic acid

The title compound was prepared from the compound of Example 15F according to the procedures of Example 1E.

### Example 15H

### [2R,3R,4S]2-(4-Methoxyphenyl)-4-(1,3-benzodioxol-5-yl)-1-(N-(2,6-diethyl)phenylaminocarbonylmethyl)-pyrrolidine-3-carboxylic acid, hydrochloride salt

The compound of Example 15G (450 mg) was dissolved in 10 mL of isopropanol. A slight excess of saturated HCI in ethanol was added, and the resultant solution was stirred for 10 min. The solvents were removed *in vacuo*, and the excess HCl was chased with isopropanol. The residue was taken up in ether and filtered, leaving 448 mg of the title compound as a white solid. MS (DCl/NH₃) m/e 531 (M+H)⁺. Anal calcd for C₃₁H₃₅N₂O₆Cl : C, 65.66 H, 6.22; N, 4.94. Found: C, 65.72; H, 6.39; N, 4.65.

### Example 16

### trans,trans-2-(4-methoxyphenyl)-4-(1,3-benzodioxol-5-yl)-1-((2,6-dimethoxy)phenylaminocarbonylmethyl)-pyrrolidine-3-carboxylic acid

### Example 16A

### 2,6-Dimethoxyaniline

To a stirred solution of 2,6-dimethoxybenzoic acid (2.00 g, 11.0 mmol) in 1,2-dichloroethane (45 mL) at ambient temperature was successively added N-methylmorpholine (1.45 mL, 13.2 mmol) and diphenylphosphoryl azide (2.60 mL, 12.1 mmol). After heating the mixture for 2 hours at 75 °C, cuprous iodide (150 mg) and benzyl alcohol (2.27 mL, 22.0 mmol) were added and heating was continued overnight. Solvents were removed *in vacuo* and the residue was chromatographed on silica gel, eluting with 4:1 hexane-ethyl acetate to give the intermediate carbamate (1.50 g, 48 % yield) as a white, crystalline solid. The solid was dissolved in methanol (15 mL) and added to a flask purged with nitrogen containing 10% palladium-on-charcoal (500 mg). The mixture was placed under a balloon of hydrogen and stirred 4 hours at ambient temperature. The mixture was filtered through a pad of Celite and solvents were removed *in vacuo* to give the title compound (800 mg, 48% yield).

### Example 16B

### trans,trans-2-(4-methoxyphenyl)-4-(1,3-benzodioxol-5-yl)-1-((2,6-dimethoxy)phenylaminocarbonylmethyl)-pyrrolidine-3-carboxylic acid

The title compound was prepared by the procedures described in Example 1, substituting the compound of Example 16A for 2,4,6-trimethylaniline in Example 1D. ¹H NMR (300MHz, CDCl₃) δ 8.18 (1H, bs), 7.39 (2H, bd, *J*=9Hz), 7.17 (1H, t, *J*=9Hz), 6.99 (1H, d, *J*=2Hz), 6.90 (2H, d, *J*=9Hz), 6.86 (1H, d, *J*=2Hz), 6.75 (1H, d, *J*=9Hz), 6.56 (2H, d, *J*=9Hz), 5.93 (2H, s), 3.88 (1H, bd, *J*=10Hz), 3.81 (3H, s), 3.71 (6H, s), 3.70 (2H, m), 3.49 (1H, bd, *J*=15Hz), 3.03 (2H, m), 2.85 (1H, bd, *J*=18Hz). NMR (DCI, NH₃) m/e 535 (MH⁺). Anal. calcd. for C₂₉H₃₀N₂O₈. 0.75 AcOH: C, 63.20, H, 5.74, N, 4.83. Found: C, 63.18, H, 5.34, N, 4.79.

### Example 17

### trans,trans-2-(4-methoxyphenyl)-4-(1,3-benzodioxol-5-yl)-1-((4-bromo-2,6-diethyl)phenylaminocarbonylmethyl)-pyrrolidine-3-carboxylic acid

### Example 17A

### 4-Bromo-2,6-diethylaniline

To a stirred solution of 2,6-diethylaniline (10.0 g, 67.0 mmol) in acetic acid (50 mL) at ambient temperature was added bromine (10.4 mL, 201 mmol). The reaction was stirred overnight at ambient temperature. The reaction mixture was diluted with diethyl ether (200 mL) and washed with 5% sodium bisulfite (4 x 50 mL) and brine. The organic phase was dried with sodium sulfate and the solvents were removed *in vacuo.* The residue was chromatographed on silica gel, eluting with 9:1 hexane-ethyl acetate to give the title compound (3.28 g, 21% yield).

### Example 17B

### trans,trans-2-(4-methoxyphenyl)-4-(1,3-benzodioxol-5-yl)-1-1-((4-bromo-2,6-diethyl)phenylaminocarbonylmethyl)-pyrrolidine-3-carboxylic acid

The title compound was prepared by the procedures described in Example 1, substituting the compound of Example 17A for 2,4,6-trimethylaniline in Example 1D. ¹H NMR (300MHz, CDCl₃) δ 8.21 (1H, bs), 7.38 (2H, d, *J*=9Hz), 7.23 (2H, s), 6.92 (2H, d, *J*=9Hz), 6.88 (1H, d, *J*=2Hz), 6.82 (1H, dd, *J*=8&2Hz), 6.75 (1H, d, *J*=9Hz), 5.93 (1H, d, *J*=2Hz), 5.91 (1H, d, *J*=2Hz), 3.95 (1H, d, *J*=9Hz), 3.82 (3H, s), 3.72 (1H, m), 3.52 (1H, m), 3.45 (1H, d *J*=18Hz), 3.14 (2H, m), 3.00 (1H, d, *J*=18Hz), 2.39 (4H, q, *J*=9Hz), 1.07 (6H, t, *J*=9Hz). MS (DCI, NH₃) m/e 609 (MH⁺). Anal. calcd. for C₃₁H₃₃BrN₂O₆ : C, 61.09, H, 5.46, N, 4.60. Found: C, 60.80, H, 5.35, N, 4.54.

### Example 18

### trans,trans-2-(4-methoxyphenyl)-4-(1,3-benzodioxol-5-yl)-1-((2-ethyl-6-methyl)phenylaminocarbonylmethyl)-pyrrolidine-3-carboxylic acid

The title compound was prepared by the procedures described in Example 1. ¹H NMR (300MHz, CDCl₃) δ 8.32 (1H, bs), 7.38 (2H, d, *J*=9Hz), 7.20-7.10 (3H, m), 6.92 (2H, d, *J*=9Hz), 6.87 (1H, d, *J*=2Hz), 6.82 (1H, dd, *J*=8&2Hz), 6.76 (1H, d, *J*=9Hz), 5.94 (1H, d, *J*=2Hz), 5.92 (1H, d, *J*=2Hz), 3.95 (1H, d, *J*=9Hz), 3.82 (3H, s), 3.73 (1H, m), 3.55 (1H, dd, J=12&6), 3.47 (1H, d *J*=18Hz), 3.14 (2H, m), 3.02 (1H, d, *J*=18Hz), 2.44 (2H, q, *J*=9Hz), 2.10 (3H, s), 1.10 (3H, t, *J*=9Hz). MS (DCl, NH₃) m/e 517 (MH⁺). Anal. calcd. for C₃₀H₃₂N₂O₆· 0.5 H₂O: C, 68.56, H, 6.33, N, 5.33. Found: C, 68.58, H, 6.29, N, 5.13.

### Example 19

### trans,trans-2-(4-methoxyphenyl)-4-(1,3-benzodioxol-5-yl)-1-((2,4,6-triethyl)phenylaminocarbonylmethyl)-pyrrolidine-3-carboxylic acid

### Example 19A

### Ethyl trans,trans-2-(4-methoxyphenyl)-4-(1,3-benzodioxol-5-yl)-1-((2,4,6-triethyl)phenylaminocarbonylmethyl)-pyrrolidine-3-carboxylate

To a mixture (purged with nitrogen) of [1,1'-bis(diphenylphosphino)ferrocene] dichloropalladium (II) (1:1 complex with dichloromethane) (13 mg) and cesium carbonate (307 mg, 0.942 mmol) in anhydrous N,N-dimethylformamide (2 mL) at ambient temperature was added ethyl *trans,trans*-2-(4-methoxyphenyl)-4-(1,3-benzodioxol-5-yl)-1-(4-bromo-2,6-diethylphenyl)aminocarbonylmethyl)-pyrrolidine-3-carboxylate (200 mg, 0.314 mmol, prepared in Example 17) in anhydrous tetrahydrofuran (8 mL). After stirring the mixture for 10 minutes at ambient temperature, 1.0 M triethylborane (0.471 mL, 0.471 mmol) in tetrahydrofuran was added. The reaction was stirred overnight at 65 °C under nitrogen. The reaction mixture was diluted with ethyl acetate (100 mL) and washed with water (2 x 30 mL) and brine. The organic phase dried with sodium sulfate, and the solvents were removed *in vacuo.* The residue was chromatographed on silica gel eluting with 3:1 hexane-ethyl acetate to give the title compound (110 mg, 60 % yield).

### Example 19B

### trans,trans-2-(4-methoxyphenyl)-4-(1,3-benzodioxol-5-yl)-1-((2,4,6-triethyl)phenylaminocarbonylmethyl)-pyrrolidine-3-carboxylic acid

The title compound was prepared by the procedures described in Example 1E. ¹H NMR (300MHz, CDCl₃) δ 8.22 (1H, bs), 7.38 (2H, d, *J*=9Hz), 6.95 (2H, s), 6.91 (2H, d, *J*=9Hz), 6.84 (1H, d, *J*=2Hz), 6.82 (1H, dd, *J*=8&2Hz), 6.75 (1H, d, *J*=9Hz), 5.93 (1H, d, *J*=2Hz), 5.91 (1H, d, *J*=2Hz), 3.95 (1H, d, *J*=10Hz), 3.82 (3H, s), 3.71 (1H, m), 3.52 (1H, dd, *J*=9&2Hz), 3.46 (1H, d *J*=18Hz), 3.13 (2H, m), 3.00 (1H, d, *J*=18Hz), 2.60 (2H, q, *J*=9Hz), 2.40 (4H, q, *J*=9Hz), 1.22 (3H, t, *J*=9Hz), 1.08 (6H, t, *J*=9Hz). MS (DCl, NH₃) m/e 559 (MH⁺). Anal. calcd. for C₃₃H₃₈N₂O₆ · 0.25 H₂O: C, 70.38, H, 6.89, N, 4.97. Found: C, 70.18, H, 7.14, N, 4.63.

### Example 20

### [2R,3R,4S]2-(4-Propoxyphenyl)-4-(1,3-benzodioxol-5-yl)-1-(N-((2,6-diethyl)phenylaminocarbonylmethyl)-pyrrolidine-3-carboxylic acid

### Example 20A

### [2R,3R,4S]2-(4-Propoxyphenyl)-4-(1,3-benzodioxol-5-yl)-1-tert-butoxycarbonylpyrrolidine-3-carboxylic acid

The racemic amino ester from Example 3 (8.00 g) was combined with 4.45 g of di-tert-butyldicarbonate in 100 mL of THF; 10 mL of triethylamine was added, and the resultant solution was stirred at ambient temperature for 3 hrs. The solvents were removed *in vacuo*; the residue was taken up in EtOAc and washed sequentially with aqueous 1 N H₃PO₄, bicarb, and brine. The crude product was dissolved in 30 mL of ethanol; 12 mL of 2.5 N NaOH solution was added, the mixture was stirred overnight at ambient temperature, then warmed to 50 °C for 2 hrs. The solvents were removed *in vacuo*; the residue was partitioned between water and ether. The aqueous extract was acidified with aqueous 1N H₃PO₄ and extracted twice with EtOAc. The organic extracts were washed with brine and dried over Na₂SO₄ to give 9.2 g of *trans,trans*-2-(4-propoxyphenyl)-4-(1,3-benzodioxol-5-yl)-1-tert-butoxycarbonyl-pyrrolidine-3-carboxylic acid. This material was dissolved in 30 mL of EtOAc, and 1.3 mL of (R)-(+)-α-methylbenzylamine was added. The solution was stirred for 10 min; the solvents were removed *in vacuo,* 50 mL of ether were added, and the resultant solution was seeded. After standing overnight, the solvents were removed in vacuo; the residue was taken up in 70 mL of ether and filtered. The solid product was recrystallized from EtOAc / ether. The crystalline material was stirred vigorously in a two-phase mixture of 1N H₃PO₄ and EtOAc; the organic layer was decanted, then washed with brine and dried over Na₂SO₄.

### Example 20B

### Ethyl[2R,3R,4S]2-(4-Propoxyphenyl)-4-(1,3-benzodioxol-5-yl)-pyrrolidine-3-carboxylate

The compound of Example 20A was dissolved in ethanol and cooled in an ice bath. Gaseous HCl was bubbled through the solution until saturated; the resultant solution was warmed to ambient temperature and allowed to stir overnight under a blanket of nitrogen. The solvents were removed *in vacuo*; the residue was taken up in bicarb and extracted with EtOAc. The organic layer was decanted, then washed with brine and dried over Na₂SO₄.

### Example 20C

### [2R,3R,4S]2-(4-Propoxyphenyl)-4-(1,3-benzodioxol-5-yl)-1-(N-(2,6-diethylphenyl)aminocarbonylmethyl)-pyrrolidine-3-carboxylic acid

The title compound was prepared from the compound of Example 20B according to the procedures of Example 1E. MS (DCl/NH₃) m/e 559 (M+H)⁺. Anal calcd for C₃₃H₃₈N₂O₆· 0.2 H₂O: C, 70.49; H, 6.88; N, 4.98. Found: C, 70.52; H, 6.78; N, 4.85.

### Example 21

### trans,trans-2-(4-methoxyphenyl)-4-(1,3-benzodioxol-5-yl)-1-((2,6-diisopropyl)phenylaminocarbonylmethyl)-pyrrolidine-3-carboxylic acid

The title compound was prepared by the procedures described in Example 1. ¹H NMR (300MHz, CDCl₃, δ) 8.29 (1H, bs), 7.39 (2H, d, *J*=9Hz), 7.29 (1H, m), 7.15 (2H, d, *J*=9Hz), 6.93 (2H, d, *J*=9Hz), 6.85 (1H, d, *J*=2Hz), 6.83 (1H, dd, *J*=8&2Hz), 6.74 (1H, d, *J*=9Hz), 5.93 (1H, d, *J*=2Hz), 5.91 (1H, d, *J*=2Hz), 3.96 (1H, d, *J*=10Hz), 3.83 (3H, s), 3.73 (1H, m), 3.55 (1H, dd, J=12&6), 3.50 (1H, d *J*=18Hz), 3.14 (2H, m), 3.01 (1H, d, *J*=18Hz), 2.84 (2H, m), 1.16 (6H, d, *J*=8Hz), 1.05 (6H, d *J*=8Hz). MS (DCl, NH₃) m/e 559 (MH⁺). Anal. calcd. for C₃₃H₃₈N₂O₆· 0.5 H₂O: C, 69.82, H, 6.92, N, 4.93. Found: C, 69.69, H, 6.63, N, 4.89.

### Example 22

### trans,trans-2-(4-methoxyphenyl)-4-(1,3-benzodioxol-5-yl)-1-((2,6-diethyl-4-methyl)phenylaminocarbonylmethyl)-pyrrolidine-3-carboxylic acid

The title compound was prepared by the procedures described in Example 19. ¹H NMR (300MHz, CDCl₃) δ 8.20 (1H, bs), 7.38 (2H, d, *J*=9Hz), 6.92 (4H, m), 6.86 (1H, d, *J*=2Hz), 6.82 (1H, dd, *J*=8&2Hz), 6.75 (1H, d, *J*=9Hz), 5.93 (1H, d, *J*=2Hz), 5.91 (1H, d, *J*=2Hz), 3.95 (1H, d, *J*=10Hz), 3.81 (3H, s), 3.72 (1H, m), 3.55 (1H, dd, *J*=9&2Hz), 3.45 (1H, d *J*=18Hz), 3.13 (2H, m), 3.00 (1H, d, *J*=18Hz), 2.39 (4H, q, *J*=9Hz), 2.28 (3H, s), 1.07 (6H, t, *J*=9Hz). NMR (DCI, NH₃) 545 m/e (MH⁺). Anal. calcd. for C₃₂H₃₆N₂O₆ · 0.5 H₂O: C, 69.42, H, 6.74, N, 5.06. Found: C, 69.43, H, 6.57, N, 4.94.

### Example 23

### (2R,3R,4S)-2-(4-ethoxyphenyl)-4-(1,3-benzodioxol-5-yl)-1-((2,6-diethyl)phenylaminocarbonylmethyl)-pyrrolidine-3-carboxylic acid

The title compound was prepared from the racemic amino ester of Example 10 according to the procedures of Example 20. ¹H NMR (300MHz, CDCl₃) δ 8.32 (1H, bs), 7.38 (2H, d, *J*=9Hz), 7.21 (1H, m), 7.12 (2H, d, *J*=10Hz), 6.90 (3H, m), 6.83 (1H, dd, *J*=8&2Hz), 6.74 (1H, d, *J*=9Hz), 5.94 (1H, d, *J*=2Hz), 5.92 (1H, d, *J*=2Hz), 4.05 (2H, m), 3.96 (1H, d, *J*=10Hz), 3.72 (1H, m), 3.53 (1H, dd, *J*=10&3Hz), 3.47 (1H, d, *J*=18Hz), 3.13 (2H, m), 3.02 (1H, d *J*=18Hz), 2.44 (4H, q, *J*=9Hz), 1.42 (3H, t, *J*=9Hz), 1.08 (6H, t, *J*=9Hz). MS (DCl, NH₃) m/e 545 (MH⁺). Anal. calcd. for C₃₂H₃₆N₂O₆· 0.5 H₂O: C, 69.42, H, 6.74, N, 5.06. Found: C, 69.67, H, 6.73, N, 4.98.

### Example 24

### trans,trans-2-(4-methoxyphenyl)-4-(1,3-benzodioxol-5-yl)-1-((4-carboxy-2,6-diethyl)phenylaminocarbonylmethyl)-pyrrolidine-3-carboxylic acid

The title compound was prepared by the procedures described in Example 19. ¹H NMR (300MHz, DMSO) δ 7.68 (2H, bs), 7.54 (2H, d, *J*=9Hz), 7.27 (2H, m), 6.93 (2H, d, *J*=9Hz), 6.83 (2H, m), 5.98 (2H, s), 3.92 (1H, d, *J*=9Hz), 3.76 (3H, s), 3.62 (1H, m), 3.45-3.00 (2H, m), 3.00-2.80 (3H, m), 2.44 (4H, q, *J*=9Hz), 1.04 (6H, t, *J*=9Hz). NMR (DCI, NH₃) m/e 575 (MH⁺). Anal. calcd. for C₃₂H₃₄N₂O₈· 0.5 H₂O: C, 65.85, H, 6.04, N, 4.80. Found: C, 66.03, H, 5.84, N, 4.67.

### Example 25

### trans,trans-2-(4-methoxyphenyl)-4-(1,3-benzodioxol-5-yl)-1-((4-nitro-2,6-diethyl)phenylaminocarbonylmethyl)-pyrrolidine-3-carboxylic acid

### Example 25A

### 2.6-Diethyl-4-nitroaniline

To a stirred solution of 2,6-diethylaniline (5.0 g, 34 mmol) in concentrated sulfuric acid (30 mL) at 0 °C was added dropwise concentrated nitric acid (15.9 M, 2.10 mL, 34 mmol). The cooling bath was removed, and the reaction was stirred for 3 hours at ambient temperature. After pouring the reaction mixture into ice, the solution was neutralized using 4 N sodium hydroxide and extracted with methylene chloride (3 x 50 mL). The extracts were dried with sodium sulfate, and solvents were removed *in vacuo* to give the title compound.

### Example 25B

### trans,trans-2-(4-methoxyphenyl)-4-(1,3-benzodioxol-5-yl)-1-((4-nitro-2,6-diethyl)phenylaminocarbonylmethyl)-pyrrolidine-3-carboxylic acid

The title compound was prepared by the procedures described in Example 1, substituting the compound of Example 25A for 2,4,6-trimethylaniline in Example 1D. ¹H NMR (300MHz, CDCl₃) δ 8.38 (1H, bs), 7.77 (1H, d, *J*=9Hz), 7.38 (2H, d, *J*=9Hz), 7.24 (1H, d, *J*=9Hz), 6.92 (2H, d, *J*=9Hz), 6.88 (1H, d, *J*=2Hz), 6.82 (1H, dd, *J*=8&2Hz), 6.75 (1H, d, *J*=9Hz), 5.93 (1H, d, *J*=2Hz), 5.91 (1H, d, *J*=2Hz), 3.97 (1H, d, *J*=9Hz), 3.83 (3H, s), 3.74 (1H, m), 3.48 (2H, m), 3.18 (2H, m), 3.04 (1H, d, *J*=18Hz), 2.63 (2H, m), 2.44 (2H, q, *J*=9Hz), 1.10 (3H, t, *J*=9Hz), 1.08 (3H, t, *J*=9Hz). MS (DCl, NH₃) m/e 576 (MH⁺). Anal. calcd. for C₃₁H₃₃N₃O₈· 0.75 H₂O: C, 63.20, H, 5.90, N, 7.13. Found: C, 63.30, H, 5.81, N, 7.14.

### Example 26

### trans,trans-2-(4-methoxyphenyl)-4-(1,3-benzodioxol-5-yl)-1-((2-isopropyl-6-methyl)phenylaminocarbonylmethyl)-pyrrolidine-3-carboxylic acid

The title compound was prepared by the procedures described in Example 1. ¹H NMR (300MHz, CDCl₃) δ 8.35 (1H, bs), 7.39 (2H, d, *J*=9Hz), 7.18 (2H, m), 7.07 (1H, dd, *J*=9&2Hz), 6.92 (2H, d, *J*=9Hz), 6.86 (1H, d, *J*=2Hz), 6.82 (1H, dd, *J*=8&2Hz), 6.75 (1H, d, *J*=9Hz), 5.94 (1H, d, *J*=2Hz), 5.92 (1H, d, *J*=2Hz), 3.96 (1H, d, *J*=10Hz), 3.83 (3H, s), 3.72 (1H, m), 3.50 (2H, m), 3.15 (2H, m), 3.02 (1H, d, *J*=18Hz), 2.86 (1H, m), 2.09 (3H, s), 1.16 (3H, d, *J*=8Hz), 1.07 (3H, d *J*=8Hz). MS (DCl, NH₃) m/e 531 (MH⁺). Anal. calcd. for C₃₁H₃₄N₂O₆· 0.5 H₂O: C, 69.00, H, 6.54, N, 5.19. Found: C, 69.27, H, 6.67, N, 5.21.

### Example 27

### trans,trans-2-(4-Methoxyphenyl)-4-(1,3-benzodioxol-5-yl)-1-(N-(2-ethyl-6-methoxy)phenylaminocarbonylmethyl)-pyrrolidine-3-carboxylic acid

### Example 27A

### Ethyl 3-oxo-4-(3-methoxy-2-nitrophenyl)-propionate

Potassium ethylmalonate (3.68 g) was combined with 2.29 g of magnesium chloride in 12 mL of DMF; the reaction mixture was heated at 60 °C for 4 hrs. The resultant mixture was cooled to ambient temperature. Simultaneously, 3-methoxy-2-nitrobenzoic acid (3.4 g) was dissolved in 12 mL of DMF; 3.06 g of 1,1-carbonyldiimidazole was added (gas evolves), and the resultant solution (after stirring at ambient temperature for 4 hrs) was added to the malonate mixture. The resultant slurry was stirred at ambient temperature for 14 hrs. Solvents were removed *in vacuo*; the residue was taken up in EtOAc and washed sequentially with 1N H₃PO₄, bicarb, and brine, and was concentrated *in vacuo.*

### Example 27B

### 2-Nitro-3-(1-hydroxyethyl)-anisole

The compound of Example 27A (3.2 g) was dissolved in 50 mL of concentrated sulfuric acid and sitrred at ambient temperature for 48 hrs. The reaction mixture was poured onto 300 mL of ice and extracted twice with EtOAc. The organic extracts were washed sequentially with water, bicarb, and brine, and were concentrated *in vacuo.* The crude product was heated neat at 160 °C for 3 hrs. The resultant dark brown residue was extracted with EtOAc. The organic extracts were concentrated. The crude product was dissolved in 15 mL of ethanol; sodium borohydride (450 mg) was added, and the resultant solution was stirred at ambient temperature for for 2 hrs. The solvents were removed *in vacuo*; the residue was taken up in 10% aqueous HCl and stirred for 15 min. The mixture was extracted with EtOAc; the organic extracts were washed sequentially with bicarb and brine, and were concentrated *in vacuo*. The crude product was purified by flash chromatography on silica gel, eluting with 1:1 EtOAc/hexanes, to give 1.08 g (32% overall) of the title compound as a colorless oil.

### Example 27C

### 2-Ethyl-6-methoxyaniline

The compound of Example 27B (310 mg) was dissolved in 10 mL of THF; 1.5 mL of H3PO4 was added, followed by 50 mg of 10% palladium-on-charcoal. The resultant mixture was purged with nitrogen, then placed under a balloon of hydrogen, and stirred overnight. Bicarb was added carefully, and the mixture was filtered through a pad of Celite. The filtrate was extracted with EtOAc; the organic extracts were washed with bicarb and brine, and were concentrated *in vacuo*. The crude product was purified by flash chromatography on silica gel, eluting with 1:1 ether/hexanes, to give 102 mg (43% yield) of the title compound as a colorless oil.

### Example 27D

### trans, trans-2-(4-Methoxyphenyl)-4-(1,3-benzodioxol-5-yl)-1-(N-(2-ethyl-6-methoxy)phenylaminocarbonylmethyl)-pyrrolidine-3-carboxylic acid

The title compound was prepared according to the procedures of Example , substituting the compound of Example 27C for 2,4,6-trimethylaniline. ¹H NMR (300 MHz, CD₃OD) δ 1.10 (t, J=8 Hz, 3H), 2.48 (d, J=8 Hz, 2H), 3.4-3.9 (m, 7H), 3.73 (s, 3H), 3.84 (s, 3H), 5.93 (s, 2H), 6.80 (d, J=8 Hz, 1H), 6.86 (d, J=8 Hz, 2H), 6.93 (dd, J=2,8 Hz, 1H), 7.03 (bd d, J=9 Hz, 2H), 7.07 (d, J=2 Hz, 1H), 7.23 (t, J=8 Hz, 1H), 7.53 (bd d, J=9 Hz, 2H). MS (APCI) m/e 533 (M+H)⁺. Anal calcd for C₃₀H₃₂N₂O₇ · 0.7 TFA: C, 61.59 H, 5.38; N, 4.57. Found: C, 61.27; H, 5.44; N, 4.61.

As an indication that the compounds described herein act through binding to endothelin receptors, the compounds have been evaluated for their ability to displace endothelin from its receptor.

### Binding Assay

### ET_{B} Receptor

### Preparation of membranes from Porcine cerebellum:

Porcine cerebellum was homogenized in 25 volumes (w/v) of 10 mM Hepes (pH 7.4) containing 0.25 M sucrose and protease inhibitors (3 mM EDTA, 0.1 mM PMSF, and 5 µg/ml Pepstatin A) by 3-10 sec polytron at 13,500 rpm with 10 sec intervals. The mixture was centrifuged at 1000xg for 10 min. The supernatant was collected and centrifuged at 30,000xg for 30 min. The precipitate was resuspended in Buffer A (20 mM Tris, 100 mM NaCl, 10 mM MgCl₂, pH 7.4) containing the aforementioned protease inhibitors and centrifuged again. The final pellet was resuspended in Buffer A containing protease inhibitors and stored at -80°C until used. Protein content was determined by the Bio-Rad dye-binding protein assay.

### [¹²⁵I]ET-3 binding to membranes:

Binding assays were performed in 96-well microtiter plates pretreated with 0.1% BSA. Membranes prepared from cells were diluted ∼100 fold in Buffer B (20 mM Tris, 100 mM NaCl, 10 mM MgCl₂, pH 7.4, with 0.2% BSA, 0.1 mM PMSF, 5 µg/mL Pepstatin A, 0.025% bacitracin, and 3 mM EDTA) to a final concentration of 0.2 mg/mL of protein. In competition studies, membranes (0.02 mg) were incubated with 0.1 nM of [¹²⁵I]ET-3 in Buffer B (final volume: 0.2 mL) in the presence of increasing concentrations of unlabeled ET-3 or a test compound for 4 hours at 25 °C. After incubation, unbound ligands were separated from bound ligands by a vacuum filtration method using glass-fiber filter strips in PHD cell harvesters (Cambridge Technology, Inc., MA), followed by washing the filter strips with saline (1 mL) for three times. Nonspecific binding was determined in the presence of 1 µM ET-1. The data are shown in Table 1. The per cent inhibition at a concentration of 1 µM is shown. The data show that the compounds of the invention bind to the endothelin receptor.

**Table 1**

| Binding Data | | | |
|---|---|---|---|
| Example | % Inhibition of ET_{B} at 1 µM | Example | % Inhibition of ETB at 1 µM |
| 1 | 96.4 | 2 | 91.5 |
| 3 | 82.1 | 4 | 94.0 |
| 5 | 96.5 | 6 | 92.9 |
| 7 | 94.5 | 8 | 93.6 |
| 9 | 94.8 | 10 | 95.2 |
| 11 | 96.0 | 12 | 96.7 |
| 13 | 91.3 | 14 | 96.6 |
| 15 | 93.4 | 16 | 92.3 |
| 17 | 97.1 | 18 | 94.9 |
| 19 | 94.9 | 20 | 95.5 |
| 21 | 97.1 | 22 | 95.3 |
| 23 | 99.1 | 24 | 93.3 |
| 25 | 95.7 | 26 | 98.0 |
| 27 | 98.8 | | |

The ability of the compounds of the invention to lower blood pressure can be demonstrated according to the methods described in Matsumura, et al., Eur. J. Pharmacol. 185 103 (1990) and Takata, et al., Clin. Exp. Pharmacol. Physiol. 10 131 (1983).

The ability of the compounds of the invention to treat congestive heart failure can be demonstrated according to the method described in Margulies, et al., Circulation 82 2226 (1990).

The ability of the compounds of the invention to treat myocardial ischemia can be demonstrated according to the method described in Watanabe, et al., Nature 344 114 (1990).

The ability of the compounds of the invention to treat coronary angina can be demonstrated according to the method described in Heistad, et al., Circ. Res. 54 711 (1984).

The ability of the compounds of the invention to treat cerebral vasospasm can be demonstrated according to the methods described in Nakagomi, et al., J. Neurosurg. 66 915 (1987) or Matsumura, et al., Life Sci. 49 841-848 (1991).

The ability of the compounds of the invention to treat cerebral ischemia can be demonstrated according to the method described in Hara et al., European. J. Pharmacol. 197: 75-82, (1991).

The ability of the compounds of the invention to treat acute renal failure can be demonstrated according to the method described in Kon, et al., J. Clin. Invest. 83 1762 (1989).

The ability of the compounds of the invention to treat chronic renal failure can be demonstrated according to the method described in Benigni, et al., Kidney Int. 44 440-444 (1993).

The ability of the compounds of the invention to treat gastric ulceration can be demonstrated according to the method described in Wallace, et al., Am. J. Physiol. 256 G661 (1989).

The ability of the compounds of the invention to treat cyclosporin-induced nephrotoxicity can be demonstrated according to the method described in Kon, et al., Kidney Int. 37 1487 (1990).

The ability of the compounds of the invention to treat endotoxin-induced toxicity (shock) can be demonstrated according to the method described in Takahashi, et al., Clinical Sci. 79 619 (1990).

The ability of the compounds of the invention to treat asthma can be demonstrated according to the method described in Potvin and Varma, Can. J. Physiol. and Pharmacol. 67 1213 (1989).

The ability of the compounds of the invention to treat transplant-induced atherosclerosis can be demonstrated according to the method described in Foegh, et al., Atherosclerosis 78 229-236 (1989).

The ability of the compounds of the invention to treat atherosclerosis can be demonstrated according to the methods described in Bobik, et al., Am. J. Physiol. 258 C408 (1990) and Chobanian, et al., Hypertension 15 327 (1990).

The ability of the compounds of the invention to treat LPL-related lipoprotein disorders can be demonstrated according to the method described in Ishida, et al., Biochem. Pharmacol. 44 1431-1436 (1992).

The ability of the compounds of the invention to treat proliferative diseases can be demonstrated according to the methods described in Bunchman ET and CA Brookshire, Transplantation Proceed. 23 967-968 (1991); Yamagishi, et al., Biochem. Biophys. Res. Comm. 191 840-846 (1993); and Shichiri, et al., J. Clin. Invest. 87 1867-1871 (1991). Proliferative diseases include smooth muscle proliferation, systemic sclerosis, cirrhosis of the liver, adult respiratory distress syndrome, idiopathic cardiomyopathy, lupus erythematosus, diabetic retinopathy or other retinopathies, psoriasis, scleroderma, prostatic hyperplasia, cardiac hyperplasia, restenosis following arterial injury or other pathologic stenosis of blood vessels.

The ability of the compounds of the invention to treat acute or chronic pulmonary hypertension can be demonstrated according to the method described in Bonvallet et al., Am. J. Physiol. 266 H1327 (1994). Pulmonary hypertension can be associated with congestive heart failure, mitral valve stenosis, emphysema, lung fibrosis, chronic obstructive pulmonary disease (COPD), acute repiratory distress syndrome (ARDS), altitude sickness, chemical exposure, or may be idiopathic.

The ability of the compounds of the invention to treat plaletet aggregation, and thrombosis, can be demonstrated according to the method described in McMurdo et al. Eu. J. Pharmacol. 259 51 (1994).

The ability of the compounds of the invention to treat cancers can be demonstrated according to the method described in Shichiri, et al., J. Clin. Invest. 87 1867 (1991).

The ability of the compounds of the invention to treat IL-2 (and other cytokine) mediated cardiotoxicity and vascular permeability disorders can be demonstrated according to the method described in Klemm et al., Proc. Nat. Acad. Sci. 92 2691 (1995).

The ability of the compounds of the invention to treat nociception can be demonstrated according to the method described in Yamamoto et al., J. Pharmacol. Exp. Therap. 271 156 (1994).

The ability of the compounds of the invention to treat colitis can be demonstrated according to the method described in Hogaboam et al (EUR. J. Pharmacol. 1996, 309, 261-269).

The ability of the compounds of the invention to treat ischemia-repurfusion injury in kidney transplantation can be demonstrated according to the method described in Aktan et al (Transplant Int 1996, 9, 201-207).

The ability of the compounds of the invention to treat angina, pulmonary hypertension, raynaud's disease, and migraine can be demonstrated according to the method described in Ferro and Webb (Drugs 1996, 51, 12-27).

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Basic addition salts can be prepared *in situ* during the final isolation and purification of the compounds of formula (I), or separately by reacting the carboxylic acid function with a suitable base such as the hydroxide, carbonate or bicarbonate of a pharmaceutically acceptable metal cation or with ammonia, or an organic primary, secondary or tertiary amine. Such pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like.

The compounds of the invention are useful for antagonizing endothelin in a human or other mammal. In addition, the compounds of the present invention are useful (in a human or other mammal) for the treatment of hypertension, acute or chronic pulmonary hypertension, Raynaud's disease, congestive heart failure, myocardial ischemia, reperfusion injury, coronary angina, cerebral ischemia, cerebral vasospasm, chronic or acute renal failure, non-steroidal antiinflammatory drug induced gastric ulceration, cyclosporin induced nephrotoxicity, endotoxin-induced toxicity, asthma, fibrotic or proliferative diseases, including smooth muscle proliferation, systemic sclerosis, cirrhosis of the liver, adult respiratory distress syndrome, idiopathic cardiomyopathy, lupus erythematosus, diabetic retinopathy or other retinopathies, psoriasis, scleroderma, prostatic hyperplasia, cardiac hyperplasia, restenosis following arterial injury or other pathologic stenosis of blood vessels, LPL-related lipoprotein disorders, transplantation-induced atherosclerosis or atherosclerosis in general, platelet aggregation, thrombosis, cancers, prostate cancer, IL-2 and other cytokine mediated cardiotoxicity and permeability disorders, and nociception, especially treatment of bone pain associated with bone cancer.

Total daily dose administered to a host in single or divided doses may be in amounts, for example, from 0.001 to 1000 mg/kg body weight daily and more usually 0.1 to 100 mg/kg for oral administration or 0.01 to 10 mg/kg for parenteral administration. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, sublingually, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be The title compound was prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The compounds of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically aceptable and metabolizable lipid capabale of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq.

A representative solid dosage form, for example, a tablet or a capsule, comprises:

| | |
|---|---|
| Compound of the invention: | 35% w/w |
| Starch, Pregelatinized, NF | 50% w/w |
| Microcrystalline Cellulose, NF | 10% w/w |
| Talc, Powder, USP | 5% w/w |

While the compounds of the invention can be administered as the sole active pharmaceutical agent, they can also be used in combination with one or more cardiovascular agents independently selected from diuretics, adrenergic blocking agents, vasodilators, calcium channel blockers, renin inhibitors, angiotensin converting enzyme (ACE) inhibitors, angiotensin II antagonists, potassium channel activators and other cardiovascular agents.

Representative diuretics include hydrochlorothiazide, chlorothiazide, acetazolamide, amiloride, bumetanide, benzthiazide, ethacrynic acid, furosemide, indacrinone, metolazone, spironolactone, triamterene, chlorthalidone and the like or a pharmaceutically acceptable salt thereof.

Representative adrenergic blocking agents include phentolamine, phenoxybenzamine, prazosin, terazosin, tolazine, atenolol, metoprolol, nadolol, propranolol, timolol, carteolol and the like or a pharmaceutically acceptable salt thereof.

Representative vasodilators include hydralazine, minoxidil, diazoxide, nitroprusside and the like or a pharmaceutically acceptable salt thereof.

Representative calcium channel blockers include amrinone, bencyclane, diltiazem, fendiline, flunarizine, nicardipine, nimodipine, perhexilene, verapamil, gallopamil, nifedipine and the like or a pharmaceutically acceptable salt thereof.

Representative renin inhibitors include enalkiren, zankiren, RO 42-5892, PD-134672 and the like or a pharmaceutically acceptable salt thereof.

Representative angiotensin II antagonists include DUP 753, A-81988 and the like.

Representative ACE inhibitors include captopril, enalapril, lisinopril and the like or a pharmaceutically acceptable salt thereof.

Representative potassium channel activators include pinacidil and the like or a pharmaceutically acceptable salt thereof.

Other representative cardiovascular agents include sympatholytic agents such as methyldopa, clonidine, guanabenz, reserpine and the like or a pharmaceutically acceptable salt thereof.

The compounds of the invention and the cardiovascular agent can be administered at the recommended maximum clinical dosage or at lower doses. Dosage levels of the active compounds in the compositions of the invention may be varied so as to obtain a desired therapeutic response depending on the route of administration, severity of the disease and the response of the patient. The combination can be administered as separate compositions or as a single dosage form containing both agents.

When administered as a combination, the therapeutic agents can be formulated as separate compositions which are given at the same time or different times, or the therapeutic agents can be given as a single composition.

The foregoing is merely illustrative of the invention and is not intended to limit the invention to the disclosed compounds, processes, compositions and methods. Variations and changes which are obvious to one skilled in the art are intended to be within the scope and nature of the invention which are defined in the appended claims.

## Claims

1. A compound of the formula: 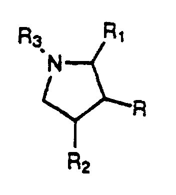 wherein
R is -(CH₂)ₘ-W wherein m is an integer from 0 to 6 and W is
(a) -C(O)₂-G wherein G is hydrogen or a carboxy protecting group which can be readily cleaved *in vivo*,
(b) -PO₃H₂,
(c) -P(O)(OH)E wherein E is hydrogen, alkyl or arylalkyl,
(d) -CN,
(e) -C(O)NHR₁₇ wherein R₁₇ is alkyl,
(f) alkylaminocarbonyl,
(g) dialkylaminocarbonyl,
(h) tetrazolyl,
(i) hydroxy,
(j) alkoxy,
(k) sulfonamido,
(I) -C(O)NHS(O)₂R₁₆ wherein R₁₆ is alkyl, haloalkyl, aryl or dialkylamino,
(m) -S(O)₂NHC(O)R₁₆ wherein R₁₆ is defined as above,
(n) 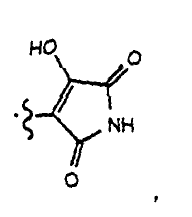
(o) 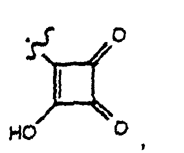
(p) 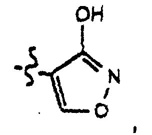
(q) 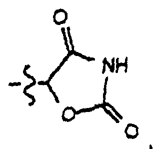
(r) 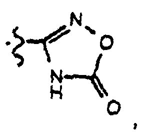
(s) 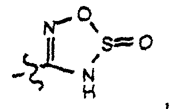
(t) 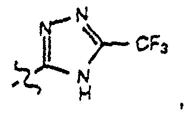 or
(u) 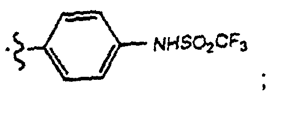
R₁ and R₂ are independently selected from hydrogen, alkyl, alkenyl, alkynyl, alkoxyalkyl, alkoxycarbonylalkyl, hydroxyalkyl, haloalkyl, haloalkoxyalkyl, alkoxyalkoxyalkyl, thioalkoxyalkoxyalkyl, cycloalkyl, cycloalkylalkyl, aminocarbonylalkyl, alkylaminocarbonylalkyl, dialkylaminocarbonylalkyl, aminocarbonylalkenyl, alkylaminocarbonylalkenyl, dialkylaminocarbonylalkenyl, hydroxyalkenyl, aryl, arylalkyl, aryloxyalkyl, arylalkoxyalkyl, heterocyclic, (heterocyclic)alkyl and
(Rₐₐ)(R_{bb})N-R_{cc}- wherein Rₐₐ is aryl or arylalkyl, R_{bb} is hydrogen or alkanoyl, and R_{cc} is alkylene, with the proviso that one or both of R₁ and R₂ is other than hydrogen;
R₃ is R₄-C(O)-R₅- or R₆-S(O)₂-R₇- wherein R5 is (i) a covalent bond, (ii) alkylene, (iii) alkenylene, (iv) -N(R₂₀)-R₈- or -R₈ₐ-N(R₂₀)-R₈- wherein R₈ and R₈ₐ are independently selected from the group consisting of alkylene and alkenylene and
R₂₀ is hydrogen, alkyl, alkenyl, haloalkyl, alkoxyalkyl, haloalkoxyalkyl, cycloalkyl or cycloalkylalkyl or (v) -O-R₉- or -R₉ₐ-O-R₉- wherein R₉ and R₉ₐ are independently selected from alkylene;
R₇ is (i) a covalent bond, (ii) alkylene, (iii) alkenylene or (iv) -N(R₂₁)-R₁₀- or -R₁₀ₐ-N(R₂₁)-R₁₀-
wherein R₁₀ and R₁₀ₐ are independently selected from the group consisting of alkylene and alkenylene and
R₂₁ is hydrogen, alkyl, alkenyl, haloalkyl, alkoxyalkyl, haloalkoxyalkyl, aryl or arylalkyl;
wherein R₄ and R₆ are 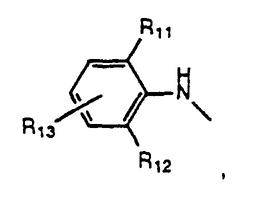 wherein R₁₁ and R₁₂ are independently selected from the group consisting of alkyl, cyano, alkoxy, halo, haloalkyl and phenyl and R₁₃ is hydrogen, alkyl, halo, carboxy, nitro or cyano;
wherein alkyl by itself or as part of another group has independently at each occurrence from 1 to 15 carbon atoms, alkenyl by itself or as part of another group has independently at each occurrence from 2 to 15 carbon atoms, alkynyl has from 2 to 15 carbon atoms, alkylene has independently at each occurrence from 1 to 15 carbon atoms, alkenylene has independently at each occurrence from 2 to 15 carbon atoms, cycloalkyl by itself or as part of another group has independently at each occurrence 3 to 10 carbon atoms and may be optionally substituted with from one to three substituents independently selected from alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, aryl by itself or as part of another group is independently at each occurrence a mono- or bicyclic carbocyclic ring system having one or two aromatic rings and may be optionally substituted with from one to three substituents independently selected from alkyl, halo, haloalkyl, haloalkoxy, hydroxyalkyl, alkenyloxy, alkoxy, alkoxyalkoxy, alkoxycarbonyl, alkoxycarbonylalkenyl, (alkoxycarbonyl)thioalkoxy, thioalkoxy, amino, alkylamino, dialkylamino, aminocarbonyl, aminocarbonylalkoxy, alkanoylamino, arylalkoxy, aryloxy, mercapto, cyano, nitro, carboxaldehyde, carboxy, carboxyalkenyl, carboxyalkoxy, alkylsulfonylamino, cyanoalkoxy, (heterocyclic)alkoxy, hydroxy, hydroxyalkoxy, phenyl and tetrazolylalkoxy or optionally substituted aryl may be tetrafluorophenyl or pentafluorophenyl, heterocyclic by itself or as part of another group is independently at each occurrence a 3- or 4-membered ring containing a heteroatom selected from oxygen, nitrogen and sulfur or is a 5-, 6- or 7-membered ring containing one, two or three nitrogen atoms-or one oxygen atom or one sulfur atom or one nitrogen and one sulfur atom or one nitrogen or one oxygen atom or two oxygen atoms in non-adjacent positions or one oxygen and one sulfur atom in non-adjacent positions or two sulfur atoms in non-adjacent positions wherein the 5-membered ring has 0-2 double bonds and the 6- and 7-membered rings have 0-3 double bonds and the nitrogen heteroatoms may be quaternized, or heterocyclic may be a bicyclic group in which any of the above heterocyclic rings is fused to a benzene ring or a cyclohexane ring or another of the above heterocyclic rings, wherein heterocyclic may be optionally substituted by one, two or three groups independently selected from the group consisting of hydroxy, halo, oxo, alkylimino, amino, alkylamino, dialkylamino, alkoxy, alkoxyalkoxy, haloalkyl, cycloalkyl, aryl, phenyl, arylalkyl, -COOH, -SO₃H, alkoxycarbonyl, nitro, cyano and alkyl;
or a pharmaceutically acceptable salt thereof.

2. The compound according to Claim 1 of the formula: 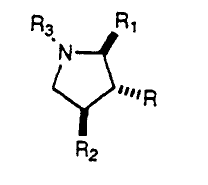 wherein R, R₁, R₂ and R₃ are as defined therein, or a pharmaceutically acceptable salt thereof.

3. The compound according to Claim 1 or 2 wherein R₃ is R₄-C(O)-R₅- wherein R₄ is as defined therein and R₅ is alkylene or R₃ is R₆-S(O)₂-R₇- wherein R₇ is alkylene and R₆ is as defined therein.

4. The compound according to Claim 3 wherein R is -C(O)₂-G wherein G is hydrogen or a carboxy protecting group or R is tetrazolyl or R is -C(O)-NHS(O)₂R₁₆ wherein R₁₆ is alkyl, haloalkyl or aryl, and R₁ and R₂ are independently selected from (i) alkyl, (ii) cycloalkyl, (iii) substituted aryl wherein aryl is phenyl substituted with one, two or three substituents independently selected from alkyl, alkoxy, halo, alkoxyalkoxy and carboxyalkoxy and (iv) substituted or unsubstituted heterocyclic.

5. The compound according to Claim 3 wherein R is -C(O)₂-G
wherein G is hydrogen or a carboxy protecting group or R is tetrazolyl or R is -C(O)-NHS(O)₂R₁₆ wherein R₁₆ is alkyl, haloalkyl or aryl, R₁ is (i) alkoxyalkyl, (ii) cycloalkyl, (iii) phenyl, (iv) pyridyl (v) furanyl or (vi) substituted or unsubstituted 4-methoxyphenyl, 4-fluorophenyl, 3-fluorophenyl, 4-ethoxyphenyl, 4-propoxyphenyl, 4-trifluoromethylphenyl, 4-pentafluoroethylphenyl, 3-fluoro-4-methoxyphenyl, 3-fluoro-4-ethoxyphenyl, 3-fluoro-4-propoxyphenyl, 3-methoxy-4-propoxyphenyl, 2-fluorophenyl, 4-methoxymethoxyphenyl, 4-hydroxyphenyl, 1,3-benzodioxolyl, 1,4-benzodioxanyl or dihydrobenzofuranyl wherein the substituent is selected from alkoxy, alkoxyalkoxy and carboxyalkoxy, R₂ is substituted or unsubstituted 1,3-benzodioxolyl, 7-methoxy-1,3-benzodioxolyl, 1,4-benzodioxanyl, 8-methoxy-1,4-benzodioxanyl, dihydrobenzofuranyl, benzofuranyl, 4-methoxyphenyl, dimethoxyphenyl, fluorophenyl or difluorophenyl.

6. The compound according to Claim 5 wherein R₃ is R₄-C(O)-R₅- wherein R₄ and R₅ are as defined therein.

7. The compound according to Claim 6 wherein R is -C(O)₂-G wherein G is hydrogen or a carboxy protecting group or R is tetrazolyl or R is -C(O)-NHS(O)₂R₁₆ wherein R₁₆ is alkyl or haloalkyl, R₁ is substituted or unsubstituted 4-methoxyphenyl, 4-fluorophenyl, 2-fluorophenyl, 4-ethoxyphenyl, 4-propoxyphenyl, 3-fluoro-4-methoxyphenyl, 3-fluoro-4-ethoxyphenyl, 3-fluoro-4-propoxyphenyl, 3-methoxy-4-propoxyphenyl, 4-trifluoromethylphenyl, 4-pentafluoroethylphenyl, 4-methoxymethoxyphenyl, 4-hydroxyphenyl, 1,3-benzodioxolyl, 1,4-benzodioxanyl or dihydrobenzofuranyl wherein the substituent is selected from alkoxy, alkoxyalkoxy and carboxyalkoxy, R₂ is 1,3-benzodioxolyl, 7-methoxy-1,3-benzodioxolyl, 1,4-benzodioxanyl, dihydrobenzofuranyl, benzofuranyl, 4-methoxyphenyl, dimethoxyphenyl, fluorophenyl or difluorophenyl.

8. The compound according to Claim 3 wherein wherein R is -C(O)₂-G wherein G is hydrogen or a carboxy protecting group, R₁ is substituted or unsubstituted 4-methoxyphenyl, 4-fluorophenyl, 2-fluorophenyl, 3-fluoro-4-ethoxyphenyl, 3-fluoro-4-methoxyphenyl, 4-ethoxyphenyl, 4-propoxyphenyl, 3-fluoro-4-propoxyphenyl, 3-methoxy-4-propoxyphenyl, 4-methoxymethoxyphenyl, 4-hydroxyphenyl, 4-ethylphenyl, 1,3-benzodioxolyl, 1,4-benzodioxanyl or dihydrobenzofuranyl wherein the substituent is selected from alkoxy, alkoxyalkoxy and carboxyalkoxy,
R₂ is 1,3-benzodioxolyl, 7-methoxy-1,3-benzodioxolyl, 1,4-benzodioxanyl, dihydrobenzofuranyl, benzofuranyl, 4-methoxyphenyl, dimethoxyphenyl, fluorophenyl or difluorophenyl and R₃ is R₄-C(O)-R₅- wherein R₄ is 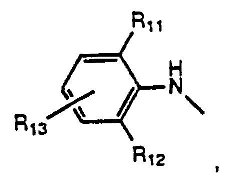 wherein R₁₁ and R₁₂ are independently selected from the group consisting of alkyl, and R₁₃ is hydrogen, alkyl, halo, carboxy, nitro, or cyano and R5 is methylene.

9. The compound according to Claim 8 wherein R₁₁ and R₁₂ are independently selected from the group consisting of methyl, ethyl and isopropyl.

10. The compound according to Claim 3 wherein wherein R is -C(O)₂-G wherein G is hydrogen or a carboxy protecting group, R₁ is substituted or unsubstituted 4-methoxyphenyl, 4-fluorophenyl, 2-fluorophenyl, 3-fluoro-4-ethoxyphenyl, 3-fluoro-4-methoxyphenyl, 4-ethoxyphenyl, 4-propoxyphenyl, 3-fluoro-4-propoxyphenyl, 3-methoxy-4-propoxyphenyl, 4-methoxymethoxyphenyl, 4-hydroxyphenyl, 4-ethylphenyl, 1,3-benzodioxolyl, 1,4-benzodioxanyl or dihydrobenzofuranyl wherein the substituent is selected from alkoxy, alkoxyalkoxy and carboxyalkoxy,
R₂ is 1,3-benzodioxolyl, 7-methoxy-1,3-benzodioxolyl, 1,4-benzodioxanyl, dihydrobenzofuranyl, benzofuranyl, 4-methoxyphenyl, dimethoxyphenyl, fluorophenyl or difluorophenyl and R₃ is R₄-C(O)-R₅- wherein R₄ is 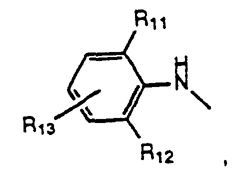 wherein R₁₁ and R₁₂ are independently selected from the group consisting of halo and alkoxy, and R₁₃ is hydrogen, alkyl, halo, carboxy, nitro, or cyano and R₅ is methylene.

11. A compound selected from the group consisting of
*trans,trans*-2-(4-Methoxyphenyl)-4-(1,3-benzodioxol-5-yl)-1-((2,6-diethyl)phenylaminocarbonylmethyl)-pyrrolidine-3-carboxylic acid;
*trans,trans*-2-(4-Propoxyphenyl)-4-(1,3-benzodioxol-5-yl)-1-((2,6-diethyl)phenylaminocarbonylmethyl)-pyrrolidine-3-carboxylic acid;
*trans,trans*-2-(3-Fluoro-4-methoxyphenyl)-4-(1,3-benzodioxol-5-yl)-1-((2,6-diethyl)phenylaminocarbonylmethyl)-pyrrolidine-3-carboxylic acid;
*trans,trans*-2-(3-Fluoro-4-ethoxyphenyl)-4-(1,3-benzodioxol-5-yl)-1-((2,6-diethyl)phenylaminocarbonylmethyl)-pyrrolidine-3-carboxylic acid;
*trans,trans*-2-(3-Fluoro-4-methoxyphenyl)-4-(7-methoxy-1,3-benzodioxol-5-yl)-1-((2,6-diethyl)phenylaminocarbonylmethyl)-pyrrolidine-3-carboxylic acid;
*trans,trans*-2-(3-methoxy-4-propoxyphenyl)-4-(1,3-benzodioxol-5-yl)-1-((2,6-diethyl)phenylaminocarbonylmethyl)-pyrrolidine-3-carboxylic acid;
*trans,trans*-2-(4-ethoxyphenyl)-4-(1,3-benzodioxol-5-yl)-1-((2,6-diethyl)phenylaminocarbonylmethyl)-pyrrolidine-3-carboxylic acid;
*trans, trans*-2-(4-propoxyphenyl)-4-(7-methoxy-1,3-benzodioxol-5-yl)-1-((2,6-diethyl)phenylaminocarbonylmethyl)-pyrrolidine-3-carboxylic acid
*trans,trans-*2-(3-methoxy-4-propoxyphenyl)-4-(7-methoxy-1,3-benzodioxol-5-yl)-1-((2,6-diethyl)phenylaminocarbonylmethyl)-pyrrolidine-3-carboxylic acid;
[*2R,3R,4S*]2-(4-Methoxyphenyl)-4-(1,3-benzodioxol-5-yl)-1-(N-((2,6-diethyl)phenylaminocarbonylmethyl)-pyrrolidine-3-carboxylic acid,
[*2R,3R,4S*]2-(4-Propoxyphenyl)-4-(1,3-benzodioxol-5-yl)-1-(N-(2,6-diethyl)phenylaminocarbonylmethyl)-pyrrolidine-3-carboxylic acid;
and (2*R*,3*R*,4*S*)-2-(4-ethoxyphenyl)-4-(1,3-benzodioxol-5-yl)-1-(2,6-diethyl)phenylaminocarbonylmethyl)-pyrrolidine-3-carboxylic acid,
or a pharmaceutically acceptable salt thereof.

12. A compound of claim 11 wherein said compound is *trans,trans*-2-(4-Propoxyphenyl)-4-(1,3-benzodioxol-5-yl)-1-((2,6-diethyl)phenylaminocarbonylmethyl)-pyrrolidine-3-carboxylic acid;
or a pharmaceutically acceptable salt thereof.

13. A compound of claim 11 wherein said compound is [*2R*,*3R*,*4S*]2-(4-Methoxyphenyl)-4-(1,3-benzodioxol-5-yl)-1 -(N-((2,6-diethyl)phenylaminocarbonylmethyl)-pyrrolidine-3-carboxylic acid, or a pharmaceutically acceptable salt thereof.

14. A compound of claim 11 wherein said compound is [*2R,3R,4S*]2-(4-Propoxyphenyl)-4-(1,3-benzodioxol-5-yl)-1-(N-((2,6-diethyl)phenylaminocarbonylmethyl)-pyrrolidine-3-carboxylic acid, or a pharmaceutically acceptable salt thereof.

15. A compound of claim 11 wherein said compound is (2*R*,3*R*,4*S*)-2-(4-ethoxyphenyl)-4-(1,3-benzodioxol-5-yl)-1-((2,6-diethyl)phenylaminocarbonylmethyl)-pyrrolidine-3-carboxylic acid, or a pharmaceutically acceptable salt thereof.

16. A pharmaceutical composition for antagonizing endothelin comprising a therapeutically effective amount of the compound of Claim 1 and a pharmaceutically acceptable carrier.

17. A pharmaceutical composition for antagonizing endothelin comprising a therapeutically effective amount of the compound of Claim 2 and a pharmaceutically acceptable carrier.

18. A compound of one of Claims 1 and 2 for use as a medicament.

19. Use of a compound of Claim 1 or of Claim 2 for manufacturing a medicament for antagonizing endothelin.

20. Use of a compound of Claim 1 or of Claim 2 for manufacturing a medicament for treating hypertension, congestive heart failure, restenosis following arterial injury, cerebral or myocardial ischemia or artherosclerosis.

21. Use of a compound of Claim 1 or of Claim 2 for manufacturing a medicament for treating coronary angina, cerebral vasospasm, acute and chronic pulmonary hypertension, acute and chronic renal failure, gastric ulceration, cyclosporin-induced nephrotoxicity, endotoxin-induced toxicity, asthma, LPL-related lipoprotein disorders, proliferative diseases, platelet aggregation, thrombosis, IL-2 mediated cardiotoxicity, nociception, colitis, vascular permeability disorders, ischemia-repurfusion injury, raynaud's disease, cancers, and migraine.

22. Use of a compound of Claim 1 or of Claim 2 in combination with one or more cardiovascular agents for manufacturing a medicament for treating hypertension, congestive heart failure, restenosis following arterial injury, cerebral or myocardial ischemia or artherosclerosis.

## Patentansprüche

1. Eine Verbindung gemäß folgender Formel: 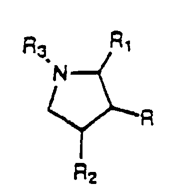 worin
R -(CH₂)ₘ-W ist, worin m eine ganze Zahl von 0 bis 6 ist und W ist
(a) -C(O)₂-G, worin G Wasserstoff oder eine CarboxySchutzgruppe ist, die leicht *in vivo* abgespalten werden kann,
(b) -PO₃H₂,
(c) -P(O) (OH)E, worin E Wasserstoff, Alkyl oder Arylalkyl ist,
(d) -CN,
(e) -C(O)NHR₁₇, worin R₁₇ Alkyl ist,
(f) Alkylaminocarbonyl,
(g) Dialkylaminocarbonyl,
(h) Tetrazolyl,
(i) Hydroxy,
(j) Alkoxy,
(k) Sulfonamido,
(l) -C(O)NHS(O)₂R₁₆, worin R₁₆ Alkyl, Haloalkyl, Aryl oder Dialkylamino ist,
(m) -S(O)₂NHC(O)R₁₆, worin R₁₆ wie oben definiert ist,
(n) 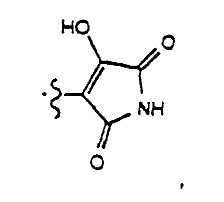
(o) 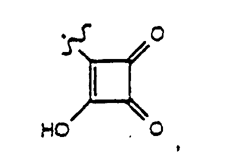
(p) 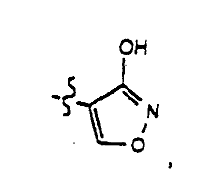
(q) 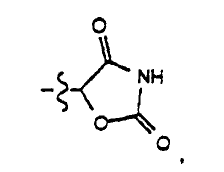
(r) 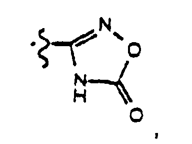
(s) 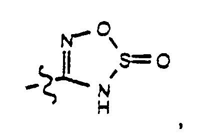
(t) 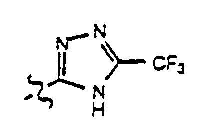 oder
(u) 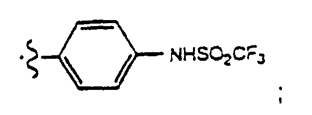
R₁ und R₂ sind unabhängig gewählt aus Wasserstoff, Alkyl, Alkenyl, Alinyl, Alkoxyalkyl, Alkoxycarbonylalkyl, Hydroxyalkyl, Haloalkyl, Haloalkoxyalkyl, Alkoxyalkoxyalkyl, Thioalkoxyalkoxyalkyl, Cycloalkyl, Cycloalkylalkyl, Aminocarbonylalkyl, Alkylaminocarbonylalkyl, Dialkylaminocarbonylalkyl, Aminocarbonylalkenyl, Alkylaminocarbonylalkenyl, Dialkylaminocarbonylalkenyl, Hydroxyalkenyl, Aryl, Arylalkyl, Aryloxyalkyl, Arylalkoxyalkyl, Heterozyklus, (Heterozyklus)Alkyl und
(Rₐₐ) (R_{bb})N-R_{cc}- worin Rₐₐ Aryl oder Arylalkyl ist, R_{bb} Wasserstoff oder Alkanoyl ist, und R_{cc} Alkylen ist, unter der Voraussetzung, daß eins oder beide von R₁ und R₂ etwas anderes ist als Wasserstoff;
R₃ ist R₄-C(O)-R₅- oder R₆-S(O)₂-R₇-, worin R₅ (i) eine kovalente Bindung ist, (ii) Alkylen, (iii) Alkenylen, (iv) -N(R₂₀)-R₈- oder R₈ₐ-N(R₂₀)-R₈-, worin R₈ und R₈ₐ unabhänging gewählt sind aus der Gruppe bstehend aus Alkylen und Alkenylen, und
R₂₀ ist Wasserstoff, Alkyl, Alkenyl, Haloalkyl, Alkoxyalkyl, Haloalkoxyalkyl; Cycloalkyl oder Cycloalkylalkyl, oder (v) -O-R₉oder R₉ₐ-O-R₉-, worin R₉ und R₉ₐ unabhängig gewählt sind aus Alkylen;
R₇ ist (i) eine kovalente Bindung, (ii) Alkylen, (iii) Alkenylen oder (iv) -N(R₂₁)-R₁₀- oder -R₁₀ₐ-N(R₂₁)-R₁₀-
worin R₁₀ und R₁₀ₐ unabhängig gewählt sind aus der Gruppe bestehend aus Alkylen und Alkenylen und
R₂₁ ist Wasserstoff, Alkyl, Alkenyl, Haloalkyl, Alkoxyalkyl, Haloalkoxyalkyl, Aryl oder Arylalkyl;
worin R₄ und R₆ folgendes sind: 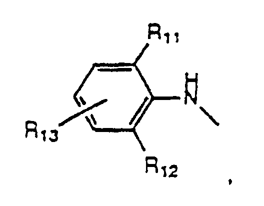 worin R₁₁ und R₁₂ gewählt sind aus der Gruppe bestehend aus Alkyl, Cyano, Alkoxy, Halo, Haloalkyl und Phenyl, und R₁₃ ist Wasserstoff, Alkyl, Halo, Carboxy, Nitro oder Cyano;
worin Alkyl selbst oder als ein Teil einer anderen Gruppe unabhängig bei jedem Auftreten von 1 bis 15 Kohlenstoffatome besitzt, Alkenyl selbst oder als ein Teil einer anderen Gruppe unabhängig bei jedem Auftreten von 2 bis 15 Kohlenstoffatome besitzt, Alkinyl von 2 bis 15 Kohlenstoffatome besitzt, Alkylen unabhängig bei jedem Auftreten von 1 bis 15 Kohlenstoffatome besitzt, Alkenylen unabhängig bei jedem Auftreten von 2 bis 15 Kohlenstoffatome besitzt, Cycloalkyl selbst oder als ein Teil einer anderen Gruppe unabhängig bei jedem Auftreten 3 bis 10 Kohlenstoffatome besitzt und wahlweise mit von ein bis drei Substituenten substituiert sein kann, die unabhängig gewählt sind aus Alkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid, Aryl selbst oder als ein Teil einer anderen Gruppe unabhängig bei jedem Auftreten ein mono- oder bizyklisches carbozyklisches Ringsystem mit ein oder zwei aromatischen Ringen ist und wahlweise mit von ein bis drei Substituenten substituiert sein kann, die unabhängig gewählt sind aus Alkyl, Halo, Haloalkyl, Haloalkoxy, Hydroxyalkyl, Alkenyloxy, Alkoxy, Alkoxyalkoxy, Alkoxycarbonyl, Alkoxycarbonylalkenyl, (Alkoxycarbonyl)thioalkoxy, Thioalkoxy, Amino, Alkylamino, Dialkylamino, Aminocarbonyl, Aminocarbonylalkoxy, Alkanoylamino, Arylakoxy, Aryloxy, Mercapto, Cyano, Nitro, Carboxaldehyd, Carboxy, Carboxyalkenyl, Carboxyalkoxy, Alkylsulfonylamino, Cyanoalkoxy, (heterozyklisch)Alkoxy, Hydroxy, Hydroxyalkoxy, Phenyl und Tetrazolylalkoxy oder wahlweise substituiertes Aryl kann Tetrafluorphenyl oder Pentafluorphenyl sein, Heterozyklus selbst oder als ein Teil einer anderen Gruppe ist unabhängig bei jedem Vorkaommen ein 3- oder 4-gliedriger Ring, der ein Heteroatom enthält, das gewählt ist aus Sauerstoff, Stickstoff und Schwefel, oder ist ein 5-, 6- oder 7-gliedriger Ring, der ein, zwei oder drei Stickstoffatome oder ein Sauerstoffatom oder ein Schwefelatom oder ein Stickstoffatom und ein Schwefelatom oder ein Stickstoffatom oder ein Sauerstoffatom oder zwei Sauerstoffatome in nicht angrenzenden Positionen enthält, oder ein Sauerstoffatom und ein Schwefelstoffatom in nicht angrenzenden Positionen, oder zwei Schwefelatome in nicht angrenzenden Positionen, worin der 5-gliedrige Ring 0-2 Doppelbindungen besitzt, und die 6- und 7-gliedrigen Ringe 0-3 Doppelbindungen besitzen, und die Stickstoffheteroatome können quaternisiert sein, oder Heterozyklus kann eine bizyklische Gruppe sein, in der jede der obigen heterozyklischen Ringe an einen Benzolring oder einen Cyclohexanring oder einen anderen der obigen heterozyklischen Ringe ankondensiert ist, worin Heterozyklus wahlweise substituiert sein kann durch ein, zwei oder drei Gruppen, die unabhängig gewählt sind aus der Gruppe bestehend aus Hydroxy, Halo, Oxo, Alkylimino, Amino, Alkylamino, Dialkylamino, Alkoxy, Alkoxyalkoxy, Haloalkyl, Cycloalkyl, Aryl, Phenyl, Arylalkyl, -COOH, -SO₃H, Alkoxycarbonyl, Nitro, Cyano und Alkyl;
oder ein pharmazeutisch verträgliches Salz davon.

2. Die Verbindung gemäß Anspruch 1 mit der Formel: 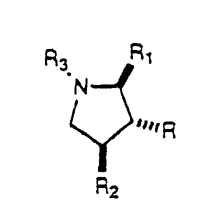 worin R, R₁, R₂ und R₃ wie darin definiert sind, oder ein pharmazeutisch verträgliches Salz davon.

3. Die Verbindung gemäß Anspruch 1 oder 2, worin R₃ R₄-C(O)-R₅- ist, worin R₄ wie darin definiert ist und R₅ ist Alkylen oder R₃ ist R₆-S(O)₂-R₇-, worin R₇ Alkylen ist und R₆ ist wie darin definiert.

4. Die Verbindung gemäß Anspruch 3, worin R -C(O)₂-G ist, worin G Wasserstoff ist oder eine Carboxyschutzgruppe oder R ist Tetrazolyl oder R ist -C(O)-NHS(O)₂R₁₆, worin R₁₆ Alkyl, Haloalkyl oder Aryl ist, und R₁ und R₂ sind unabhängig gewählt aus (i) Alkyl, (ii) Cycloalkyl, (iii) substituiertem Aryl, worin Aryl Phenyl ist, das substituiert ist mit ein, zwei oder drei Substituenten unabhängig gewählt aus Alkyl, Alkoxy, Halo, Alkoxyalkoxy und Carboxyalkoxy und (iv) substituiertem oder unsubstituiertem Heterozyklus.

5. Die Verbindung gemäß Anspruch 3, worin R -C(O)₂-G ist, worin G Wasserstoff ist oder eine Carboxyschutzgruppe, oder R ist Tetrazolyl oder R ist -C(O)-NHS(O)₂R₁₆, worin R₁₆ Alkyl, Haloalkyl oder Aryl ist, R₁ ist (i) Alkoxyalkyl, (ii) Cycloalkyl, (iii) Phenyl, (iv) Pyridyl, (v) Furanyl oder (vi) substituiertes oder unsubstituiertes 4-Methoxyphenyl, 4-Fluorphenyl, 3-Fluorphenyl, 4-Ethoxyphenyl, 4-Propoxyphenyl, 4-Trifluormethylphenyl, 4-Pentafluorethylphenyl, 3-Fluor-4-methoxyphenyl, 3-Fluor-4-ethoxyphenyl, 3-Fluor-4-propoxyphenyl, 3-Methoxy-4-propoxyphenyl, 2-Fluorphenyl, 4-Methoxymethoxyphenyl, 4-Hydroxyphenyl, 1,3-Benzodioxolyl, 1,4-Benzodioxanyl oder Dihydrobenzofuranyl, worin der Substituent gewählt ist aus Alkoxy, Alkoxyalkoxy und Carboxyalkoxy, R₂ ist substituiertes oder unsubstituiertes 1,3-Benzodioxolyl, 7-Methoxy-1,3-benzodioxolyl, 1,4-Benzodioxanyl, 8-Methoxy-1,4-benzodioxanyl, Dihydrobenzofuranyl, Benzofuranyl, 4-Methoxyphenyl, Dimethoxyphenyl, Fluorphenyl oder Difluorphenyl.

6. Die Verbindung gemäß Anspruch 5, worin R₃ R₄-C(O)-R₅ist, worin R₄ und R₅ wie darin definert sind.

7. Die Verbindung gemäß Anspruch 6, worin R -C(O)₂-G ist, worin G Wasserstoff oder eine Carboxyschutzgruppe ist, oder R ist Tetrazolyl oder R ist -C(O)-NHS(O)₂R₁₆, worin R₁₆ Alkyl oder Haloalykl ist, R₁ ist substituertes oder unsubstitiertes 4-Methoxyphenyl, 4-Fluorphenyl, 2-Fluorphenyl, 4-Ethoxypehnyl, 4-Propoxyphenyl, 3-Fluor-4-methoxyphenyl, 3-Fluor-4-ethoxyphenyl, 3-Fluor-4-propoxyphenyl, 3-Methoxy-4-propoxyphenyl, 4-Trifluormethylphenyl, 4-Pentafluorethylphenyl, 4-Methoxymethoxyphenyl, 4-Hydroxyphenyl, 1,3-Benzodioxolyl, 1,4-Benzodioxanyl oder Dihydrobenzofuranyl, worin der Substituent gewählt is aus Alkoxy, Alkoxyalkoxy und Carboxyalkoxy, R₂ ist 1,3-Benzodioxolyl, 7-Methoxy-1,3-benzodioxolyl, 1,4-Benzodioxanyl, Dihydrobenzofuranyl, Benzofuranyl, 4-Methoxyphenyl, Dimethoxyphenyl, Fluorphenyl oder Difluorphenyl.

8. Die Verbindung gemäß Anspruch 3, worin R -C(O)₂-G ist, worin G Wasserstoff oder eine Carboxyschutzgruppe ist, R₁ ist substituiertes oder unsubstituiertes 4-Methoxyphenyl, 4-Fluorphenyl, 2-Fluorphenyl, 3-Fluor-4-ethoxyphenyl, 3-Fluor-4-methoxyphenyl, 4-Ethoxyphenyl, 4-Propoxyphenyl, 3-Fluor-4-propoxyphenyl, 3-Methoxy-4-propoxyphenyl, 4-Methoxymethoxyphenyl, 4-Hydroxyphenyl, 4-Ethylphenyl, 1,3-Benzodioxolyl, 1,4-Benzodioxanyl oder Dihydrobenzofuranyl, worin der Substituent gewählt ist aus Alkoxy, Alkoxyalkoxy und Carboxyalkoxy,
R₂ ist 1,3-Benzodioxolyl, 7-Methoxy-1,3-benzodioxolyl, 1,4-Benzodioxanyl, Dihydrobenzofuranyl, Benzofuranyl, 4-Methoxyphenyl, Dimethoxyphenyl, Fluorphenyl oder Difluorphenyl und
R₃ ist R₄-C(O)-R₅-, worin R₄ folgendes ist: 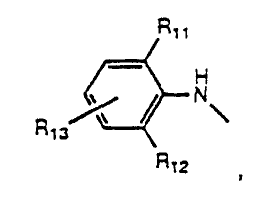 worin R₁₁ und R₁₂ unabhängig gewählt sind aus der Gruppe bestehend aus Alkyl, und R₁₃ ist Wasserstoff, Alkyl, Halo, Carboxy, Nitro oder Cyano und R₅ ist Methylen.

9. Die Verbindung gemäß Anspruch 8, worin R₁₁ und R₁₂ unabhängig gewählt sind aus der Gruppe bestehend aus Methyl, Ethyl und Isopropyl.

10. Die Verbindung gemäß Anspruch 3, worin R -C(O)₂-G ist, worin G Wasserstoff oder eine Carboxyschutzgruppe ist, R₁ ist substituiertes oder unsubstituiertes 4-Methoxyphenyl, 4-Fluorphenyl, 2-Fluorphenyl, 3-Fluor-4-ethoxyphenyl, 3-Fluor-4-methoxyphenyl, 4-Ethoxyphenyl, 4-Propoxyphenyl, 3-Fluor-4-propoxyphenyl, 3-methoxy-4-propoxyphenyl, 4-Methoxymethoxyphenyl, 4-Hydroxyphenyl, 4-Ethylphenyl, 1,3-Benzodioxolyl, 1,4-Benzodioxanyl oder Dihydrobenzofuranyl, worin der Substituent gewählt ist aus Alkoxy, Alkoxyalkoxy und Carboxyalkoxy,
R₂ ist 1,3-Benzodioxolyl, 7-Methoxy-1,3-benzodioxolyl, 1,4-Benzodioxanyl, Dihydrobenzofuranyl, Benzofuranyl, 4-Methoxyphenyl, Dimethoxyphenyl, Fluorphenyl oder Difluorphenyl und R₃ ist R₄-C(O)-R₅-, worin R₄ folgendes ist 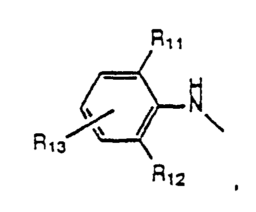 worin R₁₁ und R₁₂ unabhängig gewählt sind aus der Gruppe bestehend aus Halo und Alkoxy und R₁₃ ist Wasserstoff, Alkyl, Halo, Carboxy, Nitro oder Cyano und R₅ ist Methylen.

11. Eine Verbindung gewählt aus der Gruppe bestehend aus
*trans,trans*-2-(4-Methoxyphenyl)-4-(1,3-benzodioxol-5-yl)-1-((2,6-diethyl)phenylaminocarbonylmethyl)-pyrrolidin-3-carbonsäure;
*trans,trans*-2-(4-Propoxyphenyl)-4-(1,3-benzodioxol-5-yl)-1-((2,6-diethyl)phenylaminocarbonylmethyl)-pyrrolidin-3-carbonsäure;
*trans,trans*-2-(3-Fluor-4-methoxyphenyl)-4-(1,3-benzodioxol-5-yl)-1-((2,6-diethyl) phenylaminocarbonylmethyl) -pyrrolidin-3-carbonsäure;
*trans,trans*-2-(3-Fluor-4-ethoxyphenyl)-4-(1,3-benzodioxol-5-yl)-1-((2,6-diethyl)phenylaminocarbonylmethyl)-pyrrolidin-3-carbonsäure;
*trans,trans*-2-(3-Fluor-4-methoxyphenyl)-4-(7-methoxy-1,3-benzodioxol-5-yl)-1-((2,6-diethyl)phenylaminocarbonylmethyl)-pyrrolidin-3-carbonsäure;
*trans,trans*-2-(3-Methoxy-4-propoxyphenyl)-4-(1,3-benzodioxol-5-yl)-1-((2,6-diethyl)phenylaminocarbonylmethyl)-pyrrolidin-3-carbonsäure;
*trans,trans*-2-(4-Ethoxyphenyl)-4-(1,3-benzodioxol-5-yl)-1-((2,6-diethyl)phenylaminocarbonylmethyl)-pyrrolidin-3-carbonsäure;
*trans,trans*-2-(4-Propoxyphenyl)-4-(7-methoxy-1,3-benzodioxol-5-yl)-1-((2,6-diethyl)phenylaminocarbonylmethyl)-pyrrolidin-3-carbonsäure;
*trans,trans*-2-(3-Methoxy-4-propoxyphenyl)-4-(7-methoxy-1,3-benzodioxol-5-yl)-1-((2,6-diethyl)phenylaminocarbonylmethyl)-pyrrolidin-3-carbonsäure;
[2R,3R,4S]2-(4-Methoxyphenyl)-4-(1,3-benzodioxol-5-yl)-1-(N-((2,6-diethyl)phenylaminocarbonylmethyl)-pyrrolidin-3-carbonsäure;
[2R,3R,4S]2-(4-Propoxyphenyl)-4-(1,3-benzodioxol-5-yl)-1-(N-(2,6-diethyl)phenylaminocarbonylmethyl)-pyrrolidin-3-carbonsäure; und
(2R,3R,4S)2-(4-Ethoxyphenyl)-4-(1,3-benzodioxol-5-yl)-1-(2, 6-diethyl)phenylaminocarbonylmethyl)-pyrrolidin-3-carbonsäure;
oder ein pharmazeutisch verträgliches Salz davon.

12. Eine Verbindung von Anspruch 11, worin die Verbindung *trans,trans*-2-(4-Propoxyphenyl)-4-(1,3-benzodioxol-5-yl)-1-((2,6-diethyl)phenylaminocarbonylmethyl)-pyrrolidin-3-carbonsäure ist; oder ein pharmazeutisch verträgliches Salz davon.

13. Eine Verbindung von Anspruch 11, worin die Verbindung [2R,3R,4S]2-(4-Methoxyphenyl)-4-(1,3-benzodioxol-5-yl)-1-(N-((2,6-diethyl)phenylaminocarbonylmethyl)-pyrrolidin-3-carbonsäure ist, oder ein pharmazeutisch verträgliches Salz davon.

14. Eine Verbindung von Anspruch 11, worin die Verbindung [2R,3R,4S]2-(4-Propoxyphenyl)-4-(1,3-benzodioxol-5-yl)-1-(N-((2,6-diethyl)phenylaminocarbonylmethyl)-pyrrolidin-3-carbonsäure ist, oder ein pharmazeutisch verträgliches Salz davon.

15. Eine Verbindung von Anspruch 11, worin die Verbindung (2R,3R,4S)-2-(4-Ethoxyphenyl)-4-(1,3-benzodioxol-5-yl)-1-((2,6-diethyl)phenylaminocarbonylmethyl)-pyrrolidin-3-carbonsäure ist, oder ein pharmazeutisch verträgliches Salz davon.

16. Eine pharmazeutische Zusammensetzung zur Antagonisierung von Endothelin, die eine therapeutisch effektive Menge der Verbindung von Anspruch 1 und einen pharmazeutisch verträglichen Träger umfaßt.

17. Eine pharmazeutische Zusammensetzung zur Antagonisierung von Endothelin, die eine therapeutisch effektive Menge der Verbindung von Anspruch 2 und einen pharmazeutisch verträglichen Träger umfaßt.

18. Eine Verbindung von einem der Ansprüche 1 und 2 zur Verwendung als ein Medikament.

19. Verwendung einer Verbindung von Anspruch 1 oder von Anspruch 2 zur Herstellung eines Medikamentes zur Antagonisierung von Endothelin.

20. Verwendung einer Verbindung von Anspruch 1 oder von Anspruch 2 zur Herstellung eines Medikamentes für die Behandlung von Hypertonie, kongestivem Herzversagen, Stenoserezidiv nach Arterienverletzung, zerebraler oder myokardialer Ischämie oder Artherosklerose.

21. Verwendung einer Verbindung von Anspruch 1 oder von Anspruch 2 zur Herstellung eines Medikamentes zur Behandlung von Angina pectoris, zerebralem Vasospasmus, akuter und chronischer Lungenhypertonie, akuter und chronischer Nierenversagen, Magengeschwür, Cyclosporin-induzierter Nephrotoxizität, Endotoxin-induzierter Toxizität, Asthma, LPL-verwandten Lipoproteinerkrankungen, proliferativen Erkrankungen, Plättchenaggregation, Thrombose, IL-2 vermittelter Kardiotoxizität, Schmerzempfindung, Kolitis, vaskuläre Permeabilitätsstörungen, Ischämie-Reperfusions-Verletzung, Raynaud-Krankheit, Tumoren und Migräne.

22. Verwendung einer Verbindung von Anspruch 1 oder von Anspruch 2 in Kombination mit einem oder mehreren kardiovaskulären Wirstoffen zur Herstellung eines Medikamentes zur Behandlung von Hypertonie, kongestivem Herzversagen, Stenoserezidiv nach Arterienverletzung, zerebraler oder myokardialer Ischämie oder Artherosklerose.

## Revendications

1. Composé de formule : 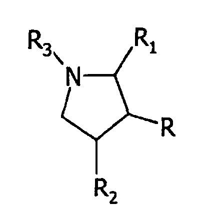 où
R est -(CH₂)ₘ-W où m est un entier de 0 à 6 et W est
(a) -C(O)₂-G où G est l'hydrogène ou un groupe carboxy-protecteur qui peut être aisément clivé in vivo,
(b) -PO₃H₂,
(c) -P(O)(OH)E où E est l'hydrogène, alkyle ou arylalkyle,
(d) -CN,
(e) -C(O)NHR₁₇ où R₁₇ est alkyle,
(f) alkylaminocarbonyle,
(g) dialkylaminocarbonyle,
(h) tétrazolyle,
(i) hydroxyle,
(j) alcoxy,
(k) sulfonamido,
(I) -C(O)NHS(O)₂R₁₆ où R₁₆ est alkyle, halogénoalkyle, aryle ou dialkylamino,
(m) -S(O)₂NHC(O)R₁₆ où R₁₆ est défini comme ci-dessus,
(n) 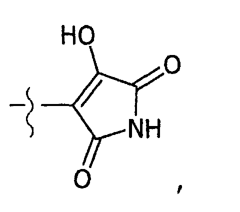
(o) 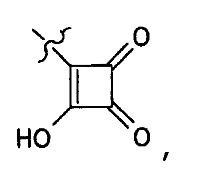
(p) 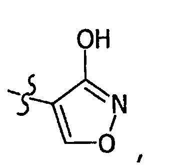
(q) 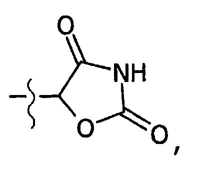
(r) 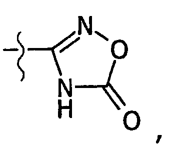
(s) 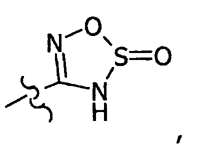
(t) 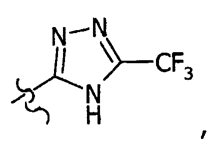 ou
(u) 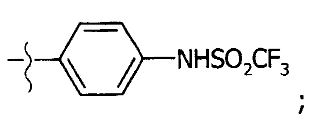
R₁ et R₂ sont choisis indépendamment parmi l'hydrogène, alkyle, alcényle, alcynyle, alcoxyalkyle, alcoxycarbonylalkyle, hydroxyalkyle, halogénoalkyle, halogénoalcoxyalkyle, alcoxyalcoxyalkyle, thioalcoxyalcoxyalkyle, cycloalkyle, cycloalkylalkyle, aminocarbonylalkyle, alkylaminocarbonylalkyle, dialkylaminocarbonylalkyle, aminocarbonylalcényle, alkylaminocarbonylalcényle, dialkylaminocarbonylalcényle, hydroxyalcényle, aryle, arylalkyle, aryloxy-alkyle, arylalcoxyalkyle, hétérocyclique, (hétérocyclique)alkyle et (Rₐₐ)(R_{bb})N-R_{cc}- où Rₐₐ est aryle ou arylalkyle, R_{bb} est l'hydrogène ou alcanoyle et R_{cc} est alkylène, à condition que, parmi R₁ et R₂, l'un ou les deux soient différents de l'hydrogène ;
R₃ est R₄-C(O)-R₅- ou R₆-S(O)₂-R₇- où R₅ est (i) une liaison covalente, (ii) alkylène, (iii) alcénylène, (iv) -N(R₂₀)-R₈- ou -R₈ₐ-N(R₂₀)-R₈- où R₈ et R₈ₐ sont choisis indépendamment dans le groupe consistant en alkylène et alcénylène et
R₂₀ est l'hydrogène, alkyle, alcényle, halogénoalkyle, alcoxyalkyle, halogénoalcoxyalkyle, cycloalkyle ou cycloalkylalkyle ou (v) -O-R₉- ou -R₉ₐ-O-R₉- où R₉ et R₉ₐ sont choisis indépendamment parmi alkylène ;
R₇ est (i) une liaison covalente, (ii) alkylène, (iii) alcénylène ou (iv) -N(R₂₁)-R₁₀- ou -R₁₀ₐ-N(R₂₁)-R₁₀-
où R₁₀ et R₁₀ₐ sont choisis indépendamment dans le groupe consistant en alkylène et alcénylène et
R₂₁ est l'hydrogène, alkyle, alcényle, halogénoalkyle, alcoxyalkyle, halogénoalcoxyalkyle, aryle ou arylalkyle ;
où R₄ et R₆ sont 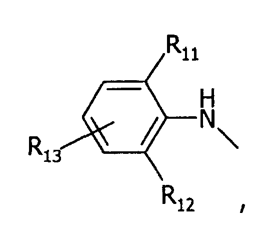 où R₁₁ et R₁₂ sont choisis indépendamment dans le groupe consistant en alkyle, cyano, alcoxy, halogéno, halogénoalkyle et phényle et R₁₃ est l'hydrogène, alkyle, halogéno, carboxyle, nitro ou cyano ;
où alkyle par lui-même ou faisant partie d'un autre groupe a indépendamment dans chaque cas de 1 à 15 atomes de carbone, alcényle par lui-même ou faisant partie d'un autre groupe a indépendamment dans chaque cas de 2 à 15 atomes de carbone, alcynyle a de 2 à 15 atomes de carbone, alkylène a indépendamment dans chaque cas de 1 à 15 atomes de carbone, alcénylène a indépendamment dans chaque cas de 2 à 15 atomes de carbone, cycloalkyle par lui-même ou faisant partie d'un autre groupe a indépendamment dans chaque cas 3 à 10 atomes de carbone et peut être éventuellement substitué par 1 à 3 substituants choisis indépendamment parmi alkyle, halogénoalkyle, alcoxy, thioalcoxy, amino, alkylamino, dialkylamino, hydroxyle, halogéno, mercapto, nitro, carboxaldéhyde, carboxyle, alcoxycarbonyle et carboxamide, aryle par lui-même ou faisant partie d'un autre groupe est indépendamment dans chaque cas un système cyclique carbocyclique mono ou bicyclique ayant 1 ou 2 cycles aromatiques et peut éventuellement être substitué par 1 à 3 substituants choisis indépendamment parmi alkyle, halogéno, halogénoalkyle, halogénoalcoxy, hydroxyalkyle, alcényloxy, alcoxy, alcoxyalcoxy, alcoxycarbonyle, alcoxycarbonyalcényle, (alcoxycarbonyl)-thioalcoxy, thioalcoxy, amino, alkylamino, dialkylamino, aminocarbonyle, aminocarbonylalcoxy, alcanoylamino, arylalcoxy, aryloxy, mercapto, cyano, nitro, carboxaldéhyde, carboxy, carboxyalcényle, carboxyalcoxy, alkylsulfonylamino, cyanoalcoxy, alcoxy (hétérocyclique), hydroxyle, hydroxyalcoxy, phényle et tétrazolylalcoxy ou aryle éventuellement substitué peut être tétrafluorophényle ou pentafluorophényle, hétérocyclique par lui-même ou faisant partie d'un autre groupe est indépendamment dans chaque cas un cycle à 3 ou 4 chaînons contenant un hétéroatome choisi parmi l'oxygène, l'azote et le soufre ou est un cycle à 5, 6, ou 7 chaînons contenant 1, 2 ou 3 atomes d'azote ou un atome d'oxygène ou un atome de soufre ou un atome d'azote et un atome de soufre ou un atome d'azote ou un atome d'oxygène ou 2 atomes d'oxygène dans des positions non adjacentes ou un atome d'oxygène et un atome de soufre dans des positions non adjacentes ou 2 atomes de soufre dans des positions non adjacentes où le cycle à 5 chaînons a 0-2 doubles liaisons et les cycles à 6 et 7 chaînons ont 0-3 doubles liaisons et les hétéroatomes d'azote peuvent être quaternisés, ou bien hétérocyclique peut être un groupe bicyclique dans lequel l'un quelconque des cycles hétérocycliques ci-dessus est fusionné à un cycle benzénique ou un cycle cyclohexane ou un autre des cycles hétérocycliques ci-dessus, où hétérocyclique peut éventuellement être substitué par 1, 2 ou 3 groupes choisis indépendamment dans le groupe consistant en hydroxyle, halogéno, oxo, alkylimino, amino, alkylamino, dialkylamino, alcoxy, alcoxyalcoxy, halogénoalkyle, cycloalkyle, aryle, phényle, arylalkyle, -COOH, -SO₃H, alcoxycarbonyle, nitro, cyano et alkyle ;
ou sel pharmaceutiquement acceptable de celui-ci.

2. Composé selon la revendication 1 de formule : 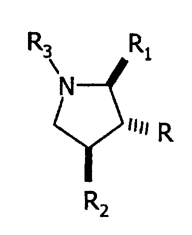 où R, R₁, R₂ et R₃ sont définis comme ici, ou sel pharmaceutiquement acceptable de celui-ci.

3. Composé selon la revendication 1 ou 2, où R₃ est R₄-C(O)-R₅- où R₄ est défini comme ici et R₅ est alkylène ou R₃ est R₆-S(O)₂-R₇- où R₇ est alkylène et R₆ est défini comme ici.

4. Composé selon la revendication 3 où R est -C(O)₂-G où G est l'hydrogène ou un groupe carboxy-protecteur ou bien R est tétrazolyle ou R est -C(O)-NHS(O)₂R₁₆ ou R₁₆ est alkyle, halogénoalkyle ou aryle et R₁ et R₂ sont choisis indépendamment parmi (i) alkyle, (ii) cycloalkyle, (iii) aryle substitué où aryle est phényle substitué par 1, 2 ou 3 substituants choisis indépendamment parmi alkyle, alcoxy, halogéno, alcoxyalcoxy et carboxyalcoxy et (iv) hétérocyclique substitué ou non substitué.

5. Composé selon la revendication 3 où R est -C(O)₂-G où G est l'hydrogène ou un groupe carboxy-protecteur ou bien R est tétrazolyle ou R est -C(O)-NHS(O)₂R₁₆ où R₁₆ est alkyle, halogénoalkyle ou aryle, R₁ est (i) alcoxyalkyle, (ii) cycloalkyle, (iii) phényle, (iv) pyridyle, (v) furanyle ou (vi) 4-méthoxyphényle, 4-fluorophényle, 3-fluorophényle, 4-éthoxyphényle, 4-propoxyphényle, 4-trifluorométhylphényle, 4-pentafluoroéthylphényle, 3-fluoro-4-méthoxyphényle, 3-fluoro-4-éthoxyphényle, 3-fluoro-4-propoxyphényle, 3-méthoxy-4-propoxyphényle, 2-fluorophényle, 4-méthoxyméthoxyphényle, 4-hydroxyphényle, 1,3-benzodioxolyle, 1,4-benzodioxanyle ou dihydrobenzofuranyle substitué ou non substitué où le substituant est choisi parmi alcoxy, alcoxyalcoxy et carboxyalcoxy,
R₂ est 1,3-benzodioxolyle, 7-méthoxy-1,3-benzodioxolyle, 1,4-benzodioxanyle, 8-méthoxy-1,4-benzodioxanyle, dihydrobenzofuranyle, benzofuranyle, 4-méthoxyphényle, diméthoxyphényle, fluorophényle ou difluorophényle substitué ou non substitué.

6. Composé selon la revendication 5, où R₃ est R₄-C(O)-R₅- où R₄ et R₅ sont définis comme ici.

7. Composé selon la revendication 6 où R est -C(O)₂-G où G est l'hydrogène ou un groupe carboxy-protecteur ou bien R est tétrazolyle ou R est -C(O)-NHS(O)₂R₁₆ où R₁₆ est alkyle ou halogénoalkyle,
R₁ est 4-méthoxyphényle, 4-fluorophényle, 2-fluorophényle, 4-éthoxyphényle, 4-propoxyphényle, 3-fluoro-4-méthoxyphényle, 3-fluoro-4-éthoxyphényle, 3-fluoro-4-propoxyphényle, 3-méthoxy-4-propoxyphényle, 4-trifluorométhylphényle, 4-pentafluoroéthylphényle, 4-méthoxyméthoxyphényle, 4-hydroxyphényle, 1,3-benzodioxolyle, 1,4-benzodioxanyle ou dihydrobenzofuranyle substitué ou non substitué où le substituant est choisi parmi alcoxy, alcoxyalcoxy et carboxyalcoxy, R₂ est 1,3-benzodioxolyle, 7-méthoxy-1,3-benzodioxolyle, 1,4-benzodioxanyle, dihydrobenzofuranyle, benzofuranyle, 4-méthoxyphényle, diméthoxyphényle, fluorophényle ou difluorophényle.

8. Composé selon la revendication 3 où R est -C(O)₂-G où G est l'hydrogène ou un groupe carboxy-protecteur, R₁ est 4-méthoxyphényle, 4-fluorophényle, 2-fluorophényle, 3-fluoro-4-éthoxyphényle, 3-fluoro-4-méthoxyphényle, 4-éthoxyphényle, 4-propoxyphényle, 3-fluoro-4-propoxyphényle, 3-méthoxy-4-propoxyphényle, 4-méthoxyméthoxyphényle, 4-hydroxyphényle, 4-éthylphényle, 1,3-benzodioxolyle, 1,4-benzodioxanyle ou dihydrobenzofuranyle substitué ou non substitué où le substituant est choisi parmi alcoxy, alcoxyalcoxy et carboxyalcoxy,
R₂ est 1,3-benzodioxolyle, 7-méthoxy-1,3-benzodioxolyle, 1,4-benzodioxanyle, dihydrobenzofuranyle, benzofuranyle, 4-méthoxyphényle, diméthoxyphényle, fluorophényle ou difluorophényle et R₃ est R₄-C(O)-R₅- où R₄ est 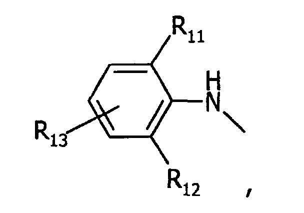 où R₁₁ et R₁₂ sont choisis indépendamment dans le groupe consistant en alkyle, et R₁₃ et l'hydrogène, alkyle, halogéno, carboxyle, nitro ou cyano et R₅ est méthylène.

9. Composé selon la revendication 8, où R₁₁ et R₁₂ sont choisis indépendamment dans le groupe consistant en méthyle, éthyle et isopropyle.

10. Composé selon la revendication 3, où R est -C(O)₂-G où G est l'hydrogène ou un groupe carboxy-protecteur, R₁ est 4-méthoxyphényle, 4-fluorophényle, 2-fluorophényle, 3-fluoro-4-éthoxyphényle, 3-fluoro-4-méthoxyphényle, 4-éthoxyphényle, 4-propoxyphényle, 3-fluoro-4-propoxyphényle, 3-méthoxy-4-propoxyphényle, 4-méthoxyméthoxyphényle, 4-hydroxyphényle, 4-éthylphényle, 1,3-benzodioxolyle, 1,4-benzodioxanyle ou dihydrobenzofuranyle substitué ou non substitué où le substituant est choisi parmi alcoxy, alcoxyalcoxy et carboxyalcoxy,
R₂ est 1,3-benzodioxolyle, 7-méthoxy-1,3-benzodioxolyle, 1,4-benzodioxanyle, dihydrobenzofuranyle, benzofuranyle, 4-méthoxyphényle, diméthoxyphényle, fluorophényle ou difluorophényle et R₃ est R₄-C(O)-R₅- où R₄ est 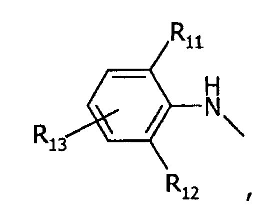 où R₁₁ et R₁₂ sont choisis indépendamment dans le groupe consistant en halogéno et alcoxy, et R₁₃ est l'hydrogène, alkyle, halogéno, carboxyle, nitro ou cyano et R₅ est méthylène.

11. Composé choisi dans le groupe consistant en l'acide
*trans,trans*-2-(4-méthoxyphényl)-4-(1,3-benzodioxol-5-yl)-1-((2,6-diéthyl)-phénylaminocarbonylméthyl)-pyrrolidine-3-carboxylique ;
l'acide trans,trans-2-(4-propoxyphényl)-4-(1,3-benzodioxol-5-yl)-1-((2,6-diéthyl)phénylaminocarbonylméthyl)-pyrrolidine-3-carboxylique ;
l'acide trans,trans-2-(3-fluoro-4-méthoxyphényl)-4-(1,3-benzodioxol-5-yl)-1-((2,6-diéthyl)phénylaminocarbonylméthyl)-pyrrolidine-3-carboxylique ;
l'acide trans,trans-2-(3-fluoro-4-éthoxyphényl)-4-(1,3-benzodioxol-5-yl)-1-((2,6-diéthyl)phénylaminocarbonylméthyl)-pyrrolidine-3-carboxylique ;
l'acide trans,trans-2-(3-fluoro-4-méthoxyphényl)-4-(7-méthoxy-1,3-benzodioxol-5-yl)-1-((2,6-diéthyl)phénylaminocarbonylméthyl)-pyrrolidine-3-carboxylique ;
l'acide trans,trans-2-(3-méthoxy-4-propoxyphényl)-4-(1,3-benzodioxol-5-yl)-1-((2,6-diéthyl)phénylaminocarbonylméthyl)-pyrrolidine-3-carboxylique ;
l'acide trans,trans-2-(4-éthoxyphényl)-4-(1,3-benzodioxol-5-yl)-1-((2,6-diéthyl)phénylaminocarbonylméthyl)-pyrrolidine-3-carboxylique;
l'acide trans,trans-2-(4-propoxyphényl)-4-(7-méthoxy-1,3-benzodioxol-5-yl)-1-((2,6-diéthyl)phénylaminocarbonylméthyl)-pyrrolidine-3-carboxylique ;
l'acide trans,trans-2-(3-méthoxy-4-propoxyphényl)-4-(7-méthoxy-1,3-benzodioxol-5-yl)-1-((2,6-diéthyl)phénylaminocarbonylméthyl)-pyrrolidine-3-carboxylique ;
l'acide [*2R,3R,4S*]-2-(4-méthoxyphényl)-4-(1,3-benzodioxol-5-yl)-1-(N-((2,6-diéthyl)phénylaminocarbonylméthyl)-pyrrolidine-3-carboxylique ;
l'acide [*2R,3R,4S*]-2-(4-propoxyphényl)-4-(1,3-benzodioxol-5-yl)-1-(N-(2,6-diéthyl)phénylaminocarbonylméthyl)-pyrrolidine-3-carboxylique ;
et l'acide (*2R,3R,4S*)-2-(4-éthoxyphényl)-4-(1,3-benzodioxol-5-yl)-1-(2,6-diéthyl)phénylaminocarbonylméthyl)-pyrrolidine-3-carboxylique,
ou un sel pharmaceutiquement acceptable de celui-ci.

12. Composé selon la revendication 11 où ledit composé est l'acide *trans,trans*-2-(4-propoxyphényl)-4-(1,3-benzodioxol-5-yl)-1-((2,6-diéthyl)phénylaminocarbonylméthyl)-pyrrolidine-3-carboxylique ;
ou un sel pharmaceutiquement acceptable de celui-ci.

13. Composé selon la revendication 11 où ledit composé est l'acide [*2R,3R,4S*]-2-(4-méthoxyphényl)-4-(1,3-benzodioxol-5-yl)-1-(N-((2,6-diéthyl)phénylaminocarbonylméthyl)-pyrrolidine-3-carboxylique,
ou un sel pharmaceutiquement acceptable de celui-ci.

14. Composé selon la revendication 11 où ledit composé est l'acide [*2R,3R,4S*]-2-(4-propoxyphényl)-4-(1,3-benzodioxol-5-yl)-1-(N-((2,6-diéthyl)phénylaminocarbonylméthyl)-pyrrolidine-3-carboxylique, ou un sel pharmaceutiquement acceptable de celui-ci.

15. Composé selon la revendication 11 où ledit composé est l'acide (*2R,3R,4S*)-2-(4-éthoxyphényl)-4-(1,3-benzodioxol-5-yl)-1-((2,6-diéthyl)phénylaminocarbonyméthyl)-pyrrolidine-3-carboxylique, ou un sel pharmaceutiquement de celui-ci.

16. Composition pharmaceutique pour antagoniser l'endothéline comprenant une quantité thérapeutiquement efficace du composé selon la revendication 1 et un support pharmaceutiquement acceptable.

17. Composition pharmaceutique pour antagoniser l'endothéline comprenant une quantité thérapeutiquement efficace du composé selon la revendication 2 et un support pharmaceutiquement acceptable.

18. Composé selon l'une des revendications 1 et 2 destiné à être utilisé comme médicament.

19. Utilisation d'un composé selon la revendication 1 ou la revendication 2 pour fabriquer un médicament pour antagoniser l'endothéline.

20. Utilisation d'un composé selon la revendication 1 ou la revendication 2 pour fabriquer un médicament pour traiter l'hypertension, l'insuffisance cardiaque congestive, la resténose consécutive à une lésion artérielle, l'ischémie cérébrale ou myocardique ou l'athérosclérose.

21. Utilisation d'un composé selon la revendication 1 ou la revendication 2 pour fabriquer un médicament pour traiter l'angine coronarienne, le vasospasme cérébral, l'hypertension pulmonaire aiguë et chronique, l'insuffisance rénale aiguë et chronique, l'ulcère gastrique, la néphrotoxicité induite par les cyclosporines, la toxicité induite par les endotoxines, l'asthme, les troubles dus aux lipoprotéines liées à LPL, les maladies prolifératives, l'agrégation plaquettaire, la thrombose, la cardiotoxicité à médiation par IL-2, la nociception, la colite, les troubles de la perméabilité vasculaire, les lésions d'ischémie-reperfusion, la maladie de Raynaud, les cancers et la migraine.

22. Utilisation d'un composé selon la revendication 1 ou la revendication 2 en combinaison avec un ou plusieurs agents cardio-vasculaires pour fabriquer un médicament pour traiter l'hypertension, l'insuffisance cardiaque congestive, la resténose consécutive à une lésion artérielle, l'ischémie cérébrale ou myocardique ou l'athérosclérose.
